(19) Europäisches Patentamt
European Patent Office
Office européen des brevets

(11) EP 1 200 417 B1

(12) **FASCICULE DE BREVET EUROPEEN**

(45) Date de publication et mention de la délivrance du brevet: **22.10.2003 Bulletin 2003/43**

(21) Numéro de dépôt: **00949673.8**

(22) Date de dépôt: **07.07.2000**

(51) Int Cl.[7]: **C07D 265/30**, C07C 215/30, C07C 271/16, C07C 33/30, C07D 303/08, C07D 309/12, C07C 47/273, C07C 33/48

(86) Numéro de dépôt international: **PCT/FR00/01966**

(87) Numéro de publication internationale: **WO 01/004105 (18.01.2001 Gazette 2001/03)**

(54) **PREPARATION DE DERIVES DE 2-(2-ARYLMORPHOLIN-2-YL)ETHANOL ET INTERMEDIAIRES**

HERSTELLUNG VON 2-(2-ARYLMORPHOLIN-2-YL)ETHANOLDERIVATEN UND ZWISCHENPRODUKTE

METHOD FOR PREPARING 2-(2-ARYLMORPHOLIN-2-YL)ETHANOL DERIVATIVES AND INTERMEDIATES

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **09.07.1999 FR 9909061**

(43) Date de publication de la demande: **02.05.2002 Bulletin 2002/18**

(73) Titulaire: **SANOFI-SYNTHELABO**
**75013 Paris (FR)**

(72) Inventeurs:
- **AULOMBARD, Alain**
  **F-34970 Lattes (FR)**
- **BERNON, Françoise**
  **F-34980 Saint Gély du Fesc (FR)**
- **BONNEFOY, Sabrina**
  **F-34090 Montpellier (FR)**
- **BURGOS, Alain**
  **Exton, PA 19341 (US)**
- **CABOS, Claude**
  **F-34990 Juvignac (FR)**
- **LUCAS, Eric**
  **F-34150 La Boissière (FR)**

(74) Mandataire: **Thouret-Lemaitre, Elisabeth et al**
**Sanofi-Synthélabo,**
**Département Brevets,**
**174, avenue de France**
**75013 Paris (FR)**

(56) Documents cités:
**WO-A-00/39072** **WO-A-96/23787**
**WO-A-99/28307**

- **TAKAHIDE N. ET AL: "An efficient synthesis of enantiomerically pure 2-[(2R)-arylmorpholin 2-yl]ethanols, key intermediates of tachykinin receptor antagonist" TETRAHEDRON : ASYMMETRY, no. 9, 1998, pages 3251-3262, XP002150499 OXFORD GB cité dans la demande**
- **K. A. VAN HOUTEN ET AL: "A new strategy for the design of monoamine oxidase inactivators. Exploratory studies with tertiary allylic and propargylic amino alcohols" JOURNAL OF THE AMERICAN CHEMICAL SOCIETY., vol. 120, no. 24, 1998, pages 5864-5872, XP002150500 DC US**
- **DATABASE CROSSFIRE [en ligne] Beilstein Informationssysteme GmbH; Frankfurt DE, XP002150501 & SYNTH. COMMUN., vol. 19, no. 19, 1989, pages 3313-3322,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet de nouveaux procédés de préparation de dérivés de 2-(2-arylmorpholin-2-yl)éthanol substitués, sous forme énantiomériquement pure, ainsi que des composés intermédiaires utiles dans ces procédés.

Les dérivés de 2-(2-arylmorpholin-2-yl)éthanol substitués de formule :

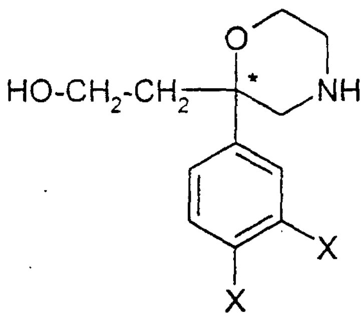

(I)

dans laquelle X représente un atome d'halogène et * indique la position de l'atome de carbone asymétrique, sont des intermédiaires clés pour la préparation de composés antagonistes des récepteurs des tachykinines tels que ceux décrits dans la demande internationale WO 96/23787 et dans la demande EP-A-776 893. Ainsi, par exemple, le (R)-(+)-3-{1-[2-(4-benzoyl-2-(3,4-difluorophényl)morpholin-2-yl)éthyl]-4-phényl pipéridin-4-yl}-1,1-diméthylurée est décrit comme un antagoniste puissant et sélectif des récepteurs $NK_2$ humains de la neurokinine A (X. Emonds-Alt et al., Neuropeptides, 1997, 31 (5), 449-458) et, en conséquence, peut être utile notamment dans le traitement des affections du système respiratoire, gastro-intestinal, urinaire, immunitaire, cardiovasculaire, et du système nerveux central ainsi que de la douleur et de la migraine.

Par atome d'halogène on entend un atome de brome, de chlore, de fluor ou d'iode.

De préférence, la présente invention a pour objet de nouveaux procédés de préparation de composés de formule (I), énantiomériquement purs, dans laquelle X représente un atome de chlore ou un atome de fluor.

La préparation de composés de formule (I) est illustrée dans la demande internationale WO 96/23787 et s'effectue selon le schéma 1 ci-après dans lequel X représente un atome d'halogène.

SCHEMA 1

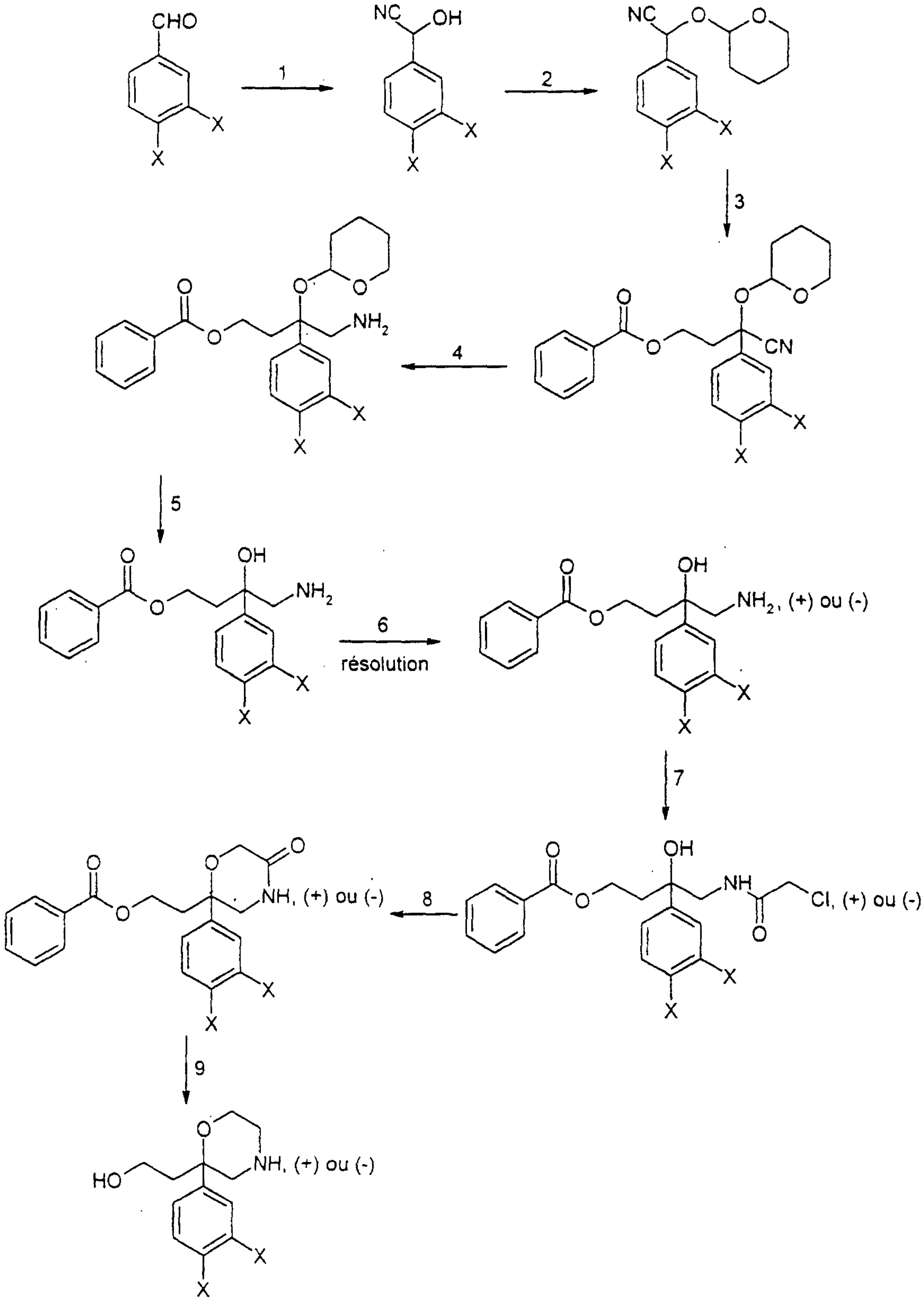


(I)

Toutefois, ce procédé comporte des inconvénients, suffisants pour l'écarter de toute utilisation à l'échelle industrielle.

Par exemple, le composé de formule (I) dans laquelle X représente un atome de fluor préparé par ce procédé

est obtenu avec un rendement très peu élevé, de l'ordre de 1 à 2 % calculé à partir du dérivé benzaldéhyde de départ, d'après la description de la demande WO 96/23787.

Le composé de formule (I) dans laquelle X représente un atome de chlore ou de fluor, peut également être préparé suivant le procédé énantiosélectif décrit dans Tetrahedron : Asymmetry, 1998, 9, 3251-3262. Toutefois ce procédé a l'inconvénient d'utiliser des matières premières tels que le dicétène et des réactifs tels que le dichlorobis (triphényl phosphine)palladium (II), l'AD-mix-β® ou l'azodicarboxylate de diéthyle dont les prix rendent la production de composé de formule (I), à l'échelle industrielle, fort onéreuse.

On a maintenant trouvé de nouveaux procédés de préparation du composé de formule (I) énantiomériquement pur à partir de matières premières et de réactifs simples et avec des rendements de l'ordre de 5 à 25 %.

Ainsi, selon un de ses aspects, la présente invention a pour objet un procédé A de préparation d'un composé, sous forme énantiomériquement pure, de formule :

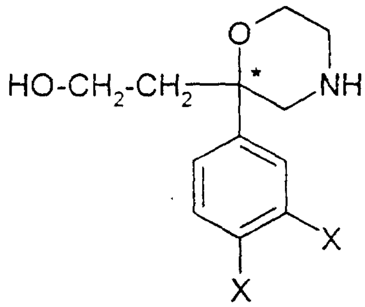

(I)

dans laquelle X représente un atome d'halogène, de ses sels avec des acides minéraux ou organiques ou de ses sels avec des acides organiques optiquement actifs, caractérisé en ce que :

a) on transforme un composé, sous forme racémique, sous forme d'un mélange de diastéréoisomères ou sous forme énantiomériquement pure, de formule :

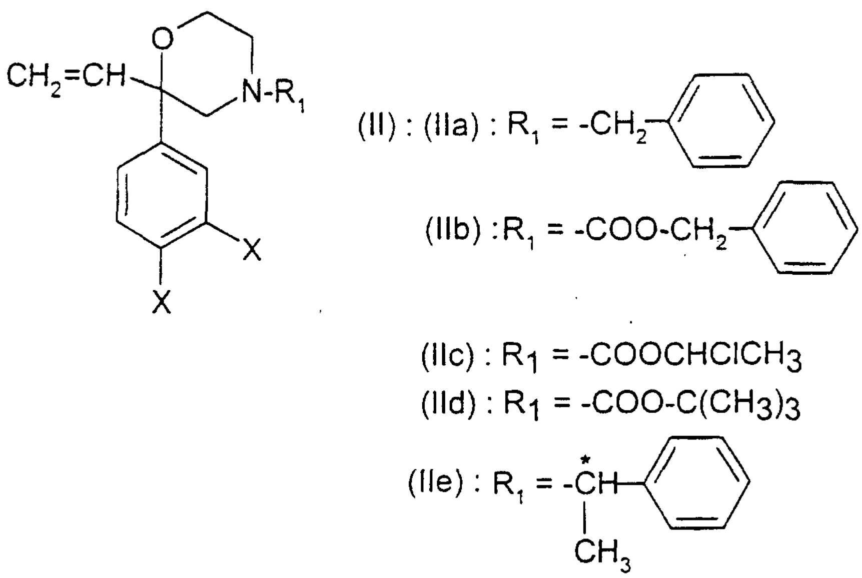

(II) : (IIa) : $R_1$ =

(IIb) : $R_1$ =

(IIc) : $R_1$ = $-COOCHClCH_3$

(IId) : $R_1$ = $-COO-C(CH_3)_3$

(IIe) : $R_1$ =

dans laquelle X est tel que défini pour un composé de formule (I) et $R_1$ représente un groupe N-protecteur choisi parmi un groupe benzyle, un groupe benzyloxycarbonyle, un groupe 1-chloroéthyloxycarbonyle, un groupe *tert*-butyloxycarbonyle ou un groupe α-méthylbenzyle, en un composé, sous forme racémique, sous forme d'un mélange de diastéréoisomères ou sous forme énantiomériquement pure, de formule :

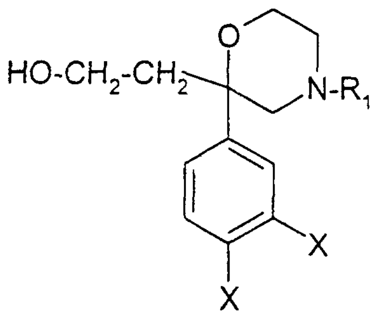

(III) : (IIIa) : $R_1$ = 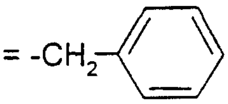

(IIIb) : $R_1$ = 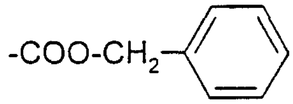

(IIIc) : $R_1$ = $-COOCHClCH_3$

(IIId) : $R_1$ = $-COO-C(CH_3)_3$

(IIIe) : $R_1$ = 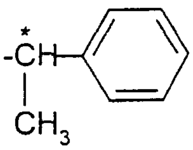

b) on déprotège le composé de formule (III) ainsi obtenu ;

c) le cas échéant, lorsque le composé de formule (I) ainsi obtenu est sous forme racémique, on sépare les énantiomères, et, éventuellement, on transforme le composé de formule (I), énantiomériquement pur, en l'un de ses sels avec des acides minéraux ou organiques.

De préférence, dans le procédé A, $R_1$ représente un groupe benzyle ou un groupe benzyloxycarbonyle.

Lorsque X représente un atome de chlore, de préférence, dans le procédé A, $R_1$ représente un groupe *tert*-butyloxycarbonyle ou un groupe 1-chloroéthyloxycarbonyle.

Lorsque, dans le procédé A selon l'invention, on utilise aux étapes a) et b) des réactions non racémisantes et conservatrices de la chiralité, on prépare directement un composé de formule (I) énantiomériquement pur, en utilisant comme composé de départ, un composé de formule (II) énantiomériquement pur.

A l'étape a) du procédé A, on transforme un composé de formule (II) en un composé de formule (III) selon les méthodes classiques bien connues de l'homme de l'art.

De préférence, à l'étape a), on soumet un composé de formule (II) d'abord à une réaction d'hydroboration puis à une réaction d'oxydation pour obtenir un composé de formule (III).

La réaction d'hydroboration d'un alcène dissymétrique de formule (II), puis la réaction d'oxydation in situ de l'organoborane formé intermédiairement pour donner l'alcool primaire de formule (III) s'effectuent selon les méthodes classiques, telles que celles décrites dans J. Am. Chem. Soc., 1974, 96 (25), 7765-7770 ou dans J. Am. Chem. Soc., 1960, 82, 4708-4712.

Les agents d'hydroboration utilisés, bien connus de l'homme de l'art, sont par exemple, soit des complexes du borane tels que, le complexe borane-tétrahydrofurane ou le complexe borane-diméthylsulfure, soit le 9-borabicyclo [3.3.1]nonane ou 9-BBN. Le borane utilisé peut également être généré in situ, selon des méthodes classiques, à partir par exemple de borohydrure de sodium ou de borohydrure de lithium et d'un acide tel qu'un acide de Lewis.

De préférence, on utilise le complexe borane-tétrahydrofurane, le 9-borabicyclo[3.3.1]nonane ou le borane généré in-situ par action du chlorure de triméthylsilyle sur le borohydrure de sodium.

Lorsqu'on utilise le complexe borane-tétrahydrofurane, ce dernier intervient dans la réaction à raison de 0,3 à 1,5 équivalents molaires par équivalent molaire de composé de formule (II).

Lorsqu'on utilise le 9-borabicyclo[3.3.1]nonane, ce dernier intervient dans la réaction à raison de 1 à 1,5 équivalents molaires par équivalent molaire de composé de formule (II).

Lorsqu'on génère le borane in-situ par action du chlorure de triméthylsilyle sur le borohydrure de sodium, ces derniers sont utilisés à raison de 3 à 5 équivalents molaires chacun par équivalent molaire de composé de formule (II).

La réaction d'hydroboration s'effectue dans un solvant inerte comme un éther tel que l'éther diéthylique, le tétrahydrofurane, le dioxane, le diméthoxyéthane ou le diéthylène glycol diméthyl éther, ou comme un hydrocarbure aromatique tel que le toluène ou le xylène, à une température comprise entre la température ambiante et la température de reflux du solvant et pendant un temps compris entre 5 et 48 heures.

L'organoborane formé intermédiairement est ensuite soumis à une réaction d'oxydation classique. De manière préférentielle, on effectue la réaction d'oxydation dans des conditions de catalyse par transfert de phase en utilisant un peroxyde, en présence d'une base forte et d'un catalyseur de transfert de phase dans un solvant inerte et de l'eau.

Comme peroxyde on préfère utiliser le peroxyde d'hydrogène. Ce dernier intervient dans la réaction à raison de 3 à 5 équivalents molaires par équivalent molaire de composé de formule (II).

La base utilisée lors de la réaction est choisie parmi un hydroxyde de métal alcalin tel que l'hydroxyde de sodium. Elle intervient dans la réaction à raison de 1 à 2 équivalents molaires par équivalent molaire de composé de formule (II).

Le catalyseur de transfert de phase est choisi parmi les sels d'ammonium quaternaires substitués tel que l'hydrogénosulfate de tétrabutylammonium. Il intervient dans la réaction à raison de 0,01 à 0,1 équivalent molaire par équivalent molaire de composé de formule (II).

La réaction d'oxydation est conduite dans l'un des solvants précités ci-dessus pour la réaction d'hydroboration et à une température comprise entre 0°C et 60°C.

Cette réaction d'oxydation est très exothermique et nécessite un contrôle du débit d'introduction du peroxyde ainsi que de la température du milieu réactionnel.

La réaction se déroule pendant un temps compris entre la durée d'introduction de la solution de peroxyde d'hydrogène et 48 heures.

Le mélange peroxyde d'hydrogène/tétrahydrofurane étant considéré comme dangereux au stade industriel, il est préférable d'effectuer la réaction d'oxydation dans un solvant aromatique, de préférence le toluène. Dans ce cas, un échange de solvant peut être nécessaire avant la réaction d'oxydation ou, préférentiellement, avant la réaction d'hydroboration.

A l'étape b) du procédé A, on déprotège le composé de formule (III) ainsi obtenu selon les méthodes classiques.

Ainsi, la déprotection du groupe benzyle du composé de formule (IIIa), la déprotection du groupe benzyloxycarbonyle du composé de formule (IIIb) ou la déprotection du groupe α-méthylbenzyle du composé de formule (IIIe) s'effectue par hydrogénolyse, de préférence par hydrogénation catalytique ou par catalyse par transfert d'hydrogène.

L'hydrogénation catalytique s'effectue dans un solvant inerte tel qu'un alcool (le méthanol, l'éthanol ou le propan-2-ol par exemple), un hydrocarbure aromatique (toluène ou xylène, par exemple), un ester (l'acétate d'éthyle par exemple), ou dans un mélange de ces solvants précités, en présence d'un catalyseur tel que le palladium sur charbon, le nickel de Raney, sous une pression comprise entre 1 et 10 bars, à une température comprise entre 0°C et 100°C et pendant un temps compris entre 1 et 24 heures.

La catalyse par transfert d'hydrogène s'effectue en utilisant le formiate d'ammonium, en présence d'un catalyseur tel que le palladium sur charbon, dans un solvant inerte tel qu'un alcool (le méthanol ou l'éthanol par exemple), à une température comprise entre la température ambiante et la température de reflux du solvant et pendant un temps compris entre 2 et 48 heures.

La déprotection du groupe 1-chloroéthyloxycarbonyle du composé de formule (IIIc) s'effectue par réaction avec le méthanol à une température comprise entre la température ambiante et la température de reflux du solvant.

La déprotection du groupe *tert*-butyloxycarbonyle du composé de formule (IIId) s'effectue par hydrolyse acide au moyen d'acide chlorhydrique ou d'acide trifluoroacétique par exemple, dans un solvant inerte tel qu'un alcool (le méthanol ou l'éthanol par exemple), un éther (l'éther diéthylique, le dioxane ou le tétrahydrofurane par exemple), un hydrocarbure halogéné (le dichlorométhane par exemple) et à une température comprise entre -10°C et la température de reflux du solvant.

Lorsque le composé de formule (I) est obtenu sous forme racémique, on sépare, à l'étape c), les énantiomères selon les méthodes connues. De préférence, la séparation est effectuée par préparation d'un sel optiquement actif, par action d'un acide organique optiquement actif tel que l'acide L-(+) ou D-(-)mandélique, l'acide L-(-) ou D-(+)-di-paratoluoyltartrique, l'acide L-(+) ou D-(-)-tartrique, l'acide L-(-) ou D-(+)-dibenzoyltartrique ou l'acide (1R)-(-) ou (1S)-(+)-10-camphorsulfonique, puis séparation des isomères, par exemple, par cristallisation. L'énantiomère recherché est libéré de son sel en milieu basique.

De préférence, on effectue la séparation des énantiomères du composé de formule (I) par formation d'un sel optiquement actif, par action de l'acide L-(-) ou D-(+)-di-para-toluoyltartrique.

Les composés de formule (I), énantiomériquement purs, sous forme de sels optiquement actifs avec des acides organiques optiquement actifs sont nouveaux et font partie de l'invention.

Les composés de formule (I), énantiomériquement purs, sous forme de sels optiquement actifs avec des acides organiques optiquement actifs, dans laquelle X représente un atome de chlore ou un atome de fluor sont préférés.

Les composés de formule (I), énantiomériquement purs, sous forme de sels avec l'acide L-(-) ou D-(+)-di-paratoluoyltartrique sont préférés.

Les composés de formule (II), sous forme énantiomériquement pure, sous forme de racémique ou sous forme d'un mélange de diastéréoisomères, et leurs sels éventuels avec des acides minéraux ou organiques sont nouveaux et font partie de l'invention.

Les composés de formule (II) dans laquelle X représente un atome de chlore ou un atome de fluor sont

préférés.

Les composés de formule (II) dans laquelle $R_1$ représente un groupe benzyle ou un groupe benzyloxycarbonyle sont préférés.

Selon un autre de ses aspects, l'invention a pour objet un procédé B de préparation d'un composé, sous forme énantiomériquement pure ou sous forme racémique, de formule

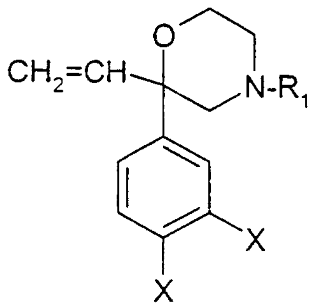

(II) : (IIb) : $R_1$ = -COO-$CH_2$- 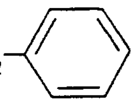

(IIc) : $R_1$ = -COOCHCl$CH_3$

dans laquelle X représente un atome d'halogène et $R_1$ représente un groupe benzyloxycarbonyle ou un groupe 1-chloroéthyloxycarbonyle,
caractérisé en ce qu'on fait réagir un composé, sous forme énantiomériquement pure ou sous forme racémique, de formule :

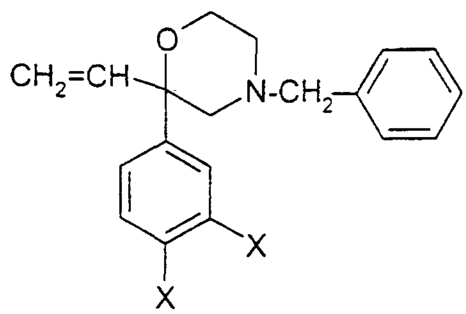

(IIa)

dans laquelle X est tel que défini pour un composé de formule (II), avec du chloroformiate de benzyle ou du chloroformiate de 1-chloroéthyle, en présence d'une base, avec ou sans solvant.

De préférence, dans le procédé B, $R_1$ représente un groupe benzyloxycarbonyle.

Le chloroformiate de benzyle ou le chloroformiate de 1-chloroéthyle est utilisé dans la réaction à raison de 1 à 1,5 équivalents molaires par équivalent molaire de composé de formule (IIa).

La base utilisée dans la réaction est choisie parmi les bases organiques telles que la triéthylamine, la N,N-diisopropyléthylamine ou la N-méthylmorpholine ou parmi les carbonates ou bicarbonates de métal alcalin tels que le carbonate de sodium, le carbonate de potassium ou le bicarbonate de sodium.

La base est utilisée dans la réaction à raison de 0,01 à 1,5 équivalents molaires par équivalent molaire de composé de formule (IIa).

Lorsque la réaction est effectuée dans un solvant celui-ci est choisi parmi un hydrocarbure aromatique tel que le toluène ou le xylène, un hydrocarbure halogéné tel que le dichlorométhane, le 1,2-dichloroéthane, le tétrachlorure de carbone, le chlorobenzène ou le dichlorobenzène, un éther tel que le tétrahydrofurane, le dioxane ou le diméthoxyéthane, un ester tel que l'acétate d'éthyle, un amide tel que le N,N-diméthylformamide, un nitrile tel que l'acétonitrile ou une cétone telle que l'acétone.

La réaction s'effectue à une température comprise entre -20°C et la température de reflux du solvant et pendant un temps compris entre 1 à 24 heures.

Selon un autre de ses aspects, l'invention, a pour objet un procédé C de préparation d'un composé, sous forme énantiomériquement pure, sous forme d'un mélange de diastéréoisomères ou sous forme racémique, de formule :

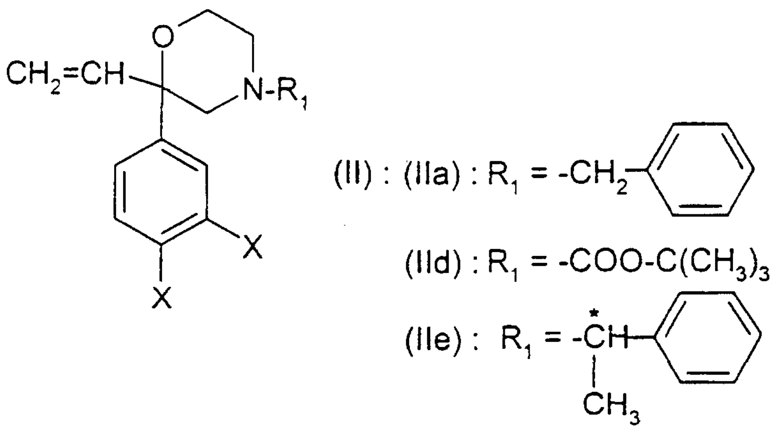

(II) : (IIa) : $R_1 = -CH_2-$

(IId) : $R_1 = -COO-C(CH_3)_3$

(IIe) : $R_1 = -\overset{*}{C}H-$ $CH_3$

dans laquelle X représente un atome d'halogène et $R_1$ représente un groupe benzyle, un groupe *tert*-butyloxycarbonyle ou un groupe α-méthylbenzyle, de ses sels éventuels avec des acides minéraux ou organiques, caractérisé en ce que on cyclise un composé, sous forme énantiomériquement pure, sous forme d'un mélange de diastéréoisomères ou sous forme de racémique, de formule :

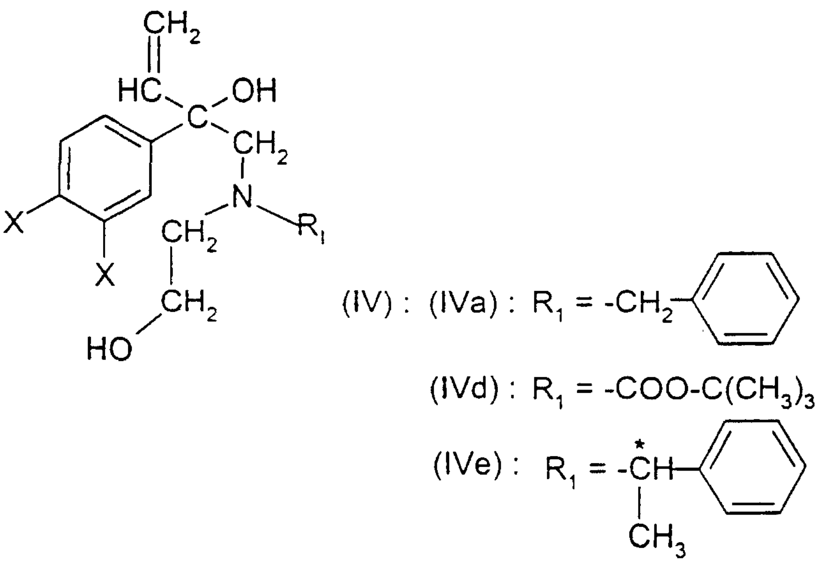

(IV) : (IVa) : $R_1 = -CH_2-$

(IVd) : $R_1 = -COO-C(CH_3)_3$

(IVe) : $R_1 = -\overset{*}{C}H-$ $CH_3$

dans laquelle X et $R_1$ sont tels que définis pour un composé de formule (II), et, éventuellement on transforme le composé de formule (II) ainsi obtenu en un de ses sels.

De préférence, dans le procédé C, $R_1$ représente un groupe benzyle.

La réaction de cyclisation d'un diol de formule (IV) en un dérivé morpholinique de formule (II) peut s'effectuer selon des méthodes connues telles que décrites, par exemple, dans J. Med. Chem., 1994, 37, 2791-2796.

De façon préférentielle, on effectue la réaction de cyclisation dans des conditions de catalyse par transfert de phase en utilisant un halogénure d'alkyle ou d'aryle sulfonyle, en présence d'une base forte et d'un catalyseur de transfert de phase, dans un solvant inerte en mélange avec de l'eau.

La réaction de l'alcool primaire du composé de formule (IV) avec un halogénure d'alkyle ou d'aryle sulfonyle, en présence d'une base forte, permet d'abord de former un alkyle ou aryle sulfonate ester qui, dans les conditions réactionnelles, se cyclise in-situ, pour former le cycle morpholinique.

On a trouvé que lorsqu'on effectue la réaction de cyclisation d'un composé de formule (IV) énantiomériquement pur dans les conditions précitées, on obtient un composé de formule (II) énantiomériquement pur dont l'atome de carbone asymétrique possède la même configuration.

Parmi les halogénures d'alkyle ou d'aryle sulfonyle on préfère le chlorure de méthanesulfonyle, le chlorure de benzènesulfonyle ou le chlorure de *p*-toluènesulfonyle.

L'halogénure d'alkyle ou d'aryle sulfonyle intervient dans la réaction à raison de 1 à 1,5 équivalents molaires

par équivalent molaire de composé de formule (IV).

La base utilisée lors de la réaction est choisi parmi un hydroxyde de métal alcalin tel que l'hydroxyde de sodium ou l'hydroxyde de potassium.

La base intervient dans la réaction à raison de 5 à 10 équivalents molaires par équivalent molaire de composé de formule (IV).

Le catalyseur de transfert de phase est choisi parmi les sels d'ammonium quaternaires substitués tel que le chlorure de benzyltriéthylammonium.

Le catalyseur intervient dans la réaction à raison de 0,01 à 0,1 équivalent molaire par équivalent molaire de composé de formule (IV).

La réaction s'effectue dans un solvant inerte comme un hydrocarbure aromatique tel que le toluène ou le xylène.

La réaction est conduite à une température comprise entre la température ambiante et 60°C et se déroule sur une période de 1 à 24 heures.

Le composé de formule (IV), sous forme énantiomériquement pure, sous forme d'un mélange de diastéréoisomères ou sous forme de racémique, et ses sels éventuels avec des acides minéraux ou organiques sont nouveaux et font partie de l'invention.

Le composé de formule (IV) dans laquelle X représente un atome de chlore ou un atome de fluor est préféré.

Le composé de formule (IV) dans laquelle $R_1$ représente un groupe benzyle est préféré.

Selon un autre de ses aspects, l'invention a pour objet un procédé D de préparation d'un composé, sous forme énantiomériquement pure, de formule

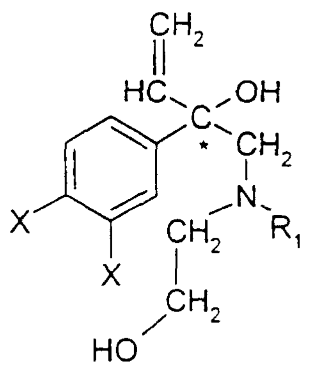

(IV) : (IVa) : 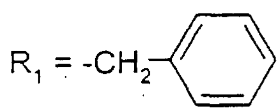

(IVe) : 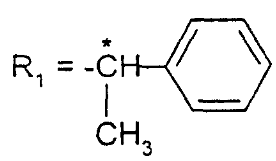

dans laquelle X représente un atome d'halogène et $R_1$ représente un groupe benzyle ou un groupe α-méthylbenzyle, de ses sels avec des acides minéraux ou organiques, caractérisé en ce que :

a) on fait réagir un composé, sous forme racémique, de formule :

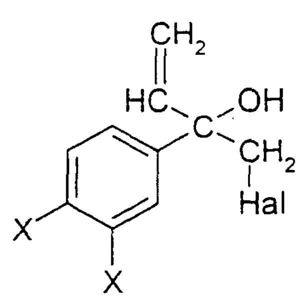

(V)

dans laquelle X est tel que défini pour un composé de formule (IV) et Hal représente un atome d'halogène, avec la benzylamine ou avec la R(+) ou la S(-)-α-méthylbenzylamine, en présence d'une base, dans un solvant inerte, pour obtenir un composé, sous forme racémique ou sous forme d'un mélange de diastéréoisomères, de formule :

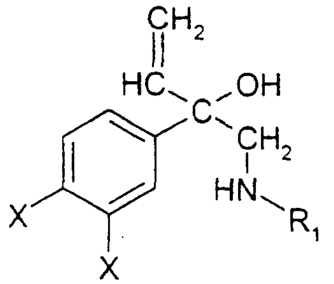

(VI) : (VIa) : 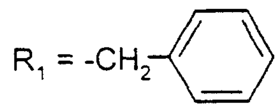

(VIe) : 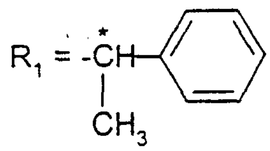

b) on sépare les énantiomères ou les diastéréoisomères du composé de formule (VI) ainsi obtenu ;
c) on fait réagir le composé de formule (VI), énantiomériquement pur :

- soit avec de l'oxyde d'éthylène, en présence catalytique d'un acide, dans un solvant inerte ;
- soit avec un composé de formule Hal""-$CH_2$-$CH_2$-O-$R_2$ (XXI) dans laquelle $R_2$ représente un groupe O-protecteur et Hal"" représente un atome d'halogène, en présence d'une base, dans un solvant inerte, suivi de la déprotection du groupe O-protecteur :

et éventuellement, on transforme le composé de formule (IV), énantiomériquement pur, en l'un de ses sels avec des acides minéraux ou organiques.

De préférence, dans le procédé D, $R_1$ représente un groupe benzyle.
De préférence, à l'étape a) du procédé D, on utilise un composé de formule (V) dans laquelle Hal représente un atome de chlore ou de brome.
A l'étape a) du procédé D, le composé de formule (V) est d'abord transformé en un dérivé époxyde intermédiaire de formule :

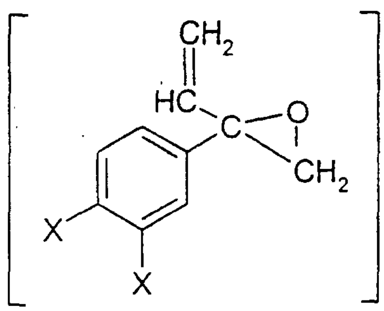

(VII)

qui, dans les conditions réactionnelles, réagit avec l'amine pour donner le composé de formule (VI).
L'amine intervient dans la réaction à raison de 1 à 1,5 équivalents molaires par équivalent molaire de composé de formule (V).
La base utilisée dans la réaction est choisie parmi les carbonates ou bicarbonates de métal alcalin tel que le carbonate de sodium, le carbonate de potassium ou le bicarbonate de sodium. De préférence, on utilise le bicarbonate de sodium.
La base intervient dans la réaction à raison de 1 à 2 équivalents molaires par équivalent molaire de composé de formule (V).
Le solvant inerte est choisi parmi les solvants polaires tels que l'acétonitrile, le propionitrile ou la 1-méthyl-2-pyrrolidinone. Le propionitrile constitue un solvant préféré.
La réaction est conduite à une température comprise entre 80°C et 120°C.
La réaction s'effectue sur une période de 5 à 24 heures.
A l'étape b) du procédé D, on sépare les énantiomères du composé de formule (VIa) ainsi obtenu selon les méthodes classiques. De préférence, la séparation peut être effectuée par préparation d'un sel optiquement actif, par action d'un acide organique optiquement actif tel que l'acide L-(+) ou D-(-)-mandélique, l'acide L-(-) ou D-(+)-di-para-

toluoyltartrique, l'acide L-(+) ou D-(-)-tartrique, l'acide L-(-) ou D-(+)-dibenzoyltartrique, puis séparation des isomères, par exemple, par cristallisation. L'énantiomère recherché est libéré de son sel en milieu basique.

De préférence, on effectue la séparation des énantiomères du composé de formule (VIa) par formation d'un sel optiquement actif, par action de l'acide L-(+) ou D-(-)-mandélique.

A l'étape b) du procédé D, on sépare les diastéréoisomères du composé de formule (VIb) ainsi obtenu selon les méthodes classiques. De préférence, la séparation peut être effectuée par préparation d'un sel avec un acide minéral ou organique, ou avec un acide organique optiquement actif tel que ceux précédemment cités, puis séparation des diastéréoisomères, par exemple, par cristallisation. Le diastéréoisomère recherché peut être libéré de son sel en milieu basique.

A l'étape c) du procédé D, la réaction du composé de formule (VI), énantiomériquement pur, avec l'oxyde d'éthylène est mise en oeuvre dans un réacteur de type hydrogénateur, puisqu'aux températures utilisées l'oxyde d'éthylène est sous forme gazeuse.

L'oxyde d'éthylène intervient dans la réaction à raison de 5 à 15 équivalents molaires par équivalent molaire de composé de formule (VI).

Le solvant peut être par exemple un alcool tel que le méthanol.

L'acide utilisé en quantité catalytique dans la réaction est choisi parmi les acides minéraux ou organiques classiques tel que l'acide chlorhydrique ou l'acide acétique.

On a trouvé que l'on peut effectuer la réaction entre le composé de formule (VI) et l'oxyde d'éthylène sans catalyse acide en utilisant comme composé de départ le composé de formule (VI) énantiomériquement pur sous forme de sel avec un acide optiquement actif, de préférence avec l'acide L-(+) ou D(-)-mandélique.

La réaction est conduite à une température comprise entre 0°C et 60°C et s'effectue pendant un temps compris entre 2 et 24 heures.

Selon l'autre alternative de l'étape c) du procédé D, le groupe protecteur $R_2$ est choisi parmi les groupe O-protecteurs classiques bien connus de l'homme de l'art tel que le groupe tétrahydropyran-2-yle.

De préférence, on utilise un composé de formule (XXI) dans laquelle Hal"" représente un atome de chlore ou de brome.

Le composé de formule (XXI) intervient dans la réaction à raison de 1 à 2 équivalents molaires par équivalent molaire du composé de formule (VI).

La base utilisée dans la réaction est choisie parmi les carbonates ou bicarbonates de métal alcalin tel que le carbonate de sodium, le carbonate de potassium ou le bicarbonate de sodium.

La base intervient dans la réaction à raison de 1 à 2 équivalents molaires par équivalent molaire du composé de formule (VI).

Le solvant inerte est choisi parmi les solvants polaires tels que l'acétonitrile, le propionitrile ou la 1-méthyl-2-pyrrolidone.

La réaction est conduite à une température comprise entre 80°C et 120°C et s'effectue sur une période de 5 à 24 heures.

La déprotection du groupe O-protecteur $R_2$ s'effectue selon les méthodes connues de l'homme de l'art. Par exemple, lorsque $R_2$ représente un groupe tétrahydropyran-2-yle, la déprotection s'effectue par hydrolyse acide en utilisant un acide tel que l'acide chlorhydrique.

La réaction s'effectue dans un solvant éthéré tel que l'éther diéthylique ou dans un alcool tel que le méthanol ou dans un solvant aromatique tel que le toluène, à une température comprise entre 0°C et la température de reflux du solvant et pendant un temps compris entre 1 et 24 heures.

Le composé de formule (VI), sous forme racémique, sous forme d'un mélange de diastéréoisomères ou sous forme énantiomériquement pure, et ses sels avec des acides minéraux ou organiques ou ses sels avec des acides optiquement actifs, sont nouveaux et font partie de l'invention.

Le composé de formule (VIa), énantiomériquement pur, sous forme de sel avec l'acide L-(+) ou D-(-)-mandélique est préféré.

Le composé de formule (VI) dans laquelle X représente un atome de chlore ou de fluor est préféré.

Le composé de formule (VI) dans laquelle $R_1$ représente un groupe benzyle est préféré.

Le composé de formule (XXI) se prépare par protection de dérivés 2-halogénoéthanol selon les méthodes classiques telles que celles décrites dans Protective Groups in Organic Chemistry, J.F.W. McOmie, Ed. Plenum Press, 1973 et dans Protective Groups in Organic Synthesis, T.W. Greene et P.G.M. Wutts, Ed. John Wiley et Sons, 1991.

Selon un autre de ses aspects, l'invention a pour objet un procédé E de préparation d'un composé, sous forme racémique ou sous forme d'un mélange de diastéréoisomères, de formule :

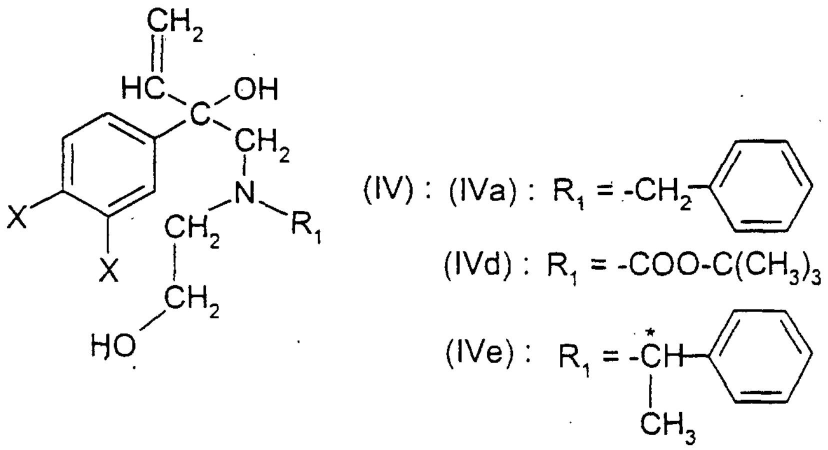

(IV) : (IVa) :

(IVd) : $R_1$ = -COO-C($CH_3$)$_3$

(IVe) :

dans laquelle X représente un atome d'halogène et $R_1$ représente le groupe benzyle, le groupe tert-butyl-oxycarbonyle ou le groupe α-méthylbenzyle, de ses sels éventuels avec des acides minéraux ou organiques, caractérisé en ce que :

a) on fait réagir un composé, sous forme racémique, de formule :

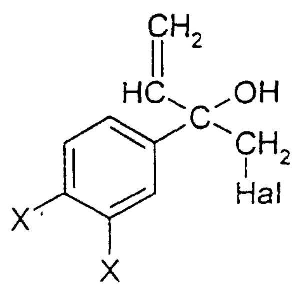

(V)

dans laquelle X est tel que défini pour un composé de formule (IV) et Hal représente un atome d'halogène, soit avec du 2-(benzylamino)-1-éthanol, soit avec du 2-amino-1-éthanol ou soit avec du (R) ou (S)-2-(α-méthylbenzylamino)-1-éthanol, en présence d'une base et dans un solvant inerte, et, éventuellement on transforme le composé de formule (IVa) ou (IVe) ainsi obtenu en un de ses sels avec des acides minéraux ou organiques.

b) le cas échéant, lorsqu'à l'étape a), on met en oeuvre le composé de formule (V) avec du 2-amino-1-éthanol, on traite le composé ainsi obtenu de formule :

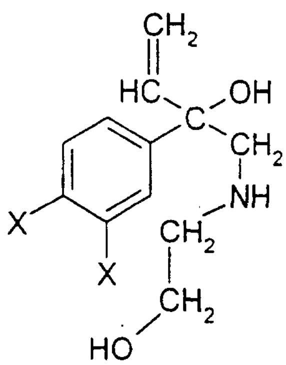

(IV'd)

avec du di-*tert*-butyldicarbonate, en présence d'une base et dans un solvant inerte pour obtenir le composé de formule (IVd).

De préférence, dans le procédé E, $R_1$ représente un groupe benzyle.

De préférence, à l'étape a) du procédé E, on utilise un composé de formule (V) dans laquelle Hal représente

un atome de chlore ou de brome.

Comme à l'étape a) du procédé D, le composé de formule (V) est d'abord transformé en dérivé époxyde intermédiaire de formule (VII) qui, dans les conditions réactionnelles, réagit in-situ avec le dérivé aminoéthanol pour donner le composé de formule (IVa), (IVe) ou (IV'd).

Le dérivé aminoéthanol intervient dans la réaction à raison de 1 à 1,5 équivalents molaires par équivalent molaire de composé de formule (V).

La base utilisée dans la réaction est choisie parmi les carbonates ou bicarbonates de métal alcalin tels que le carbonate de sodium, le carbonate de potassium ou le bicarbonate de sodium. De préférence, on utilise le bicarbonate de sodium.

La base intervient à raison de 1 à 2 équivalents molaires par équivalent molaire de composés de formule (V).

Le solvant inerte est choisi parmi les solvants polaires tels que l'acétonitrile, le propionitrile ou la 1-méthyl-2-pyrrolidinone. La 1-méthyl-2-pyrrolidinone constitue un solvant préféré.

La réaction est conduite à une température comprise entre 80 et 120°C et pendant un temps compris entre 10 et 24 heures.

Le cas échéant, à l'étape b) du procédé E, on fait réagir le composé de formule (IV'd) avec du di-*tert*-butyldicarbonate, en présence d'une base organique telle que la triéthylamine, la N,N-diisopropyléthylamine ou la N-méthylmorpholine, dans un solvant inerte tel que le dichlorométhane, le tétrahydrofurane ou le N,N-diméthylformamide et à une température comprise entre 0°C et 60°C.

La (R) ou (S)-2-(α-méthylbenzylamino)-1-éthanol se prépare selon la méthode décrite dans J. Am. Chem. Soc., 1984, 106, 747-754.

Les composés de formule (V) sont nouveaux et font partie de l'invention.

Le composé de formule (V) dans laquelle X représente un atome de chlore ou un atome de fluor et Hal représente un atome de chlore ou de brome est préféré.

Selon un autre de ses aspects, l'invention à pour objet un procédé F de préparation d'un composé de formule :

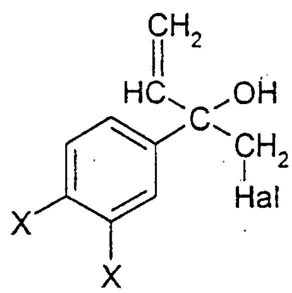
(V)

dans laquelle X représente un atome d'halogène et Hal représente un atome d'halogène, caractérisé en ce que :

a) on fait réagir un composé de formule :

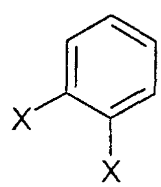
(VIII)

dans laquelle X est tel que défini pour un composé de formule (V), avec un composé de formule :

$$Hal'\text{-}CO\text{-}CH_2\text{-}Hal \qquad (IX)$$

dans laquelle Hal' et Hal représentent un atome d'halogène, en présence d'un acide de Lewis et dans un solvant inerte, pour obtenir un composé de formule :

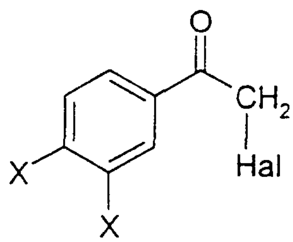

(X)

b) on fait réagir le composé de formule (X) ainsi obtenu avec un composé de formule :

$$CH_2 = CH\text{-}Mg\text{-}Hal'' \qquad (XI)$$

dans laquelle Hal" représente un atome d'halogène, dans un solvant inerte, suivi d'une hydrolyse et on obtient le composé de formule (V) attendu.

Selon un aspect préférentiel, l'invention se rapporte à la préparation d'un composé de formule (V) dans laquelle Hal représente un atome de chlore ou de brome.

De préférence, à l'étape a) on utilise un composé de formule (IX) dans laquelle Hal' et Hal représentent chacun indépendamment un atome de chlore ou de brome et à l'étape b) on utilise un composé de formule (XI) dans laquelle Hal" représente un atome de chlore ou de brome.

L'étape a) du procédé F est une réaction de Friedel et Craft effectuée dans des conditions de Perrier selon les méthodes classiques.

L'acide de Lewis est choisi parmi les acides de Lewis classiques, de préférence on utilise le chlorure d'aluminium.

L'acide de Lewis est utilisé dans la réaction à raison de 1 à 1,5 équivalents molaires par équivalent molaire de composé de formule (VIII).

Parmi les halogénures d'halogénoacétyle de formule (IX), on préfère utiliser le chlorure de chloroacétyle.

Le composé de formule (IX) est utilisé à raison de 1 à 1,5 équivalents molaires par équivalent molaire de composé de formule (VIII).

Le solvant est choisi parmi les hydrocarbures aromatiques tels que le toluène ou le xylène, les hydrocarbures chlorés tels que le dichlorométhane, le dichloroéthane, le tétrachlorure de carbone, le chlorobenzène ou le dichlorobenzène, les éthers tels que le tétrahydrofurane, le dioxane ou le diméthoxyéthane. De préférence, on utilise le dichlorométhane.

La réaction s'effectue à une température comprise entre 0°C et 100°C et pendant un temps compris entre 1 et 24 heures.

L'étape b) du procédé F est une réaction de Grignard effectuée selon les méthodes classiques.

Le composé de formule (XI) est utilisé dans la réaction à raison de 1 à 1,5 équivalents molaires par équivalent molaire de composé de formule (X).

Le solvant est choisi parmi les éthers tels que l'éther diéthylique, l'éther diisopropylique, le tétrahydrofurane ou le dioxane. De préférence, on utilise le tétrahydrofurane.

La réaction s'effectue à une température comprise entre -20°C et 0°C et pendant un temps compris entre 1 et 24 heures.

A la fin de la réaction, on hydrolyse le mélange réactionnel selon les méthodes classiques, en le versant, par exemple, sur une solution saturée de chlorure d'ammonium.

Selon un autre de ses aspects, l'invention a pour objet un procédé G de préparation d'un composé, sous forme racémique, de formule :

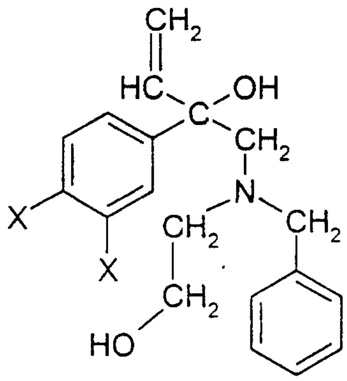

(IVa)

dans laquelle X représente un atome d'halogène, de ses sels avec des acides minéraux ou organiques, caractérisé en ce que :

a) on procède comme à l'étape a) du procédé F ;
b) on fait réagir le composé de formule (X) ainsi obtenu, avec un composé de formule :

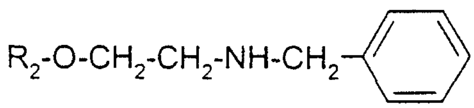

(XII)

dans laquelle $R_2$ représente un groupe O-protecteur, en présence d'une base et dans un solvant inerte, pour obtenir un composé de formule :

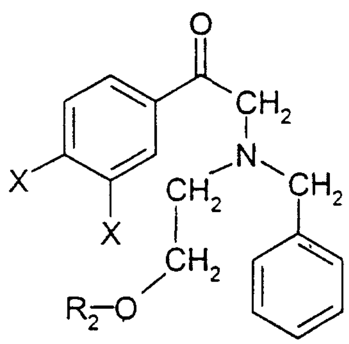

(XIII)

c) on fait réagir le composé de formule (XIII) ainsi obtenu avec un composé de formule

$$CH_2{=}CH\text{-}Mg\text{-}Hal'' \qquad (XI)$$

dans laquelle Hal" représente un atome d'halogène, dans un solvant inerte, suivi d'une hydrolyse, pour obtenir un composé de formule :

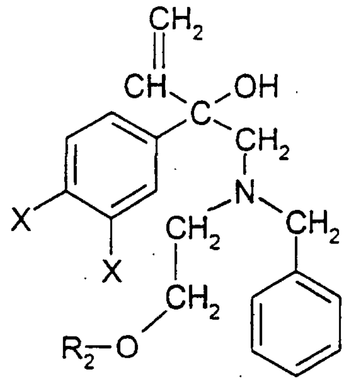

(XIV)

d) on déprotège le composé de formule (XIV) et, éventuellement, on transforme le composé de formule (IVa) ainsi obtenu en un de ses sels avec des acides minéraux ou organiques.

De préférence, à l'étape a) on utilise un composé de formule (IX) dans laquelle Hal' et Hal représentent chacun indépendamment un atome de chlore ou de brome.

A l'étape b) du procédé G, le groupe protecteur $R_2$ est choisi parmi les groupes O-protecteur classiques bien connus de l'homme de l'art tels que le groupe tétrahydropyran-2-yle.

Le composé de formule (XII) intervient dans la réaction à raison de 1 à 1,5 équivalents molaires par équivalent molaire de composé de formule (X).

La base est choisie parmi les carbonates ou bicarbonates de métal alcalin, de préférence le bicarbonate de sodium.

La base est utilisée à raison de 1 à 1,5 équivalents molaires par équivalent molaire de composé de formule (X).

Le solvant inerte est choisi parmi les solvants polaires tels que l'acétonitrile ou le propionitrile, ou les éthers tel que le tétrahydrofurane, ou les solvants halogénés tel que le dichlorométhane. De préférence, on utilise le tétrahydrofurane.

La réaction s'effectue à une température comprise entre la température ambiante et la température de reflux du solvant et pendant un temps compris entre 1 et 24 heures.

L'étape c) du procédé G est une réaction de Grignard effectuée selon les méthodes classiques.

De préférence, à l'étape c) on utilise un composé de formule (XI) dans laquelle Hal" représente un atome de chlore ou de brome.

Le composé de formule (XI) est utilisé dans la réaction à raison de 1,5 à 2 équivalents molaires par équivalent molaire de composé de formule (XIII).

Le solvant est choisi parmi les éthers tels que l'éther diéthylique, l'éther diisopropylique, le tétrahydrofurane ou le dioxane. De préférence, on utilise le tétrahydrofurane.

La réaction s'effectue à une température comprise entre la température ambiante et la température de reflux du solvant et se déroule pendant un temps compris entre 1 et 24 heures.

A la fin de la réaction, on hydrolyse le mélange réactionnel en le versant, par exemple, sur une solution saturée de chlorure d'ammonium.

Le composé de formule (XIV) est déprotégé à l'étape d) du procédé G selon les méthodes connues de l'homme de l'art. Par exemple, lorsque $R_2$ représente un groupe tétrahydropyran-2-yle, la déprotection s'effectue par hydrolyse acide en utilisant un acide tel que l'acide chlorhydrique. Ce dernier peut être généré in-situ à partir de chlorure d'acétyle et de méthanol.

La réaction s'effectue dans un solvant éthéré tel que l'éther diéthylique ou un alcool tel que le méthanol, à une température comprise entre 0°C et la température de reflux du solvant et pendant un temps compris entre 1 et 24 heures.

Le composé de formule (XIII) et ses sels avec des acides minéraux ou organiques sont nouveaux et font partie de l'invention.

Le composé de formule (XIII) dans laquelle X représente un atome de chlore ou de fluor est préféré.

Le composé de formule (XIV) et ses sels avec des acides minéraux ou organiques sont nouveaux et font partie de l'invention.

Le composé de formule (XIV) dans laquelle X représente un atome de chlore ou de fluor est préféré.

Le composé de formule (XII) se prépare par protection du 2-(benzyl amino)-1-éthanol selon les méthodes classiques telles que celles décrites dans Protective Groups in Organic Chemistry, J.F.W. McOmie, Ed. Plenum Press, 1973 et dans Protective Groups in Organic Synthesis, T.W. Greene et P.G.M. Wutts, Ed. John Wiley et Sons, 1991.

Selon un autre de ses aspects, l'invention a pour objet un procédé énantiosélectif H de préparation d'un composé, sous forme énantiomériquement pure, de formule :

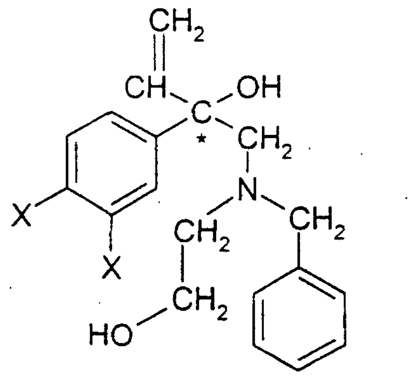

(IVa)

dans laquelle X représente un atome d'halogène, de ses sels avec des acides minéraux ou organiques, caractérisé en ce que :

a) on fait réagir un composé de formule :

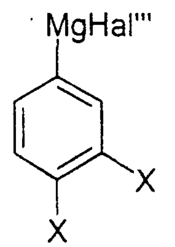

(XV)

dans laquelle X est tel que défini pour un composé de formule (IVa) et Hal''' représente un atome d'halogène, avec le (R)- ou le (S)-2-phénylhexahydro-pyrrolo[1,2-c]imidazole-3-carboxylate de méthyle, de formule :

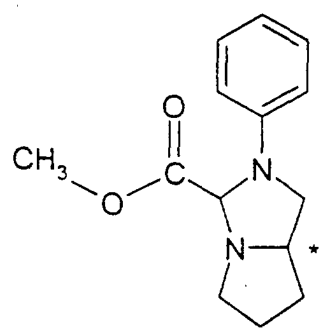

(XVI)

en présence de chlorure de magnésium, dans un solvant inerte, suivi d'une hydrolyse, pour obtenir un composé, sous forme énantiomériquement pure, de formule :

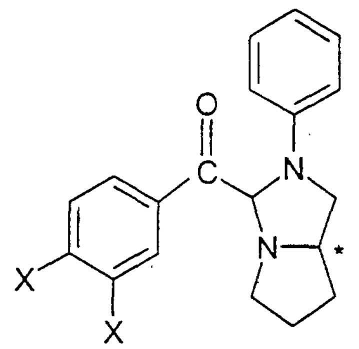

(XVII)

b) on fait réagir le composé de formule (XVII) ainsi obtenu avec un composé de formule :

$$CH_2 = CH\text{-}Mg\text{-}Hal'' \qquad (XI)$$

dans laquelle Hal" représente un atome d'halogène, dans un solvant inerte, suivi d'une hydrolyse, pour obtenir un composé, sous forme énantiomériquement pure, de formule :

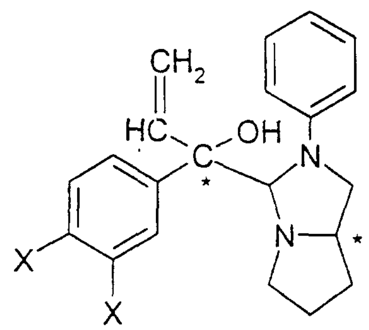

(XVIII)

c) on hydrolyse le composé de formule (XVIII) ainsi obtenu par action d'un acide, dans un solvant inerte en mélange avec de l'eau, pour obtenir un composé, sous forme énantiomériquement pure, de formule :

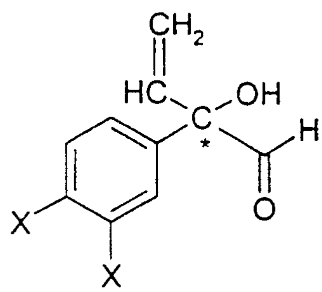

(XIX)

d) on fait réagir le composé de formule (XIX) ainsi obtenu avec du 2-(benzylamino)-1-éthanol en présence d'un acide, dans un solvant inerte, puis on réduit le sel d'iminium formé intermédiairement au moyen d'un agent réducteur, et, éventuellement, on transforme le composé de formule (IVa), énantiomériquement pur, en l'un de ses sels avec des acides minéraux ou organiques.

De préférence, à l'étape a) on utilise un composé de formule (XV) dans laquelle Hal"' représente un atome de chlore ou de brome et à l'étape b) on utilise un composé de formule (XI) dans laquelle Hal" représente un atome

de chlore ou de brome.

Les étapes a), b) et c) du procédé H, s'effectuent selon la méthode de synthèse asymétrique d'α-hydroxyaldéhydes décrite par T. Mukaiyama dans Tetrahedron, 1981, 37 (23), 4111-4119.

A l'étape d), on fait réagir un α-hydroxyaldéhyde de formule (XIX) avec du 2-(benzylamino)-1-éthanol, en présence d'un acide tel que l'acide acétique, dans un solvant polaire tel que l'acétonitrile, pour former in-situ une imine intermédiaire qui est réduite chimiquement en utilisant par exemple, le triacétoxyborohydrure de sodium ou le cyanoborohydrure de sodium ou catalytiquement en utilisant l'hydrogène et un catalyseur tel que le palladium sur charbon.

Le composé de formule (XIX) énantiomériquement pur ou racémique est nouveau et fait partie de l'invention.

Le composé de formule (XIX) dans laquelle X représente un atome de chlore ou de fluor est préféré.

Selon un autre de ses aspects, l'invention a pour objet un procédé énantiosélectif I de préparation d'un composé, sous forme énantiomériquement pure, de formule :

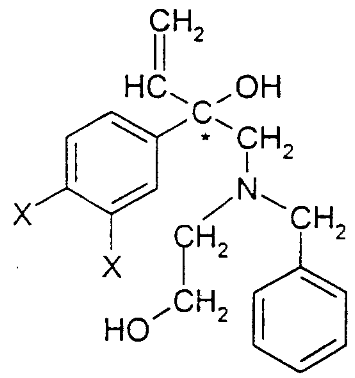

(IVa)

dans laquelle X représente un atome d'halogène, de ses sels avec des acides minéraux ou organiques, caractérisé en ce que :

a) b) c) on procède comme aux étapes a), b), c) du procédé H ;

d) on réduit le composé de formule (XIX) ainsi obtenu, au moyen d'un agent réducteur, dans un solvant inerte, pour obtenir un composé, énantiomériquement pur, de formule :

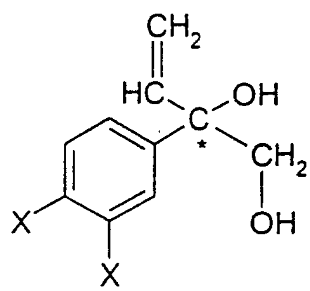

(XX)

e) on cyclise le composé de formule (XX) ainsi obtenu, pour obtenir un composé, sous forme énantiomériquement pure, de formule :

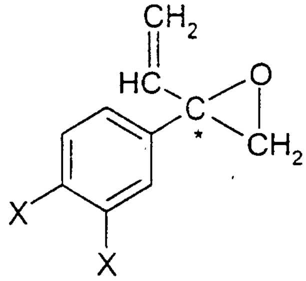

(VII)

f) on fait réagir le composé de formule (VII) ainsi obtenu avec du 2-(benzylamino)-1-éthanol, en présence d'une base et dans un solvant inerte, et, éventuellement, on transforme le composé de formule (IVa), énantiomériquement pur, en l'un de ses sels avec des acides minéraux ou organiques.

A l'étape d), la réduction de l'aldéhyde de formule (XIX) en un diol de formule (XX) s'effectue selon les méthodes classiques en utilisant un agent réducteur tel que le borohydrure de sodium, l'hydrure de diisobutyl aluminium ou l'hydrure d'aluminium et de lithium. De préférence, on utilise le borohydrure de sodium.

La réaction s'effectue dans un solvant inerte comme un solvant aromatique tel que le toluène, un alcool tel que l'éthanol, un éther tel que le tétrahydrofurane, ou un mélange de ces solvants.

La réaction s'effectue à une température comprise entre -70°C et la température de reflux du solvant et pendant un temps compris entre 1 et 24 heures.

A l'étape e), la cyclisation d'un diol de formule (XX) en un dérivé oxirane de formule (VII) s'effectue, de préférence, dans des conditions de catalyse par transfert de phase en utilisant un halogénure d'alkyle ou d'arylsulfonyle, en présence d'une base forte et d'un catalyseur de transfert de phase, dans un solvant inerte en mélange avec de l'eau.

On a trouvé que lorsqu'on effectue la réaction de cyclisation d'un composé de formule (XX) énantiomériquement pur dans les conditions précitées, on obtient un composé de formule (VII) énantiomériquement pur dont l'atome de carbone asymétrique possède la même configuration.

Parmi les halogénures d'alkyle ou d'arylsulfonyle on préfère le chlorure de méthanesulfonyle, le chlorure de benzènesulfonyle ou le chlorure de *p*-toluènesulfonyle.

L'halogénure d'alkyle ou d'arylsulfonyle intervient dans la réaction à raison de 1 à 1,5 équivalents molaires par équivalent molaire de composé de formule (XX).

La base utilisée lors de la réaction est choisi parmi un hydroxyde de métal alcalin tel que l'hydroxyde de sodium ou l'hydroxyde de potassium.

La base intervient dans la réaction à raison de 5 à 10 équivalents molaires par équivalent molaire de composé de formule (XX).

Le catalyseur de transfert de phase est choisi parmi les sels d'ammonium quaternaires substitués tel que le chlorure de benzyltriéthylammonium.

Le catalyseur intervient dans la réaction de 0,01 à 0,1 équivalent molaire par équivalent molaire de composé de formule (XX).

La réaction s'effectue dans un solvant inerte comme un hydrocarbure aromatique tel que le toluène ou le xylène, un solvant chloré tel que le dichlorométhane.

La réaction est conduite à une température comprise entre la température ambiante et 60°C et pendant un temps compris entre 1 et 24 heures.

A l'étape f), l'ouverture du dérivé oxirane de formule (VII) par du 2-(benzylamino)-1-éthanol s'effectue selon les méthodes classiques.

L'amine intervient dans la réaction à raison de 1 à 1,5 équivalents molaires par équivalent molaire de composé de formule (VII).

La base est choisie parmi les carbonates ou bicarbonates de métal alcalin tel que le carbonate de sodium, le carbonate de potassium ou le bicarbonate de sodium.

La base intervient dans la réaction à raison de 1 à 2 équivalents molaires par équivalent molaire de composé de formule (VII).

Le solvant est choisi parmi les solvants polaires tels que l'acétonitrile, le propionitrile ou la 1-méthyl-2-pyrrolidinone.

La réaction est conduite à une température comprise entre la température ambiante et 120°C et pendant un temps compris entre 1 à 48 heures.

On a trouvé que lorsqu'on effectue la réaction d'ouverture du composé de formule (VII) énantiomériquement pur dans les conditions précitées, on obtient un composé de formule (IVa) dont l'atome de carbone asymétrique possède la même configuration.

Le composé de formule (XX), énantiomériquement pur ou racémique, est nouveau et fait partie de l'invention.

Le composé de formule (XX) dans laquelle X représente un atome de chlore ou de fluor est préféré.

Le composé de formule (VII), énantiomériquement pur ou racémique, est nouveau et fait partie de l'invention.

Le composé de formule (VII) dans laquelle X représente un atome de chlore ou de fluor est préféré.

Le composé de formule (XVI) utilisé à l'étape a) du procédé H ou à l'étape a) du procédé I, se prépare à partir de la (S) ou de la (R)-proline, selon les procédés décrits dans Tetrahedron, 1981, 37 (23), 4111-4119, et tels qu'illustrés dans les Exemples.

Au cours de chacun des procédés A à I ci-dessus ou, au cours des différentes étapes constitutives de ces procédés, les composés de formule (I), (IIa), (IIb), (IIc), (lld), (IIe), (III), (IVa), (IVd), (IVe), (IV'd), (V), (VIa), (VIe), (VIII), (X), (XIII), (XIV), (XVII), (XVIII), (XIX) et (XX) ainsi obtenus peuvent être ultérieurement séparés du milieu réactionnel

selon les méthodes classiques telles que l'extraction, la cristallisation, la distillation, la chromatographie. De plus, les différents composés précités obtenus peuvent être soit isolés, soit directement engagés dans le procédé suivant ou l'étape suivante dans le milieu dans lequel ils ont été obtenus. Chacun des procédés A à I ou chacune des étapes constitutives de ces procédés peuvent ainsi être combinés pour la préparation des composés de formule (I).

Le cas échéant, les composés de formule (I), (IIa), (IIe), (IIIa), (IIIe), (IVa), (IVe), (IV'd), (VIa), (VIe), (XIII) et (XIV) ainsi obtenus peuvent être isolés sous forme de base libre ou de sel, selon les techniques classiques.

Ces sels comprennent ceux avec des acides minéraux ou organiques qui permettent une séparation ou un cristallisation convenable des composés de formule (I), (IIa), (IIe), (IIIa), (IIIe), (IVa), (IVe), (IV'd), (VIa), (VIe), (XIII) et (XIV), tels que le chlorhydrate, le bromhydrate, le sulfate, l'hydrogénosulfate, le dihydrogénophosphate, le méthanesulfonate, le méthylsulfate, le maléate, le fumarate, le succinate, le naphtalène-2-sulfonate, le glyconate, le gluconate, le citrate, l'iséthionate, le benzynesulfonate, le paratoluènesulfonate.

Lorsque les composés de formule (I), (IIa), (IIe), (IIIa), (IIIe), (IVa), (IVe), (IV'd), (VIa), (VIe), (XIII) et (XIV) sont obtenus sous forme de base libre, la salification est effectuée par traitement avec l'acide choisi dans un solvant organique. Par traitement de la base libre, dissoute par exemple dans un éther tel que l'éther diéthylique ou dans un alcool tel que le méthanol, l'éthanol ou le propan-2-ol, ou dans l'acétone, ou dans le dichlorométhane ou dans l'acétate d'éthyle avec une solution de l'acide choisi dans un des solvants précités, on obtient le sel correspondant qui est isolé selon les techniques classiques.

Ainsi, on prépare par exemple chlorhydrate, le bromhydrate, le sulfate, l'hydrogénosulfate, le dihydrogénophosphate, le méthanesulfonate, le benzynesulfonate, le paratoluènesulfonate, l'oxalate, le maléate, le succinate, le fumarate, le naphtalène-2-sulfonate.

Les composés de formule (I), (IIa), (IIe), (IIIa), (IIIe), (IVa), (IVe), (IV'd), (VIa), (VIe), (XIII) et (XIV) peuvent être isolés sous forme d'un de leurs sels, par exemple chlorhydrate, ou l'oxalate ; dans ce cas, s'il est nécessaire, la base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

Selon un aspect préférentiel, l'invention a pour objet des procédés de préparation A à I de composé de formule (I), (IIa), (IIb), (IIc), (IId), (IIe), (IVa), (IVd), (IVe) et (V) dans lesquelles X représente un atome de chlore ou un atome de fluor.

Selon un autre aspect préférentiel, l'invention a pour objet des composés de formule (I) sous forme de sels optiquement actifs avec l'acide L-(-) ou D(+)- diparatoluoyltartrique, (II), (IV), (V), (VI), (VII), (XIII) dans lequel $R_2$ représente un groupe tétrahydropyran-2-yle, (XIX) et (XX) dans lesquelles X représente un atome de chlore ou un atome de fluor.

Ainsi, selon l'invention, on prépare les composés de formule (I) en utilisant les procédés A à.I ci-dessus et en suivant les voies de synthèses ci-après définies et telles qu'illustrées dans les Exemples :

- voie de synthèse I : procédé F étapes a et b ; puis procédé E étape a ou étape a et b ; puis procédé C ; puis procédé A étapes a, b et c.
- voie de synthèse II : procédé F étapes a et b ; puis procédé D étapes a, b et c ; puis procédé C ; puis procédé A étapes a, b.
- voie de synthèse III : procédé G étapes a, b, c et d ; puis procédé C ; puis procédé A étapes a, b et c.
- voie de synthèse IV : procédé H étapes a, b, c et d ; puis procédé C ; puis procédé A étapes a et b.
- voie de synthèse V : procédé I étapes a, b, c, d, e et f ; puis procédé C ; puis procédé A étapes a et b.
- voies de synthèses VI à X : respectivement identiques aux voies de synthèses I à V mais en effectuant le procédé B entre le procédé C et le procédé A.

Les Exemples suivants illustrent l'invention sans toutefois la limiter.

Dans les Exemples ci-après on utilise les abréviations suivantes :

DCM: dichlorométhane
THF : tétrahydrofurane
TA :. température ambiante.

Les spectres de résonance magnétique nucléaire du proton ($RMN^1H$) sont enregistrés à 200 MHz dans DMSO-$d_6$, en utilisant le pic du DMSO-$d_6$, comme référence. Les déplacements chimiques $\delta$ sont exprimés en parties par million (ppm). Les signaux observés sont exprimés ainsi : s : singulet ; d : doublet ; d.d : doublet dédoublé ; t : triplet ; t.d : triplet dédoublé ; q : quadruplet ; m : multiplet.

Les puretés énantiomériques ont été déterminées par analyse par Chromatographie Liquide Haute Performance (CLHP) sur phase chirale CHIRACEL OD ou AD ( phases stationnaires à base de cellulose) ainsi que par CLHP

supercritique.

EXEMPLE 1 - Voie de synthèse I.

(R)-(+)-2-[2-(3,4-Difluorophényl)morpholin-2-yl]-1-éthanol, sel avec l'acide L-(-)-di-paratoluoyltartrique.

(I) : X = F

A) 2-Chloro-1-(3,4-difluorophényl)éthanone.

(X) : X = F ; Hal = Cl ;

procédé F étape a).

A une suspension de 247,7 g de chlorure d'aluminium dans 450 ml de DCM, on ajoute en 10 minutes sous atmosphère d'azote, 227,6 g de chlorure de chloroacétyle (IX : Hal = Hal' = Cl), puis chauffe à reflux la solution jaune obtenue. On ajoute ensuite, en 2 heures, et en goutte à goutte, 200 g de 1,2-difluorobenzène (VIII : X = F) puis, maintient le mélange réactionnel à reflux pendant 1 heure. Après refroidissement du mélange réactionnel de couleur rouge à 20°C, on coule ce dernier sur 1 kg de glace et laisse 30 minutes sous agitation. Après décantation, on soutire la phase organique, extrait la phase aqueuse par 500 ml de DCM, lave les phases organiques jointes par 2 x 500 ml d'eau, par 500 ml d'une solution saturée de $NaHCO_3$, par 500 ml d'eau, sèche la phase organique sur $MgSO_4$ et évapore sous vide le solvant. On obtient 327,6 g du produit attendu sous forme d'huile jaune lacrymogène.

Rendement : 97,6 %

RMN$^1$H : δ (ppm) : 5,2 : s : 2H ; 7,6 : dd : 1H ; 7,8 : m : 1H ; 8,0 : dd : 1H.

B) 1-Chloro-2-(3,4-difluorophényl)but-3-èn-2-ol.

(V) X = F ; Hal = Cl ;

procédé F étape b).

On refroidit à -10°C, sous atmosphère d'azote, 800 ml d'une solution 1M de bromure de vinylmagnésium (XI : Hal" = Br) dans le THF, ajoute en 4 heures et 30 minutes et en maintenant la température masse à -10°C, une solution de 152,4 g du composé obtenu à l'étape, précédente dans 800 ml de THF, puis laisse le mélange réactionnel sous agitation pendant 20 minutes et à une température de -10°C. On hydrolyse le mélange réactionnel en le coulant sur 2 litres d'une solution aqueuse saturée de chlorure d'ammonium et laisse 30 minutes sous agitation. Après décantation, on lave la phase organique par 2 x 1 litre d'une solution saturée de NaCl, puis filtre la phase organique pour éliminer les sels minéraux. On obtient 1,5 litre d'une solution du composé attendu dans le THF qui est directement engagée dans l'étape suivante.

RMN$^1$H : δ (ppm) : 3,9 : dd : 2H ; 5,3 : dd : 2H ; 5,9 : s : 1H ; 6,2 : m : 1H ; 7,3-7,5 : m : 3H.

C) 1-[Benzyl(2-hydroxyéthyl)amino]-2-(3,4-difluorophényl)but-3-èn-2-ol.

(IVa) : X = F, $R_1$ = $-CH_2-$ 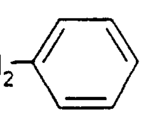 ;

procédé E étape a).

On chauffe à 120°C un mélange de 67,2 g de $NaHCO_3$ et 133 g de 2-(benzylamino)-1-éthanol dans 500 ml de 1-méthyl-2-pyrrolidinone, puis coule, en 3 heures, la solution du composé obtenu à l'étape précédente dans le THF (1,5 litre), tout en distillant le THF dès son introduction dans le milieu réactionnel. Après distillation de 1,5 litre de THF, on laisse le mélange réactionnel sous agitation pendant 12 heures à 120°C. Après refroidissement à 20°C, on coule le mélange réactionnel sur 2 litres d'eau, extrait le mélange par 2 litres de toluène puis lave la phase organique par 2 x 500 ml d'eau. On ajoute 2 litres d'eau à la phase organique et coule 75 ml d'une solution d'HCl concentrée. On lave la phase aqueuse acide par 500 ml de toluène, alcafinise la phase aqueuse par ajout

de 96 ml d'une solution de NaOH 10N, extrait par 1,2 litre de toluène et lave la phase organique par 500 ml d'eau. On obtient 1,23 kg de solution toluènique contenant le composé attendu et qui est directement engagée dans l'étape suivante.

Un extrait sec de 50 g de solution contient 8,9 g du composé attendu, soit, ramené à la totalité de la solution, 219 g du composé attendu.

Rendement : 82 %, calculé à partir du composé de départ de l'étape B de formule (X) : X = F ; Hal = Cl.

RMN$^1$H : $\delta$ (ppm) : 2,5 : q : 2H ; 2,9 : s : 2H ; 3,3 : q : 2H ; 3,6 : dd : 2H ; 4,5 : t : 1H ; 5,2 : dd : 2H ; 5,5 : s : 1H ; 6,3 : dd : 1H ; 7,1-7,5 : m : 8H.

D) Maléate de 4-benzyl-2-(3,4-difluorophényl)-2-vinylmorpholine.

(IIa), $C_4H_4O_4$ : X = F, $R_1$ = $-CH_2-$ 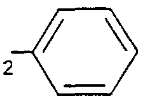 ;

procédé C.

On place sous agitation à 760t/mn la solution toluènique du composé obtenu à l'étape précédente, ajoute 7,5 g de chlorure de benzyltriéthylammonium, puis ajoute une solution fraichement préparée et chaude de 215,5 g de NaOH en pastilles dans 215 ml d'eau, la température du milieu réactionnel s'élevant à 48°C. On ajoute alors, en goutte à goutte, pendant 2 heures, et à un débit permettant de maintenir la température masse à 45°C, 139,23 g de chlorure de benzènesulfonyle. Après refroidissement du mélange réactionnel à 20°C, on l'hydrolyse par ajout de 1 litre d'eau et laisse 1 heure sous agitation. Après décantation, on lave la phase organique par 2 x 1 litre d'eau (pH = 7), sèche sur $MgSO_4$ et évapore sous vide le solvant. On obtient 200 g du composé attendu sous forme de base libre.

Rendement : 96,5 %.

On chauffe à reflux pendant 10 minutes une suspension de 94,7 g d'acide maléique dans 530 ml d'AcOEt, puis ajoute une solution de 257,3 g du composé (IIa : X = F) base libre dans 116 ml d'AcOEt. On laisse le mélange réactionnel revenir à température ambiante, et à 43°C, on amorce la cristallisation par ajout de 1 g de maléate du composé (IIa : X = F) pour obtenir une précipitation rapide. On refroidit le mélange réactionnel à 0°C et laisse 12 heures sous agitation. On essore le produit cristallisé, le lave par 3 x 100 ml d'AcOEt froid et le sèche sous vide à 30°C. On obtient 250 g du composé attendu sous forme de maléate.

Rendement de la salification : 71 %.

RMN$^1$H : $\delta$ (ppm) : 2,5 : m : 2H ; 2,9 : dd : 2H ; 3,7 : m : 2H+2H ; 5,7 : td : 2H ; 5,9 : dd : 1H ; 6,1 : s : 2H ; 7,1-7,6 : m : 8H.

E) Chlorhydrate de 2-[4-benzyl-2-(3,4-difluorophényl)morpholin-2-yl]-1-éthanol.

(IIIa), HCl : X = F, $R_1$ = $-CH_2-$ 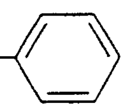 ;

procédé A étape a).

Dans un réacteur "Pilote système" (température de l'huile de la double enveloppe asservie à la température masse), mis sous atmosphère d'azote, on introduit 1 litre d'une solution 0,5M de 9-borabicyclo[3.3.1]nonane dans le THF et distille à pression atmosphérique 500 ml de THF avec une température masse de 74°C. On effectue alors un échange de solvant à volume constant par introduction de 500 ml de toluène, l'échange durant 3 heures et la température finale étant de 110°C. On refroidit la masse à une température de 20°C et observe la précipitation du dimère du 9-borabicyclo[3.3.1]nonane. On ajoute ensuite, en 25 minutes, une solution de 131,4 g du composé obtenu à l'étape précédente sous forme de base libre dans 150 ml de toluène et laisse le mélange réactionnel 12 heures sous agitation. On refroidit le mélange réactionnel à 5°C, agite à 500t/mn, ajoute 50 ml d'une solution de NaOH 10N puis 7 g d'hydrogénosulfate de tétrabutylammonium. On règle la consigne de température masse à 20°C, et ajoute 60 g (53 ml) d'une solution 11M de peroxyde d'hydrogène dans l'eau (33 %, 130 volumes, d = 1,13) à un débit masse de 1 g/mn. Puis on règle la consigne masse à 35°C et ajoute 60 g de la solution 11M de peroxyde d'hydrogène à un débit masse de 1,5 g/mn. Enfin on règle la consigne masse à 50°C et ajoute 60 g de la solution 11M de peroxyde d'hydrogène à un débit masse de 3 g/mn. On laisse 1 heure sous agitation à une température de 50°C. Après décantation à chaud, on observe 3 phases : toluène/cis-1,5-cyclooctanediol/eau. Après élimination de la phase aqueuse, on refroidit à 20°C et lave la phase toluènique par 3 x 200 ml d'eau pour éliminer le diol. On élimine l'eau résiduelle de la phase toluènique par entraînement azéotropique à volume constant avec 200 ml de toluène. On ajoute alors à la phase toluènique limpide ainsi obtenue 1 g de chlorhydrate du composé (IIIa : X = F) attendu puis, on ajoute, goutte à goutte et en 40 minutes, 68 ml d'une solution 6,1M d'HCl dans l'éthanol. On refroidit à 20°C, laisse 3 heures sous agitation, essore le précipité formé, le lave par 3 x 100 ml de toluène et le sèche sous vide à 30°C. On obtient 124,3 g du composé attendu sous forme de chlorhydrate.

Rendement : 80,6 %.

RMN$^1$H : δ (ppm) : 2,0 : m : 2H ; 3,0 : m : 2H ; 3,2 : m : 2H ; 3,4 : m : 2H ; 4,0 : dd : 2H ; 4,4 : s : 2H ; 7,1-7,8 : m : 8H.

F) 2-[2-(3,4-difluorophényl)morpholin-2-yl]-1-éthanol, racémique.

(I) : X = F ,

procédé A étape b).

Dans un réacteur d'hydrogénation, purgé à l'azote, on introduit 26 g de palladium sur charbon à 10 % et à 50 % d'humidité, puis verse une solution de 236,3 g du composé obtenu à l'étape précédente sous forme de base libre dans 2,6 litres de MeOH et ajoute 53 ml d'une solution d'HCl concentrée. On hydrogène sous une pression de 3 bars, à une température de 40°C et pendant 1 heure. On refroidit à 20°C, filtre le mélange réactionnel sur filtre Whatman®, lave avec 500 ml de MeOH et concentre sous vide le filtrat jusqu'à un volume de 500 ml. On effectue un échange de solvant avec 500 ml d'eau, puis ajoute à la solution aqueuse ainsi obtenue 80 ml de NaOH 10N et laisse 1 heure sous agitation à 15°C. On essore le précipité formé, le lave avec 200 ml d'eau, reprend le précipité dans 400 ml d'éther diisopropylique et laisse 1 heure sous agitation. On essore le précipité, le lave par 200 ml d'éther diisopropylique et le sèche sous vide une nuit à 40°C. On obtient 120 g du produit attendu.

Rendement : 78 %.

G) (R)-(+)-2-[2-(3,4-difluorophényl)morpholin-2-yl]-1-éthanol, sel avec l'acide L-(-)-di-paratoluoyltartrique.

(I) : X = F ;

procédé A étape c).

On chauffe à 40°C une suspension de 60 g du composé obtenu à l'étape précédente dans 780 ml de MeOH. On agite à 240 t/mn, ajoute en 50 minutes une solution de 95,3 g d'acide L-(-)-di-paratoluoyltartrique dans 300 ml de MeOH, tout en maintenant la température masse à 40°C. On laisse ensuite, 10 minutes, sous agitation à 40°C, puis 1 heure à 35°C et refroidit à 20°C en 2 heures et 30 minutes. On laisse 12 heures sous agitation à 20°C, essore le précipité formé, le reprend dans 150 ml de MeOH et laisse 30 minutes sous agitation. On essore à nouveau le précipité, le lave avec 60 ml de MeOH et le sèche sous vide à 40°C. On obtient 64,3 g du composé attendu sous forme de sel avec l'acide L-(-)-di-paratoluoyltartrique.

Rendement : 41,3 %.

Rendement final : 14,2 % calculé à partir du composé de départ de l'étape A de formule (VIII) : X = F.

Pureté énantiomérique : 97,5 % (e.e = 95 %).

EXEMPLE 2 - Voie de synthèse II.

(R)-(+)-2-[2-(3,4-difluorophényl)morpholin-2-yl]-1-éthanol.

(I) : X = F.

A) 2-Chloro-1-(3,4-difluorophényl)éthanone.

(X) : X = F ; Hal = Cl ;

procédé F étape a).

Le mode opératoire est identique à celui de l'étape A de l'Exemple 1.

B) 1-Chloro-2-(3,4-difluorophényl)but-3-èn-2-ol.

(V) : X = F ; Hal = Cl ;

procédé F étape b).

On refroidit à -15°C, sous atmosphère d'azote, 560 ml d'une solution 1,8M de chlorure de vinylmagnésium (XI : Hal" = Cl) dans le THF et ajoute, en 2 heures, en maintenant la température masse à -15°C, une solution de 170 g du composé obtenu à l'étape précédente dans 510 ml de THF. On hydrolyse le mélange réactionnel en le coulant sur 1 litre de solution aqueuse saturée de chlorure d'ammonium et laisse 1 heure sous agitation à température ambiante. Après décantation, on lave la phase organique par 2 x 1 litre d'une solution aqueuse saturée de NaCl. On obtient 840 ml d'une solution du composé attendu dans le THF qui est directement engagée dans l'étape suivante.

C) 1-(Benzylamino)-2-(3,4-difluorophényl)but-3-èn-2-ol.

(VIa) : X = F, $R_1$ = -$CH_2$- 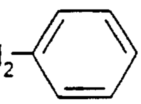 ;

procédé D étape a).

On chauffe à reflux un mélange de 81 g de $NaHCO_3$ et 112,7 g de benzylamine dans 190 ml de THF et ajoute rapidement 300 ml de la solution du composé obtenu à l'étape précédente dans le THF. Dès que le reflux est à nouveau atteint, on distille à volume constant le THF, tout en additionnant le restant de la solution précédente (540 ml). A la fin de l'addition, on concentre le mélange réactionnel à un volume de 400 ml de THF. On effectue alors un échange de solvant à volume constant avec 580 ml de propionitrile, la température masse étant de 85°C en fin d'échange. On laisse sous agitation à 85°C pendant 8 heures. Puis on élimine le propionitrile par échange de solvant à volume constant par ajout, en 4 heures 30 minutes, de 1,1 litre de toluène, la température masse étant de 106°C en fin d'échange. Après refroidissement à température ambiante, on rajoute 1,3 litre de toluène pour obtenir 1,9 litre de solution finale, puis lave la phase toluènique par 2 litres d'une solution aqueuse contenant 12,5 ml d'acide acétique. On ajoute à la phase organique 1,3 litre d'une solution aqueuse contenant 80 ml d'HCl concentrée, complète par ajout de 400 ml de toluène et de la quantité suffisante d'eau pour obtenir 2,5 litres de phase aqueuse afin de solubiliser l'huile obtenue. Après extraction et décantation, on lave la phase organique par 500 ml d'eau et réunit les phases aqueuses. On alcalinise les phases aqueuses par ajout de 105 ml de NaOH 10N, extrait par 1,3 litre puis par 0,7 litre d'éther diisopropylique, lave les phases organiques jointes par 3 x 2 litres d'eau, sèche sur $MgSO_4$ et évapore sous vide le solvant. On obtient 163 g du produit attendu.

Rendement : 64 %, calculé à partir du composé de départ de l'étape B de formule (X) : X = F ; Hal = Cl.

RMN$^1$H : δ (ppm) : 3,2 : dd : 2H ; 4,1 : s : 2H ; 5,2-5,4 : dd : 2H ; 6,1-6,3 : dd : 1H ; 7,2-7,6 : m : 8H.

D) R-(+)-1-(Benzylamino)-2-(3,4-difluorophényl)but-3-èn-2-ol, sel avec l'acide L-(+)-mandélique.

(VIa) : X = F, $R_1$ = -$CH_2$- 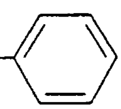 ;

procédé D étape b).

On chauffe à 60°C une suspension de 36,25 g d'acide L-(+)-mandélique dans 1,1 litre d'éther diisopropylique, ajoute en 4 heures une solution de 137,9 g du composé obtenu à l'étape précédente dans 555 ml d'éther diisopropylique puis, à la fin de l'addition, chauffe le milieu réactionnel à 75°C pendant 1 heure 30 minutes pour solubiliser le précipité déjà formé. On refroidit le mélange réactionnel à 20°C en utilisant une rampe de refroidissement à raison de 0,3°C/mn et laisse 8 heures sous agitation à 20°C. On essore le précipité formé, le remet en suspension dans 600 ml d'éther diisopropylique, essore à nouveau et le sèche sous vide à 30°C. On obtient 103 g du composé attendu. On reprend le composé dans 720 ml d'AcOEt, chauffe à 60°C en utilisant une rampe de température à raison de 1°C/mn et maintient, sous agitation, la solution obtenue à 60°C pendant 15 minutes. On refroidit à 0°C avec une rampe de refroidissement de 1°C/mn, amorce la cristallisation et laisse 4 heures sous agitation à 0°C. On essore le précipité formé, le lave avec 150 ml d'éther diisopropylique et le sèche sous vide à 30°C. On obtient 60 g du composé attendu, sous forme de sel avec l'acide L-(+)-mandélique.

Rendement : 28,5 %.

Pureté énantiomérique : 97,4 % (e.e : 94,8 %).

$$\alpha_D^{25} = +44,6° \ (c = 1 ; MeOH).$$

E) (R)-(+)-1-[Benzyl(2-hydroxyéthyl)amino]-2-(3,4-difluorophényl)but-3-èn-2-ol.

(IVa) : X = F, $R_1$ = -$CH_2$- 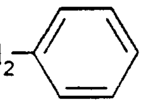 ;

procédé D étape c).

Dans un réacteur de Parr, on introduit une solution de 70,25 g du composé obtenu à l'étape précédente dans 200 ml de MeOH, met le réacteur sous vide et refroidit la solution à 0°C. On introduit ensuite de l'oxyde d'éthylène préalablement chauffée à 30°C jusqu'à une pression de 1 bar, puis chauffe lentement le milieu réactionnel à 40°C et laisse 4 heures sous agitation. Après avoir purger l'oxyde d'éthylène, puis le milieu réactionnel par bullage d'azote, on concentre sous vide le MeOH. On reprend l'huile résiduelle dans 250 ml d'eau, acidifie la phase aqueuse par ajout de 13 ml d'HCl concentré, lave la phase aqueuse acide par 2 x 250 ml de méthyl-tert-butyléther, alcalinise la phase aqueuse par ajout de 18 ml de NaOH 10N et extrait par 2 x 250 ml de toluène. Les phases organiques jointes sont chromatographiées sur 500 g de silice en éluant par 5 x 250 ml d'un mélange toluène/AcOEt (50/50 ; v/v). On réunit les phases et concentre sous vide les solvants. On obtient 43,5 g du composé attendu.

Rendement : 81 %.

$$\alpha_D^{25} = +13,9°$$

(c = 1 ; MeOH).

F) Chlorhydrate de (R)-(+)-4-benzyl-2-(3,4-difluorophényl)-2-vinylmorpholine.

(IIa), HCl : X = F ; $R_1$ = -$CH_2$- 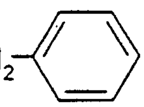 ;

procédé C.

On agite à 760 t/mn une solution de 40 g du composé obtenu à l'étape précédente dans 200 ml de toluène, ajoute 1,4 g de chlorure de benzyltriéthylammonium, puis ajoute une solution fraichement préparée et chaude de 40 g de NaOH en pastilles dans 40 ml d'eau, la température du milieu réactionnel s'élevant à 48°C. On ajoute alors, en goutte à goutte, pendant 1 heure et à un débit permettant de maintenir la température masse à 45°C, 25,43 g de chlorure de benzènesulfonyle. Après refroidissement du mélange réactionnel à 20°C, on l'hydrolyse par ajout de 200 ml d'eau et laisse 1 heure sous agitation. Après décantation, on lave la phase organique par 2 x 200 ml d'eau (pH = 7) et sèche sur $MgSO_4$. A la solution toluènique ainsi obtenue, on ajoute, en goutte à goutte, 20 ml d'une solution 6,1M d'HCl dans l'éthanol, laisse 1 heure sous agitation et concentre sous vide les solvants. On reprend le résidu dans 200 ml de toluène, agite et essore le précipité formé. On lave le précipité par 100 ml de toluène et le sèche sous vide à 30°C. On obtient 37 g du composé attendu sous forme de chlorhydrate.

Rendement : 87 %.

$$\alpha_D^{25} = +19,7°$$

(c = 1 ; MeOH).

G) Chlorhydrate de R-(+)-2-[4-benzyl-2-(3,4-difluorophényl)morpholin-2-yl]-1-éthanol.

(IIIa), HCl : X = F ; $R_1$ = -$CH_2$- 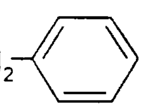 ;

procédé A étape a).

On distille sous vide, sous atmosphère d'azote, 100 ml de THF des 200 ml d'une solution 0,5M de 9-borabicyclo [3.3.1]nonane dans le THF. On effectue alors un échange de solvant à volume constant par introduction de 100 ml de toluène, puis refroidit la solution obtenue à 20°C (précipitation du dimère du 9-BBN). On ajoute une solution de 30 g du composé obtenue à l'étape précédente sous forme de base libre dans 35 ml de toluène et laisse 8 heures sous agitation. On ajoute ensuite, 11 ml d'une solution de NaOH 10N puis, 1,6 g d'hydrogénosulfate de tétrabutylammonium dissout dans 2 ml d'eau. On agite le mélange réactionnel à 500 t/mn, chauffe à 45°C masse, ajoute, en 40 minutes, 31 g d'une solution 11M de peroxyde d'hydrogène dans l'eau (33 %, 130 volumes, d = 1,13) à un débit permettant de maintenir 45°C dans le milieu réactionnel et laisse 15 minutes sous agitation à 45°C. Après décantation, on élimine la phase aqueuse alcaline, ajoute 100 ml d'eau pour dissoudre le cis-1,5-cyclooctanediol sous forme d'huile, agite et refroidit à 20°C. Après décantation, on lave la phase organique par 2 x 100 ml d'eau et élimine l'eau résiduelle de la phase organique par entraînement azéotropique à volume constant jusqu'à obtenir une phase limpide. On ajoute alors, en goutte à goutte, 16,4 ml d'une solution 6,1M d'HCl dans l'éthanol et laisse 1 heure sous agitation à 20°C. On essore le précipité formé, le lave par 3 x 20 ml de toluène et le sèche sous vide à 40°C. On obtient 28 g du produit attendu sous forme de chlorhydrate.

Rendement : 90 %.

$$\alpha_D^{25} = +41,6°$$

(c = 1 ; MeOH).

H) R-(+)-2-[2-(3,4-difluorophényl)morpholin-2-yl]-1-éthanol.

(I) : X = F ;

procédé A étape b).

A 3 g de palladium sur charbon à 10 % et à 50 % d'humidité on ajoute, sous atmosphère d'azote, une solution de 30 g du composé obtenu à l'étape précédente dans 300 ml de MeOH, puis 30 ml de toluène. On hydrogène sous une pression de 1 bar, à une température de 30°C. On filtre le catalyseur sur filtre Whatman®, lave au MeOH, puis effectue un échange de solvant du filtrat avec 120 ml d'eau. Après refroidissement, on lave la phase aqueuse par 2 x 120 ml de méthyl-*tert*-butyléther, alcalinise la phase aqueuse par ajout de 9 ml de NaOH 10N et laisse 1 heure sous agitation à froid. On essore le précipité formé, le lave par 100 ml d'éther diisopropylique et le sèche sous vide à 40°C. On obtient 14 g du produit attendu.

Rendement : 65 %.

Rendement final : 7,3 %, calculé à partir du composé de départ de l'étape A de formule (VIII) : X = F.

$$\alpha_D^{25} = +21,9°$$

(c = 1; MeOH).

Pureté énantiomérique : 99 % (e.e. = 98 %).

EXEMPLE 3 - Voie de synthèse III.

(R)-(+)-2-[2-(3,4-Difluorophényl)morpholin-2-yl]-1-éthanol, sel avec l'acide L-(-)-di-paratoluoyltartrique.

(I): X = F.

A) Maléate de N-benzyl-2-(tétrahydro-2H-pyran-2-yloxy)-1-éthylamine.

(XII), $C_4H_4O_4$ : $R_2$ = 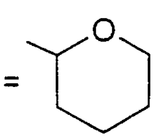

A un mélange de 110 g d'acide benzènesulfonique dans 1,8 litre de DCM, on ajoute, à température ambiante 100 g de 2-(benzylamino)-1-éthanol, la température à la fin de l'ajout étant de 34,4°C et le pH = 2. On refroidit le mélange réactionnel à 20°C, et ajoute, en goutte à goutte, 105 ml de 3,4-dihydro-2*H*-pyrane, le mélange réactionnel se colorant en jaune, puis en rose et enfin en violet à la fin de l'addition. On verse le mélange sur 2 litres d'une solution aqueuse de $K_2CO_3$ à 10 %, puis après décantation, on sèche la phase organique sur $Na_2SO_4$ et évapore sous vide le solvant. On dissout l'huile obtenue dans 200 ml d'AcOEt, ajoute une solution chaude de 72,7 g d'acide maléique dans 655 ml d'AcOEt et laisse une nuit sous agitation à température ambiante. On essore le précipité formé, le lave par 3 x 100 ml d'AcOEt et le sèche sous vide à température ambiante. On obtient 207 g du produit attendu sous forme de maléate.

Rendement : 89 %.

RMN$^1$H : δ (ppm) : 1,2-1,7 : m : 6H ; 3,0 : t : 2H ; 3,1-3,8 : m : 2H ; 4,1 : s : 2H ; 4,5 : t : 1H ; 7,2-7,4 : m : 5H ; 8,0 : s : 1H.

B) 2-Chloro-1-(3,4-difluorophényl)éthanone.

(X) : X = F ; Hal = Cl ;

procédé G étape a).

Le mode opératoire est identique à celui de l'étape A de l'Exemple 1.

C) 2-{Benzyl[2-(tétrahydro-2*H*-pyran-2-yloxy)éthyl]amino}-1-(3,4-difluorophényl)-1 -éthanone.

(XIII) : X = F ; $R_2$ = 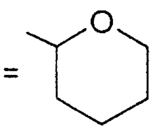 ;

procédé G étape b).

A une suspension de 200 g du composé obtenu à l'étape A dans 1 litre de DCM, on ajoute 1,5 litre d'une solution aqueuse de $K_2CO_3$ à 10 % puis, après décantation, évapore sous vide le solvant et dissout l'huile obtenue dans 250 ml de THF. On ajoute cette solution à une solution de 52,65 g de $NaHCO_3$ dans 10,35 ml d'eau et chauffe le mélange réactionnel à reflux. On ajoute alors, en goutte à goutte et en 50 minutes, une solution de 108,55 g du composé obtenu à l'étape B dans 250 ml de THF et laisse 3 heures sous agitation à reflux. Sous atmosphère d'azote, on élimine l'eau par entraînement azéotropique avec ajout simultané de 600 ml de THF pendant 2 heures. Après refroidissement à température ambiante, on ajoute à la solution du tamis moléculaire 4 angström et laisse une nuit sous agitation. Après filtration sous atmosphère d'azote, on obtient 800 ml d'une solution du composé attendu dans le THF qui est directement engagée dans l'étape suivante.

RMN$^1$H : δ (ppm) : 1,4 : m : 6H ; 2,7 : td : 1H ; 3,3 : m : 2H ; 3,6 : m : 2H ; 3,7 : s : 2H ; 4,0 : s : 2H ; 4,4 : m : 2H ; 7,0-7,4 : m : 8H.

D) 1-{Benzyl[2-(tétrahydropyran-2-yloxy)éthyl]amino}-2-(3,4-difluoro phényl)but-3-èn-2-ol.

(XIV) : X = F ; $R_2$ = 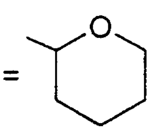 ;

procédé G étape c).

A 911 ml d'une solution 1M de bromure de vinylmagnésium (XI : Hal" = Br) dans le THF, on ajoute, sous atmosphère d'azote, en 30 minutes et à un débit permettant d'obtenir une température masse de 55°C, la solution du composé obtenu à l'étape précédente dans le THF, puis laisse deux heures sous agitation à 50°C. On hydrolyse le mélange réactionnel en le coulant en 45 minutes sur 1,5 litre d'une solution saturée de chlorure d'ammonium et, en maintenant une température masse de 30°C. On ajoute 500 ml d'eau. Après décantation, on extrait la phase aqueuse par 1 litre d'éther diisopropylique, joint les phases organiques et évapore sous vide les solvants. On obtient 241 g du composé attendu qui est engagée sans autre traitement à l'étape suivante.

E) 1-[Benzyl(2-hydroxyéthyl)amino]-2-(3,4-difluorophényl)but-3-èn-2-ol.

(IVa) : X = F, $R_1$ = $-CH_2-$ 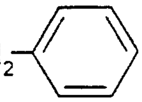 ;

procédé G étape d).

On refroidit à 5°C 500 ml de MeOH et, ajoute en goutte à goutte, 57 ml de chlorure d'acétyle en maintenant une température masse de 10°C. On ajoute alors cette solution, en 1 heure et à température ambiante, à une solution de 241 g du composé obtenu à l'étape précédente dans 500 ml de MeOH et laisse 2 heures sous agitation à température ambiante. On concentre sous vide le mélange réactionnel, dissout le résidu huileux avec 1 litre d'eau, lave la phase aqueuse par 2 x 500 ml d'éther diisopropylique, alcalinise la phase aqueuse par ajout de 40 g de NaOH en pastilles et extrait par 2 x 800 ml de toluène. On ajoute à la phase organique 500 ml d'eau et 400 ml de toluène et décante. On obtient 2,083 litres d'une solution du composé attendu dans le toluène qui est directement engagée dans l'étape suivante (teneur par HPLC : 91 mg/ml, soit une masse de 189,5 g du composé (IVa) : X = F)).

F) Maléate de 4-benzyl-2-(3,4-difluorophényl)-2-vinylmorpholine.

(IIa), $C_4H_4O_4$ : X = F, $R_1$ = $-CH_2-$ 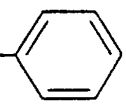 ;

procédé C.

A la solution toluènique du composé obtenu à l'étape précédente, on ajoute 6,4 g de chlorure de benzyltriéthylammonium, puis ajoute une solution fraîchement préparée et chaude de 187 g de NaOH en pastilles dans 187 ml d'eau, la température masse étant de 50°C. On ajoute alors, goutte à goutte, en 2 heures et à un débit permettant de maintenir une température masse de 55°C maximum, 86 ml de chlorure de benzènesulfonyle. Après refroidissement à TA, on ajoute 1 litre d'eau et laisse 15 minutes sous agitation. Après décantation, on lave la phase organique par 2 x 1 litre d'eau et par 1 litre d'une solution saturée de NaCl. On chromatographie la phase organique sur 180 g de silice, puis rince la silice par 400 ml de toluène. On réunit les phases toluèniques et concentre sous vide le solvant. On obtient 137,8 g du composé attendu sous forme de base libre.

On chauffe à reflux une suspension de 50,7 g d'acide maléique dans 284 ml d'AcOEt, puis ajoute une solution de 137,8 g du composé obtenu précédemment base libre dans 62 ml d'AcOEt et laisse 12 heures sous agitation en laissant la température revenir à TA. On refroidit à 0°C, laisse 1 heure sous agitation, essore le précipité formé, le lave par 4 x 50 ml d'AcOEt froid et le sèche sous vide à TA. On obtient 157 g du composé attendu sous forme de maléate.

Rendement : 60 % calculé à partir du composé de départ de l'étape C) de formule (X) ; X = F ; Hal = Cl.

G) Chlorhydrate de 2-[4-benzyl-2-(3,4-difluorophényl)morpholin-2-yl]-1-éthanol.

(IIIa), HCl : X = F ; $R_1$ = $-CH_2-$ 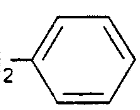 ;

procédé A étape a).

A une suspension de 245,3 g du composé obtenu à l'étape précédente dans 500 ml de toluène, on ajoute 500 ml d'eau puis coule 120 ml d'une solution de soude 10N. Après décantation, on extrait la phase aqueuse par 500 ml de toluène, joint les phases organiques et élimine l'eau par entraînement azéotropique à volume constant par ajout de toluène. On concentre la solution toluènique jusqu'à un volume de 350 ml.

Dans un réacteur "Pilote system", mis sous atmosphère d'azote, on introduit 800 ml d'une solution 0,5M de 9-borabicyclo[3.3.1]nonane dans le THF et concentre à pression atmosphérique jusqu'à un volume de 400 ml. On ajoute à nouveau 600 ml de la solution 0,5M de 9-BBN dans le THF et reconcentre jusqu'à un volume de 700 ml de THF. On procède alors à un échange de solvant à volume constant par introduction de 700 ml de toluène, la température masse passant de 68°C à 110°C. On refroidit la masse à 20°C et observe la précipitation du dimère du 9-BBN. On ajoute ensuite la solution de 350 ml du composé (IIa) : X = F, préparée ci-dessus et laisse 8 heures sous agitation. On refroidit le mélange réactionnel à 5°C, ajoute 9,6 g d'hydrogénosulfate de tétrabutylammonium, puis 69 ml de solution de NaOH 10N. On règle la consigne de température masse à 20°C et ajoute 72,3 g d'une solution 11M de peroxyde d'hydrogène dans l'eau (33 %, 130 volumes, d = 1,13) à un débit masse de 1,2 g/mn.

Puis on règle la consigne masse à 35°C et ajoute 72,3 g de la solution 11M de peroxyde d'hydrogène à un débit masse de 1,8 g/mn. Enfin on règle la consigne masse à 50°C et ajoute 72,3 g de la.solution 11M de peroxyde d'hydrogène à un débit masse de 3 g/mn. On laisse 1 heure sous agitation à 50°C, décante à chaud et obtient 3 phases : toluène/cis-1,5-cyclooctane diol/eau. On élimine la phase aqueuse, lave la phase toluènique par 2 x 200 ml d'eau pour éliminer le diol puis refroidit la phase organique à 20°C et sèche la phase organique sur $Na_2SO_4$. On ajoute à la phase organique 95 ml d'une solution 6,1M d'HCl dans l'éthanol, amorce par ajout de 1 g de chlorhydrate du composé (IIIa : X = F) et laisse 3 heures sous agitation à TA. On essore le précipité formé, le lave par 3 x 250 ml de toluène et le sèche sous vide à TA. On obtient 164 g du composé attendu sous forme de chlorhydrate.

Rendement : 86 %.

H) 2-[2-(3,4-difluorophényl)morpholin-2-yl]-1-éthanol, racémique.

(I) : X = F ;

procédé A étape b).

Dans un réacteur d'hydrogénation, mis sous atmosphère d'azote, on introduit 16 g de palladium sur charbon à 10 % et 50 % d'humidité, puis verse avec précaution une solution de 164 g du composé obtenu à l'étape précédente sous forme de chlorhydrate dans 1,6 litre de MeOH. On hydrogène, sous une pression de 3 bars, à 40°C et pendant 3 heures le mélange. Après refroidissement à TA, on filtre le catalyseur sur filtre Whatman®, lave avec 100 ml de MeOH, ajoute au filtrat 55 ml de NaOH 10N et concentre sous vide le MeOH jusqu'à un volume de 200 ml. On effectue un échange de solvant avec de l'eau par entraînement azéotropique. Après avoir éliminé tout le MeOH, on laisse 1 heure sous agitation, essore le précipité formé, le lave par 2 x 100 ml d'eau et le sèche sous vide une nuit à 40°C. On obtient 102 g du composé attendu.

Rendement : 94,8 %.

I) (R)-(+)-2-[2-(3,4-Difluorophényl)morpholin-2-yl]-1-éthanol, sel avec l'acide L-(-)-di-paratoluoyltartrique.

(I) : X = F ;

procédé A étape c).

On chauffe à 40°C une suspension de 95 g du composé obtenu à l'étape précédente dans 1,235 litre de MeOH et ajoute en 1 heure une solution de 150,88 g d'acide L-(-)-di-paratoluoyltartrique dans 475 ml de MeOH. A la moitié de la coulée, on amorce par ajout de 0,4 g de sel résolu et, en fin de coulée, laisse 4 heures et 30 minutes sous agitation en laissant revenir la température à TA. On refroidit à 20°C, poursuit l'agitation pendant 2 heures, essore le précipité formé, le lave par 2 x 100 ml d'EtOH et le sèche sous vide à TA. On obtient 100 g du composé attendu sous forme de sel avec l'acide L-(-)-di-paratoluoyltartrique.

Rendement : 40,8 %.

Rendement final : 19,5 %, calculé à partir du composé de départ de l'étape B) de formule (VIII) : X = F.

Pureté énantiomérique : 97,4 % (e.e. = 94,8 %).

EXEMPLE 4 - Voie de synthèse IV.

(S)-(-)-2-[2-(3,4-difluorophényl)morpholin-2-yl]-1-éthanol.

(I) : X = F.

A) 2-Phénylhexahydro-1*H*-pyrrolo[1,2-c]imidazole-3-carboxylate de méthyle, isomère unique, (XVI).

a) (S)-1-[(Benzyloxy)carbonyl]proline.

Ce composé est commercial.

b) (S)-2-(Anilinocarbonyl)-1-pyrrolidinecarboxylate de benzyle.

A une solution de 20 g du composé obtenu à l'étape précédente dans 200 ml d'acétonitrile, on ajoute, sous atmosphère d'azote, 9,7 ml de N-méthylmorpholine, puis refroidit à 0°C cette solution et ajoute, en 45 minutes, 7,7 ml de chloroformiate d'éthyle. On refroidit le milieu réactionnel à 0°C et ajoute, en 45 minutes, 7,3 ml d'aniline. On concentre sous vide le mélange réactionnel, extrait le résidu huileux par 200 ml d'AcOEt,

lave la phase organique par 2 x 200 ml d'une solution tampon pH = 2, par 200 ml d'eau, par 2 x 200 ml d'une solution aqueuse de $NaHCO_3$ à 10 %, rajoute 200 ml d'AcOEt à la phase organique, puis sèche sur $Na_2SO_4$, filtre et concentre sous vide le solvant. On reprend le résidu par 100 ml de *tert*-butylméthyléther, laisse 1 heure sous agitation, essore le précipité formé, le lave par 20 ml de *tert*-butylméthyléther et le sèche sous vide à 40°C. On obtient 20,8 g du produit attendu.

Rendement : 80 %.

RMN$^1$H : δ (ppm) :1,8 : m : 2H ; 2,2 : m : 2H ; 3,5 : m : 2H ; 4,3 : t.d : 1H ; 4,9 : d : 1H ; 5,1 : d : 1H ; 7,0-7,4 : m : 8H ; 8,6 : m : 2H ; 10 : d : 1H.

c) (S)-N-Phényl-2-pyrrolidinecarboxamide.

On verse avec précaution, sous atmosphère d'azote, une solution de 20 g du composé obtenu à l'étape précédente et 5,3 ml d'HCl concentré dans 300 ml de MeOH sur 2 g de palladium sur charbon à 10 % et 50 % d'humidité et, hydrogène pendant 2 heures, à pression atmosphérique et à TA. On ajoute 5 ml d'HCl concentré, filtre le catalyseur et concentre sous vide le filtrat. On dissout le résidu dans 200 ml d'eau, lave la phase aqueuse par 200 ml d'AcOEt, alcalinise la phase aqueuse par ajout de 5 g de NaOH en pastilles et extrait par 3 x 200 ml de DCM. On élimine le DCM par entraînement azétropique avec du THF, puis évapore sous vide le THF. On obtient 11,5 g du produit attendu.

Rendement : 94,5 %.

RMN$^1$H : δ (ppm) : 1,7 : m : 2H ; 2,0 : m : 2H ; 2,9 : t : 2H ; 3,3 : s : 1H ; 3.7 : m : 1H ; 7,1 : d.d : 1H ; 7,3 : d.d : 1H ; 7,7 : d :2H ; 9,9 : s : 1H.

d) (S)-N-(2-Pyrrolidinylméthyl)aniline.

On chauffe à reflux, sous atmosphère d'azote, 80, ml d'une solution 1M d'hydrure d'aluminium et de lithium dans le THF et ajoute, lentement, une solution de 12 g du composé obtenu à l'étape précédente dans 120 ml de THF. On laisse revenir le mélange réactionnel à TA, ajoute, avec précaution, 3 ml d'eau, 3 ml de NaOH 10N et 9 ml d'eau, et laisse une nuit sous agitation à TA. On filtre le mélange et concentre sous vide. On distille sous vide l'huile obtenue (Eb = 158-164°C/1mbar). On obtient 8,6 g du produit attendu. Rendement : 76 %.

RMN$^1$H : δ (ppm) : 1,1 : m : 2H ; 1,6 : m : 2H ; 2,6-2,8 : m : 4H ; 3,1 : m : 1H ; 5,3 : t : 1H ; 6,4 : m : 3H ; 7,0 : m : 2H.

e) 2-Phénylhexahydro-1H-pyrrolo[1,2-c]imidazole-3-carboxylate de méthyle, isomère unique.

A une solution de 2,64 g du composé obtenu à l'étape précédente dans 25 ml de toluène, on ajoute 1,89 g d'hydroxyméthoxyacétate de méthyle puis chauffe à reflux pendant 1 heure 30 minutes, en éliminant azéotropiquement l'eau formée. On laisse le mélange réactionnel revenir à TA, ajoute 25 ml d'eau pour dissoudre les insolubles, sèche la phase organique sur $MgSO_4$ et évapore sous vide le solvant. On obtient 3,67 g du produit attendu.

Rendement : quantitatif.

RMN$^1$H : δ (ppm) : 1,4-2,0 : m : 4H ; 2,6 : m : 1H ; 3,1 : m : 2H ; 3,5 : m : 1H ; 3,6 : s : 3H ; 3,8 : m : 1H ; 4,8 : s : 1H ; 6,4 : d.d : 2H ; 6,6 : dd : 1H ; 7,1 : dd : 2H.

B) (3,4-Difluorophényl )-(2-phénylhexahydro-1*H*-pyrrolo[1,2-c]imidazol-3-yl)méthanone, isomère unique.

(XVII) : X = F ;

procédé H étape a).

On prépare une solution de bromure de 3,4-difluorophénylmagnésium (XV : X = F, Hal''' = Br) à partir de 0,85 g de magnésium en tournures, 4 g de 1-bromo-3,4-difluorobenzène et 20 ml de THF et conserve à TA cette solution.

D'autre part, à un mélange de 0,4 g de magnésium en tournures dans 20 ml de THF, on ajoute, sous atmosphère d'azote, 0,13 ml de 1,2-dichloroéthane et chauffe jusqu'à apparition d'un trouble et d'un dégagement gazeux d'éthylène. On ajoute alors, 1,17 ml de 1,2-dichloroéthane par portions de 0,13 ml et chauffe à reflux pendant 1 heure. On laisse revenir à TA cette suspension de $MgCl_2$ anhydre ainsi obtenue, ajoute une solution de 3,67 g du composé obtenu à l'étape A) dans 37 ml de THF, chauffe à reflux pendant 1 heure et laisse revenir à TA. Puis on refroidit à -70°C cette solution, ajoute, en maintenant une température masse de -70°C, 20,4 ml de la solution de bromure de 3,4-difluorophénylmagnésium préparée précédemment et laisse 1 heure sous agitation à -70°C. On verse le mélange réactionnel sur 30 ml d'une solution saturée de $NH_4Cl$ et laisse 15 minutes sous agitation. On concentre sous vide le THF, reprend le résidu par 50 ml d'éther diisopropylique, lave la phase organique par 30 ml d'eau, par 30 ml d'une solution saturée de NaCl, sèche sur $MgSO_4$ et concentre sous vide le solvant. On obtient 4,59 g du produit attendu.

Rendement : 85 %.

C) 1-(3,4-Difluorophényl)-1-(2-phénylhexahydro-1H-pyrrolo[1,2-c] imidazol-3-yl)-2-propèn-1-ol, isomère unique.

(XVIII) : X = F ;

procédé H étape b).

On refroidit à -70°C une solution de 4,59 g du composé obtenu à l'étape précédente dans 30 ml de THF, ajoute lentement, en maintenant une température masse de -70°C, 28 ml d'une solution 1M de bromure de vinylmagnésium (XI : Hal" = Br) dans le THF et laisse 1 heure sous agitation à -70°C. On verse le mélange réactionnel sur 30 ml d'une solution saturée de $NH_4Cl$, décante et concentre sous vide le solvant de la phase organique. On extrait le résidu par 70 ml d'éther diéthylique, lave la phase organique par 30 ml d'eau et engage directement cette phase organique à l'étape suivante.

D) 2-(3,4-Difluorophényl)-2-hydroxybut-3-ènal, isomère unique.

(XIX) : X = F ;

procédé H étape c).

On refroidit à 5°C la solution éthérée obtenue à l'étape précédente, ajoute 174 ml d'HCl à 2 % et laisse une nuit sous agitation à 5°C. On lave la phase organique par 30 ml d'eau, sèche sur $MgSO_4$ et concentre sous vide le solvant. On obtient 2,67 g du produit attendu.

Rendement : 96 % (calculé à partir du composé de départ de l'étape C de formule (XVII) : X = F.

E) (S)-(-)-1-[Benzyl(2-hydroxyéthyl)amino]-2-(3,4-difluorophényl)but-3-èn-2-ol.

(IVa) : X = F, $R_1 = -CH_2-$ 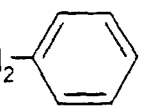 ;

procédé H étape d).

A une solution de 1,7 g du composé obtenu à l'étape précédente dans 34 ml d'acétonitrile, on ajoute 1,2 ml de 2-(benzylamino)-1-éthanol, puis 3,63 g de triacétoxyborohydrure de sodium et 3 gouttes d'acide acétique, et laisse 1 heure sous agitation à TA. On hydrolyse le mélange réactionnel par ajout de 50 ml d'HCl 1,2M, concentre sous vide la phase organique, lave la phase aqueuse par 50 ml d'éther diisopropylique, alcalinise la phase aqueuse par ajout de 7 ml de NaOH 10N, extrait par 50 ml d'éther diisopropylique, lave la phase organique par 2 x 50 ml d'eau, sèche sur $MgSO_4$ et évapore sous vide le solvant. On obtient 1 g du produit attendu.

Rendement : 35 %.

Pureté énantiomérique : 99,4 % (e.e. = 98,8 %).

F) (S)-(-)-4-Benzyl-2-(3,4-difluorophényl)-2-vinylmorpholine.

(IIa) : X = F, $R_1 = -CH_2-$ 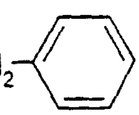 ;

procédé C.

A une solution de 1,2 g du composé obtenu à l'étape précédente dans 12 ml de toluène, on ajoute 0,04 g de chlorure de benzyltriéthylammonium, puis ajoute une solution fraichement préparée et chaude de 2,34 g de NaOH en pastilles dans 2,4 ml d'eau, la température du milieu s'élevant à 45°C. On ajoute alors, en maintenant la température à 50°C, 0,55 ml de chlorure de benzènesulfonyle. Après refroidissement à TA, on ajoute 10 ml d'eau et laisse 1 heure sous agitation. Après décantation, on lave la phase organique par 2 x 50 ml d'eau, sèche sur $Na_2SO_4$ et évapore sous vide le solvant. On chromatographie le résidu sur 60 g de silice en éluant par le mélange cyclohexane/éther diéthylique (90/10 ; v/v). On obtient 0,8 g du produit attendu.

Rendement : 70 %.

G) (S)-(-)-(2-[4-Benzyl-2-(3,4-difluorophényl)morpholin-2-yl]-1-éthanol.

(IIIa) : X = F, $R_1$ = -$CH_2$- 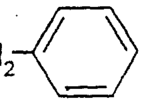 ;

procédé A étape a).

A 0,8 g du composé obtenu à l'étape précédente, on ajoute, sous atmosphère d'azote, 6 ml d'une solution 0,5M de 9-borabicyclo[3.3.1]-nonane dans le THF, amène la température masse à 25°C et laisse 24 heures sous agitation. On rajoute 6 ml de THF, refroidit le mélange réactionnel à 0°C, ajoute, par portions de 5 x 0,2 ml une solution contenant 1 ml d'une solution 11M (130 volumes) de péroxyde d'hydrogène dans l'eau et 0,64 g de NaOH pastilles et laisse 15 minutes sous agitation à TA. On concentre le THF sous vide, extrait le résidu par 10 ml de toluène, lave la phase organique par 2 x 10 ml d'eau, sèche sur $MgSO_4$ et évapore sous vide le solvant. On chromatographie l'huile obtenue sur 20 g de silice en éluant par le mélange DCM/éther diéthylique (73/3 ; v/v). On obtient 0,78 g du produit attendu. Rendement : 93 %.

H) (S)-(-)-2-[2-(3,4-Difluorophényl)morpholin-2-yl]-1-éthanol.

(I) : X = F ;

procédé A étape b).

A 0,078 g de palladium sur charbon à 10 % et 50 % d'humidité on ajoute, sous atmosphère d'azote, une solution de 0,78 g du composé obtenu à l'étape précédente dans 10 ml de MeOH et 0,195 ml d'HCl concentré. On hydrogène pendant 2 heures, à pression atmosphérique et à TA. On filtre le catalyseur et concentre sous vide le filtrat. On obtient 0,5 g du produit attendu.

Rendement : 85 %.

Pureté énantiomérique : 99,95 %.

EXEMPLE 5 - Voie de synthèse V.

(S)-(-)-2-[2-(3,4-Difluorophényl)morpholin-2-yl)-1-éthanol.

(I) : X = F.

A) 2-(3,4-Difluorophényl)-2-hydroxybut-3-ènal, isomère unique.

Ce composé est préparé en utilisant les modes opératoires des étapes A, B, C et D de l'EXEMPLE 4.

B) 2-(3,4-Difluorophénlyl)but-3-ène-1,2-diol, isomère unique.

(XX) : X = F ;

procédé I étape d).

On refroidit à 0°C une solution de 3,2 g du composé obtenu à l'étape précédente dans 110 ml de toluène et ajoute, goutte à goutte, une solution de 0,3 g de borohydrure de sodium dans 10 ml d'EtOH. On hydrolyse le mélange réactionnel par ajout de 100 ml d'une solution saturée de $NH_4Cl$, après décantation lave la phase organique par 3 x 30 ml d'eau, sèche sur $MgSO_4$ et évapore sous vide le solvant. On chromatographie le résidu huileux sur gel de silice en éluant par le mélange DCM/éther diéthylique (90/10 ; v/v). On obtient 1 g du produit attendu.

Rendement : 31 %.

C) 2-(3,4-Difluorophényl)-2-vinyloxirane, isomère unique.

(VII) : X = F ;

procédé I étape e).

A une solution de 2 g de NaOH en pastilles dans 2 ml d'eau, on ajoute une solution de 1 g du composé obtenu à l'étape précédente et 0,04 g de chlorure de benzyltriéthylammonium dans 5 ml de DCM, puis coule, à TA, 0,91

g de chlorure de benzènesulfonyle, la température masse s'élevant à 35°C. On rajoute 5 ml de DCM et laisse 30 minutes sous agitation. On hydrolyse le mélange réactionnel par ajout de 20 ml d'eau et dilue avec 20 ml de DCM. Après décantation, on lave la phase organique par 4 x 10 ml d'eau, sèche sur $MgSO_4$ et évapore sous vide le solvant. On obtient 0,91 g du produit attendu.

Rendement : quantitatif.

D) (S)-(-)-1-[Benzyl(2-hydroxyéthyl)amino]-2-(3,4-difluorophényl)but-3-èn-2-ol.

(IVa) : X = F, $R_1$ = $-CH_2-$ 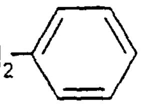 ;

procédé I étape f).

A une solution de 0,894 g du composé obtenu à l'étape précédente dans 4,5 ml d'acétonitrile, on ajoute 0,742 g de 2-(benzylamino)-1-éthanol puis chauffe à reflux pendant 24 heures. On concentre sous vide le mélange réactionnel, reprend le résidu par 20 ml de toluène, lave la phase organique par 3 x 10 ml d'eau, acidifie par ajout de 20 ml d'HCl 0,5N et extrait par 10 ml d'eau. On lave la phase aqueuse acide par 10 ml de toluène, alcalinise par ajout de 0,41 g de NaOH en pastilles, extrait par 20 ml de toluène, lave la phase organique par 3 x 10 ml d'eau, sèche sur $MgSO_4$ et évapore sous vide le solvant. On obtient 0,99 g du produit attendu.

Rendement : 60 %.

Pureté énantiomèrique : 98,8 % (e.e. = 97,6 %).

E) (S)-(-)-2-[2-(3,4-Difluorophényl)morpholin-2-yl]-1-éthanol.

(I) : X = F ;

procédé C puis procédé A étapes a) et b).

On procède comme aux étapes F, G et H de l'EXEMPLE 4, à partir du composé obtenu à l'étape précédente, et obtient le produit attendu.

EXEMPLE 6 - Voie de synthèse VI.

(R)-(+)-2-[2-(3,4-Difluorophényl)morpholin-2-yl]-1-éthanol, sel avec l'acide L-(-)-diparatoluoyltartrique.

(I) : X = F.

A) Maléate de 4-benzyl-2-(3,4-difluorophényl)-2-vinylmorpholine, (IIa),

(IIa) : $C_4H_4O_4$ : X = F, $R_1$ = $-CH_2-$ 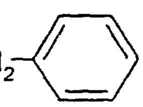

Ce composé est préparé en utilisant les modes opératoires des étapes A, B, C et D de l'Exemple 1.

B) 2-(3,4-Difluorophényl)-2-vinylmorpholine-4-carboxylate de benzyle.

(IIb) : X = F, $R_1$ = $-COO-CH_2-$ 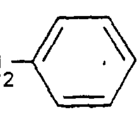 ; procédé B.

On chauffe à 60°C un mélange de 41,98 g du composé obtenu à l'étape A sous forme de base libre (huile) et 0,55 g de $K_2CO_3$, puis ajoute, sous atmosphère d'azote, en goutte à goutte et en 25 minutes, 28,68 g de chloroformiate de benzyle et laisse 5 heures sous agitation à 20 C°. On ajoute au mélange réactionnel encore chaud 100 ml de toluène et 70 ml d'eau, puis après décantation, lave la phase organique à l'eau, sèche sur $MgSO_4$ et

concentre sous vide le solvant. On obtient 64 g du produit attendu qui est utilisé tel quel à l'étape suivante.
C) 2-(3,4-Difluorophényl)-2-(2-hydroxyéthyl)morpholine-4-carboxylate de benzyle.

(IIIb) : X = F, $R_1$ = -COO-$CH_2$- 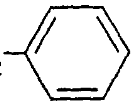 ;

procédé A étape a).

A un mélange de 2,21 g de borohydrure de sodium dans 50 ml de THF, on ajoute, sous atmosphère d'azote et en 15 minutes, une solution de 6,35 g de chlorure de triméthyl silyle dans 8 ml de THF et laisse 30 minutes sous agitation à TA. On ajoute ensuite, en goutte à goutte, une solution de 7,17 g du composé obtenu à l'étape précédente dans 11 ml de THF, laisse 1 heure sous agitation à TA, puis chauffe à reflux pendant 1 heure et laisse une nuit sous agitation en laissant revenir la température à TA. On ajoute au mélange réactionnel, en goutte à goutte et avec précaution, 1,05 ml d'eau puis concentre sous vide le THF. On reprend le résidu par 60 ml de toluène, ajoute 0,24 g d'hydrogènosulfate de tétrabutylammonium, puis lentement 5,4 ml de NaOH 10N et ensuite 3,8 ml d'une solution 11M de peroxyde d'hydrogène dans l'eau (33 %, 130 volumes, d = 1,13), la température masse atteignant 45°C. Après 15 minutes sous agitation, on ajoute 60 ml d'eau au mélange réactionnel, après décantation, lave deux fois la phase organique à l'eau (pH = 7), sèche sur $MgSO_4$ et évapore sous vide le solvant. On obtient 4,83 g du produit attendu que l'on utilise tel quel à l'étape suivante.
D) 2-[2-(3,4-Difluorophényl)morpholin-2-yl]-1-éthanol, racémique.

(I) : X = F ;

procédé A étape b).

A 0,05 g de palladium sur charbon à 10 % et 50 % d'humidité, on ajoute, sous atmosphère d'azote, une solution de 0,77 g du composé obtenu à l'étape précédente dans 2,3 ml de toluène. On hydrogène pendant 2 heures et 30 minutes, à pression atmosphérique et à 40°C. Après refroidissement à TA, on ajoute de l'eau au mélange, acidifie par ajout de 0,105 ml d'HCl concentré et filtre. Après décantation on lave la phase aqueuse acide au toluène, alcalinise par ajout de 0,141 ml de NaOH 10N et laisse 1 heure sous agitation à TA. On essore le précipité formé, le lave à l'eau et le sèche sous vide à 60°C. On obtient 0,17 g du produit attendu.
E) (R)-(+)-2-[2-(3,4-Difluorophényl)morpholin-2-yl]-1-éthanol, sel avec l'acide L-(-)-diparatoluoyltartrique.

(I) : X = F ;

procédé A étape c).

On procède comme à l'étape G de l'EXEMPLE 1, à partir du composé obtenu à l'étape précédente, et obtient le produit attendu.

## Revendications

**1.** Procédé de préparation d'un composé, sous forme énantiomériquement pure, de formule :

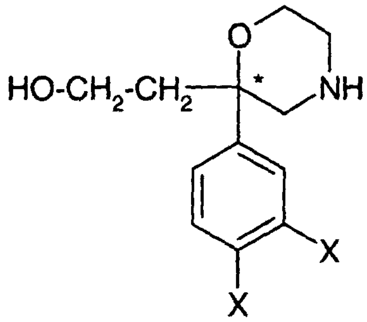

(I)

dans laquelle X représente un atome d'halogène, de ses sels avec des acides minéraux ou organiques ou de ses sels avec des acides organiques optiquement actifs, **caractérisé en ce que** :

a) on transforme un composé, sous forme racémique, sous forme d'un mélange de diastéréoisomères ou sous forme énantiomériquement pure, de formule :

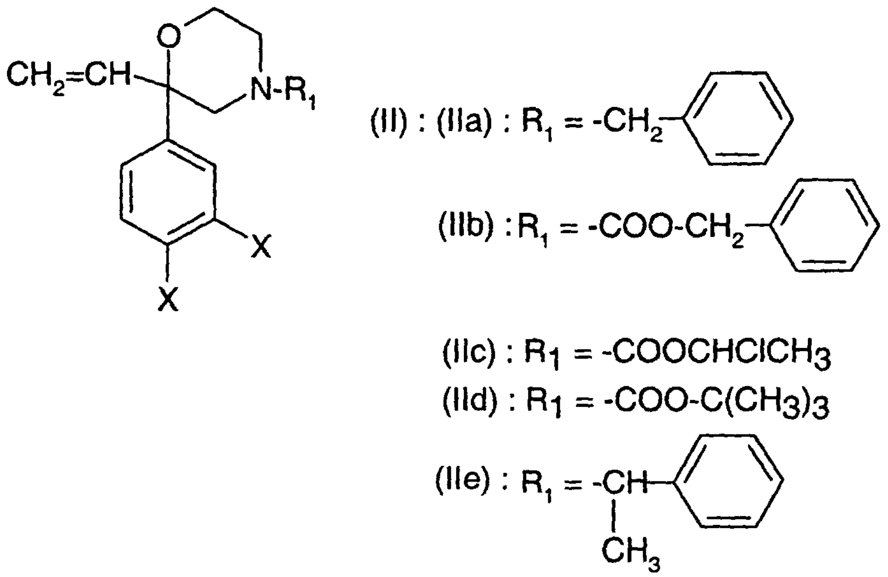

(II) : (IIa) : $R_1$ = $-CH_2-$

(IIb) : $R_1$ = $-COO-CH_2-$

(IIc) : $R_1$ = $-COOCHClCH_3$

(IId) : $R_1$ = $-COO-C(CH_3)_3$

(IIe) : $R_1$ =

dans laquelle X est tel que défini pour un composé de formule (I) et $R_1$ représente un groupe N-protecteur choisi parmi un groupe benzyle, un groupe benzyloxycarbonyle, un groupe 1-chloroéthyloxycarbonyle, un groupe *tert*-butyloxycarbonyle ou un groupe α-méthylbenzyle, en un composé, sous forme racémique, sous forme d'un mélange de diastéréoisomères ou sous forme énantiomériquement pure, de formule :

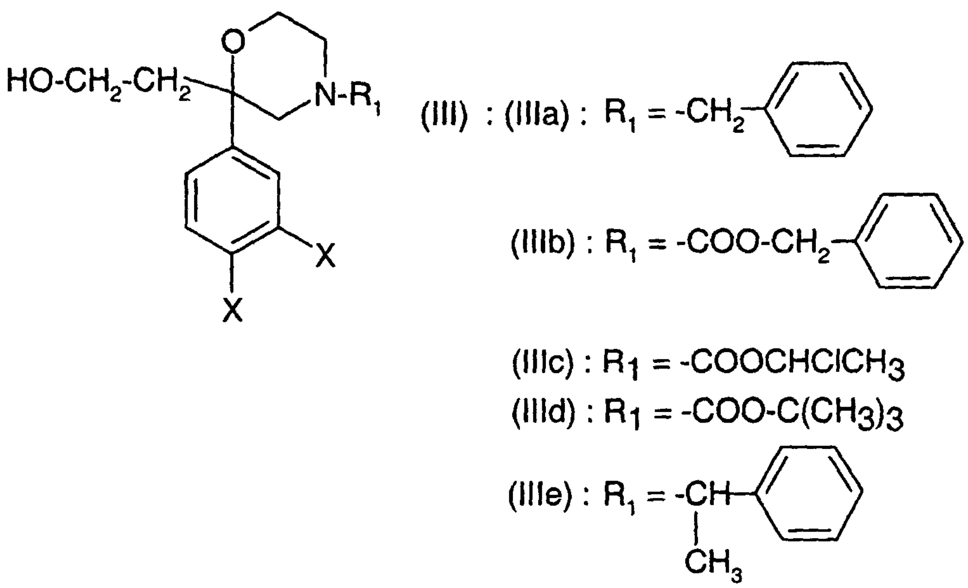

(III) : (IIIa) : $R_1$ = $-CH_2-$

(IIIb) : $R_1$ = $-COO-CH_2-$

(IIIc) : $R_1$ = $-COOCHClCH_3$

(IIId) : $R_1$ = $-COO-C(CH_3)_3$

(IIIe) : $R_1$ =

b) on déprotège le composé de formule (III) ainsi obtenu ;

c) le cas échéant, lorsque le composé de formule (I) ainsi obtenu est sous forme racémique, on sépare les énantiomères, et, éventuellement, on transforme le composé de formule (I), énantiomériquement pur, en l'un de ses sels avec des acides minéraux ou organiques.

**2.** Procédé selon la revendication 1 **caractérisé en ce que** l'on prépare un composé, sous forme énantiomériquement pure ou sous forme racémique, de formule :

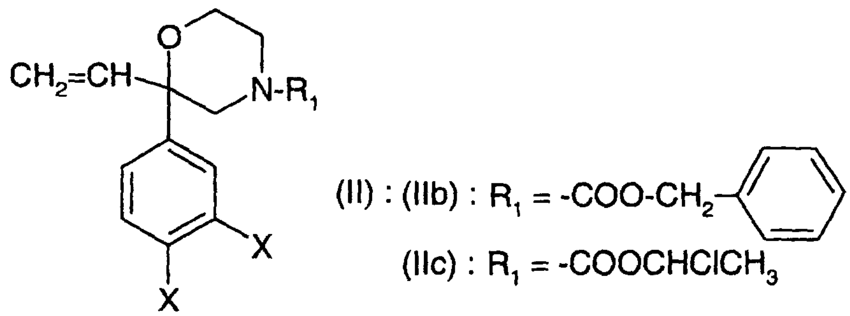

dans laquelle X représente un atome d'halogène et $R_1$ représente un groupe benzyloxycarbonyle ou un groupe 1-chloroéthyloxycarbonyle, par réaction d'un composé, sous forme énantiomériquement pure ou sous forme racémique, de formule :

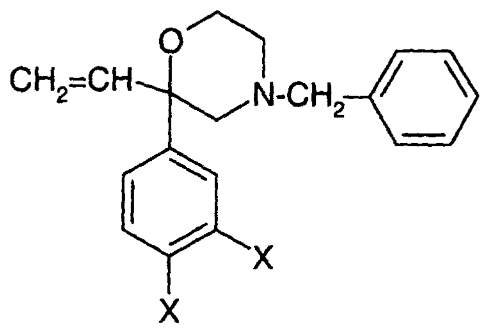

(IIa)

dans laquelle X est tel que défini pour un composé de formule (II), avec du chloroformiate de benzyle ou du chloroformiate de 1-chloroéthyle, en présence d'une base, avec ou sans solvant.

**3.** Procédé selon l'une des revendications 1 ou 2 **caractérisé en ce que** l'on prépare un composé, sous forme énantiomériquement pure, sous forme d'un mélange de diastéréoisomères ou sous forme racémique, de formule :

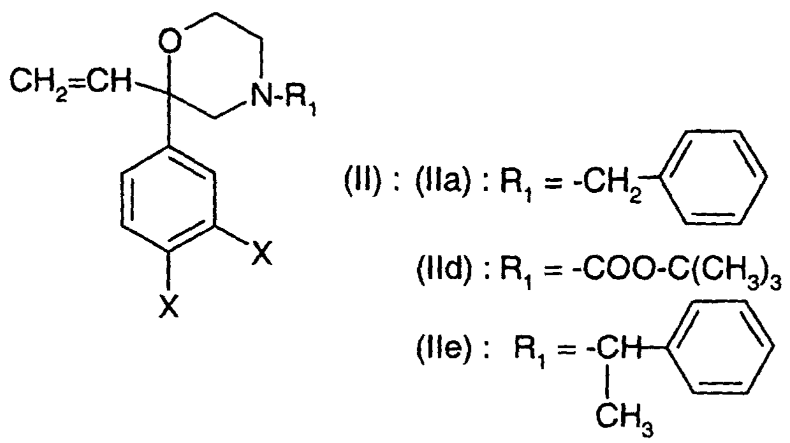

dans laquelle X représente un atome d'halogène et $R_1$ représente un groupe benzyle, un groupe *tert*-butyloxycarbonyle ou un groupe $\alpha$-méthylbenzyle, de ses sels éventuels avec des acides minéraux ou organiques, par cyclisation d'un composé, sous forme énantiomériquement pure, sous forme d'un mélange de diastéréoisomères ou sous forme de racémique, de formule :

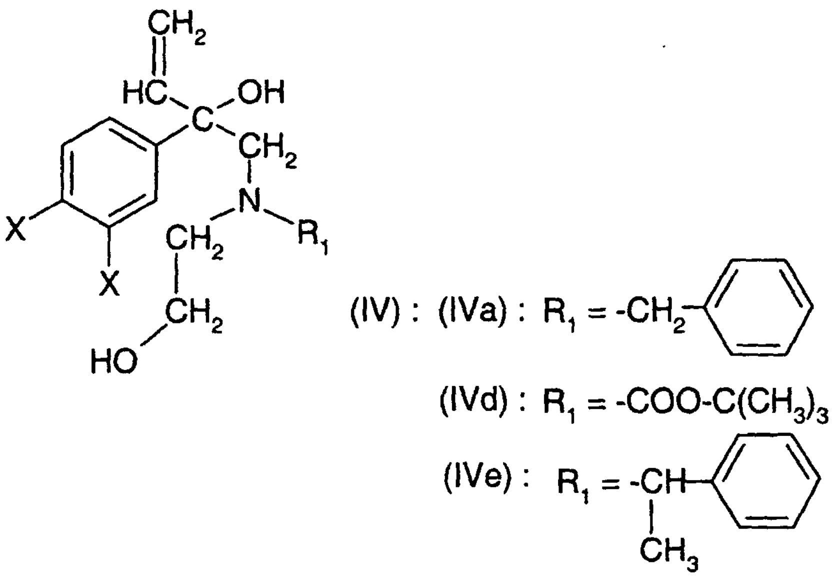

(IV) : (IVa) : $R_1$ = $-CH_2-$

(IVd) : $R_1$ = $-COO-C(CH_3)_3$

(IVe) : $R_1$ = -CH-

$CH_3$

dans laquelle X et $R_1$ sont tels que définis pour un composé de formule (II), et, éventuellement on transforme le composé de formule (II) ainsi obtenu en un de ses sels.

4. Procédé selon la revendication 3 **caractérisé en ce que** l'on prépare un composé, sous forme énantiomériquement pure, de formule :

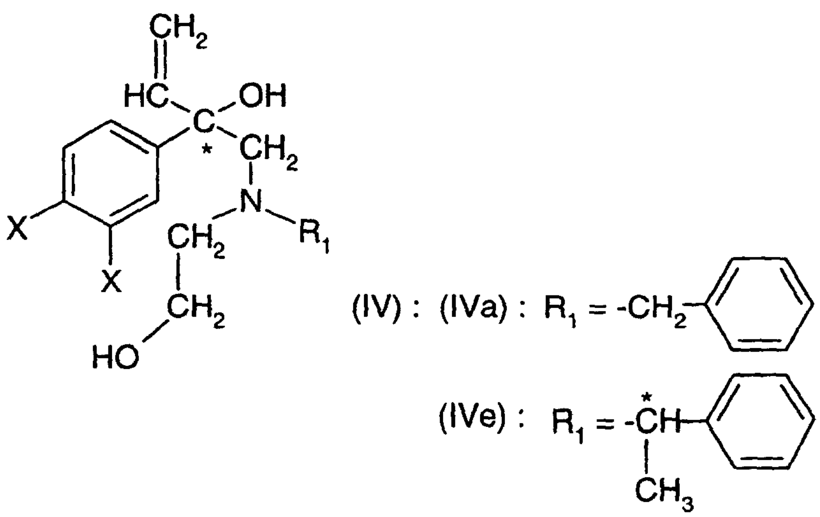

(IV) : (IVa) : $R_1$ = $-CH_2-$

(IVe) : $R_1$ = $-\overset{*}{C}H-$

$CH_3$

dans laquelle X représente un atome d'halogène et $R_1$ représente un groupe benzyle ou un groupe α-méthylbenzyle, de ses sels avec des acides minéraux ou organiques :

a) par réaction d'un composé, sous forme racémique, de formule :

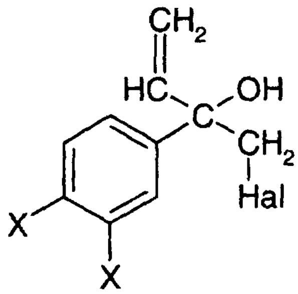

(V)

dans laquelle X est tel que défini pour un composé de formule (IV) et Hal représente un atome d'halogène, avec la benzylamine ou avec la (R)(+) ou la (S)(-)-α-méthylbenzylamine, en présence d'une base, dans un solvant inerte, pour obtenir un composé, sous forme racémique, de formule :

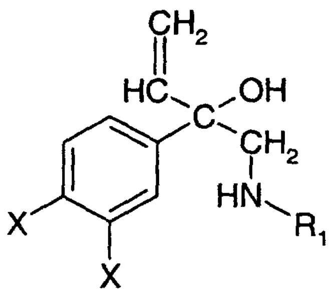

(VI) : (VIa) : 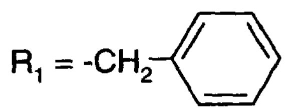

(VIe) : 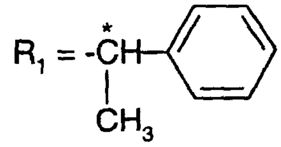

b) par séparation des énantiomères ou des diastéréoisomères du composé de formule (VI) ainsi obtenu ;
c) par réaction du composé de formule (VI), énantiomériquement pur ainsi obtenu :

- soit avec de l'oxyde d'éthylène, en présence catalytique d'un acide, dans un solvant inerte ;
- soit avec un composé de formule Hal''''-$CH_2$-$CH_2$-O-$R_2$ (XXI) dans laquelle $R_2$ représente un groupe O-protecteur et Hal'''' représente un atome d'halogène, en présence d'une base, dans un solvant inerte, suivi de la déprotection du groupe O-protecteur :

et éventuellement, par transformation du composé de formule (IV), énantiomériquement pur, ainsi obtenu en l'un de ses sels avec des acides minéraux ou organiques.

**5.** Procédé selon la revendication 4 **caractérisé en ce qu'**à l'étape a), on utilise un composé de formule (V) dans laquelle Hal représente un atome de chlore ou de brome.

**6.** Procédé selon la revendication 3 **caractérisé en ce que** l'on prépare un composé, sous forme racémique ou sous forme d'un mélange de diastéréoisomères, de formule :

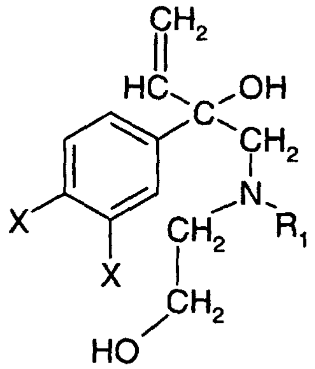

(IV) : (IVa) : 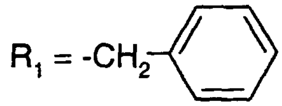

(IVd) : $R_1 = -COO-C(CH_3)_3$

(IVe) : 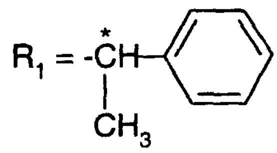

dans laquelle X représente un atome d'halogène et $R_1$ représente le groupe benzyle, le groupe *tert*-butyloxycarbonyle ou le groupe $\alpha$-méthylbenzyle, ou l'un de ses sels éventuels avec des acides minéraux ou organiques :

a) par réaction d'un composé, sous forme racémique, de formule :

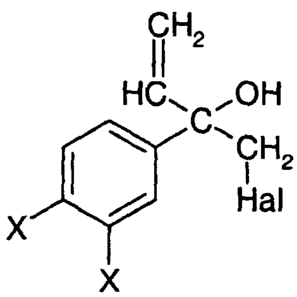

(V)

dans laquelle X est tel que défini pour un composé de formule (IV) et Hal représente un atome d'halogène, soit avec du 2-(benzylamino)-1-éthanol, soit avec du 2-amino-1-éthanol ou soit avec du (R) ou (S)-2-(a-méthylbenzylamino)-1-éthanol, en présence d'une base et dans un solvant inerte, et, éventuellement par transformation du composé de formule (IVa) ou (IVe) ainsi obtenu en un de ses sels avec des acides minéraux ou organiques.

b) le cas échéant, lorsqu'à l'étape a), le composé de formule (V) est mis en oeuvre avec du 2-amino-1-éthanol, par réaction du composé ainsi obtenu de formule :

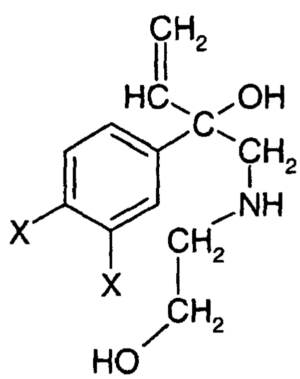

(IV'd)

avec du di-*tert*-butyldicarbonate, en présence d'une base et dans un solvant inerte pour obtenir le composé de formule (IVd).

**7.** Procédé selon la revendication 6 **caractérisé en ce qu'**on utilise un composé de formule (V) dans laquelle Hal représente un atome de chlore ou de brome.

**8.** Procédé selon l'une des revendications 4 ou 6 **caractérisé en ce que** l'on prépare un composé de formule :

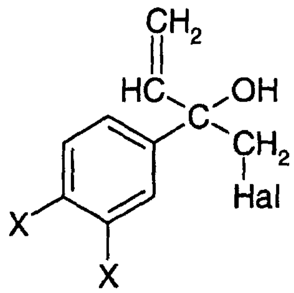

(V)

dans laquelle X représente un atome d'halogène et Hal représente un atome d'halogène :

a) par réaction d'un composé de formule :

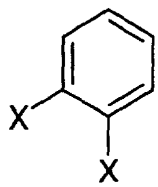

(VIII)

dans laquelle X est tel que défini pour un composé de formule (V), avec un composé de formule :

$$Hal'\text{-}CO\text{-}CH_2\text{-}Hal \qquad (IX)$$

dans laquelle Hal' et Hal représentent un atome d'halogène, en présence d'un acide de Lewis et dans un solvant inerte, pour obtenir un composé de formule :

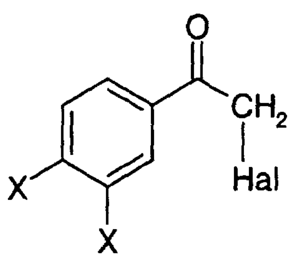

(X)

b) par réaction du composé de formule (X) ainsi obtenu avec un composé de formule :

$$CH_2 = CH\text{-}Mg\text{-}Hal'' \qquad (XI)$$

dans laquelle Hal" représente un atome d'halogène, dans un solvant inerte, suivi d'une hydrolyse pour obtenir le composé de formule (V).

**9.** Procédé selon la revendication 8 **caractérisé en ce que** l'on prépare un composé de formule (V) dans laquelle Hal représente un atome de chlore ou de brome.

**10.** Procédé selon la revendication 8 **caractérisé en ce qu'**à l'étape a) on utilise un composé de formule (IX) dans laquelle Hal' et Hal représentent chacun indépendamment un atome de chlore ou de brome et à l'étape b) on utilise un composé de formule (XI) dans laquelle Hal" représente un atome de chlore ou de brome.

**11.** Procédé selon la revendication 3 **caractérisé en ce que** l'on prépare un composé, sous forme racémique, de formule :

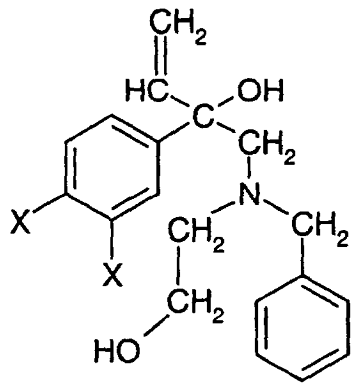

(IVa)

dans laquelle X représente un atome d'halogène, ou l'un de ses sels avec des acides minéraux ou organiques

a) par réaction d'un composé de formule :

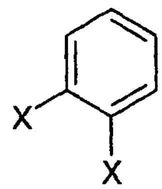

(VIII)

dans laquelle X est tel que défini pour un composé de formule (IVa), avec un composé de formule :

$$Hal'\text{-}CO\text{-}CH_2\text{-}Hal \quad (IX)$$

dans laquelle Hal' et Hal représentent un atome d'halogène, en présence d'un acide de Lewis et dans un solvant inerte, pour obtenir un composé de formule :

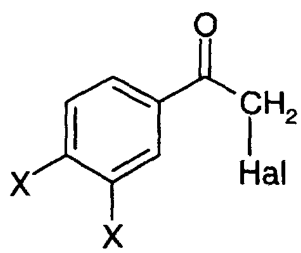

(X)

b) par réaction du composé de formule (X) ainsi obtenu, avec un composé de formule :

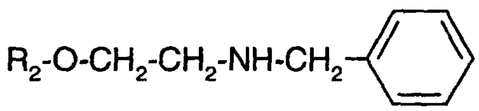

(XII)

dans laquelle $R_2$ représente un groupe O-protecteur, en présence d'une base et dans un solvant inerte, pour obtenir un composé de formule :

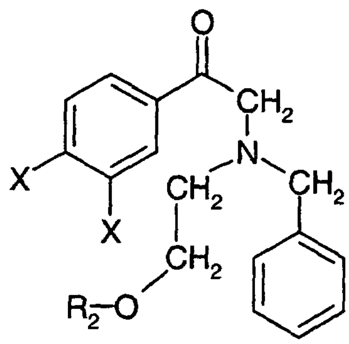

(XIII)

c) par réaction du composé de formule (XIII) ainsi obtenu avec un composé de formule :

$$CH_2=CH\text{-}Mg\text{-}Hal'' \qquad (XI)$$

dans laquelle Hal" représente un atome d'halogène, dans un solvant inerte, suivi d'une hydrolyse, pour obtenir un composé de formule :

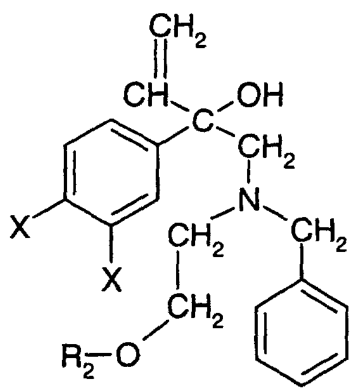

(XIV)

d) par déprotection du composé de formule (XIV) et, éventuellement, par transformation du composé de formule (IVa) ainsi obtenu en un de ses sels avec des acides minéraux ou organiques.

**12.** Procédé selon la revendication 11 **caractérisé en ce qu'**à l'étape a) on utilise un composé de formule (IX) dans laquelle Hal et Hal' représentent chacun indépendamment un atome de chlore ou de brome, à l'étape b) on utilise un composé de formule (XII) dans laquelle $R_2$ représente un groupe tétrahydropyran-2-yle et à l'étape c) on utilise un composé de formule (XI) dans laquelle Hal" représente un atome de chlore ou de brome.

**13.** Procédé selon la revendication 3 **caractérisé en ce que** l'on prépare un composé, sous forme énantiomériquement pure, de formule :

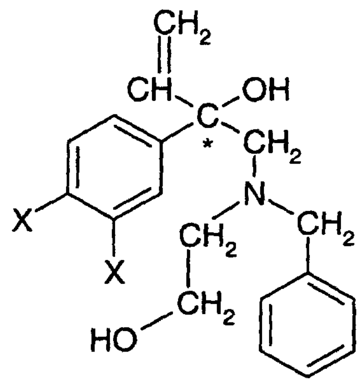

(IVa)

dans laquelle X représente un atome d'halogène, ou l'un de ses sels avec des acides minéraux ou organiques :

a) par réaction d'un composé de formule :

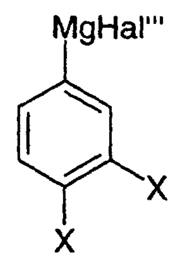

(XV)

dans laquelle X est tel que défini pour un composé de formule (IVa) et Hal''' représente un atome d'halogène, avec le (R)- ou le (S)-2-phénylhexahydro-pyrrolo[1,2-c]imidazole-3-carboxylate de méthyle, de formule :

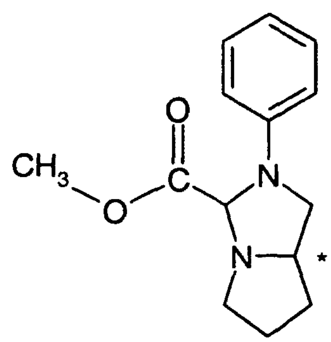

(XVI)

en présence de chlorure de magnésium, dans un solvant inerte, suivi d'une hydrolyse, pour obtenir un composé, sous forme énantiomériquement pure, de formule :

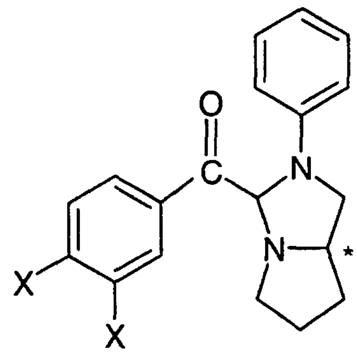

(XVII)

b) par réaction du composé de formule (XVII) ainsi obtenu avec un composé de formule :

$$CH_2 = CH\text{-}Mg\text{-}Hal'' \qquad (XI)$$

dans laquelle Hal" représente un atome d'halogène, dans un solvant inerte, suivi d'une hydrolyse, pour obtenir un composé, sous forme énantiomériquement pure, de formule :

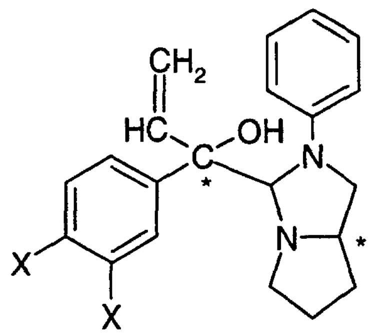

(XVIII)

c) par hydrolyse du composé de formule (XVIII) ainsi obtenu par action d'un acide, dans un solvant inerte en mélange avec de l'eau, pour obtenir un composé, sous forme énantiomériquement pure, de formule :

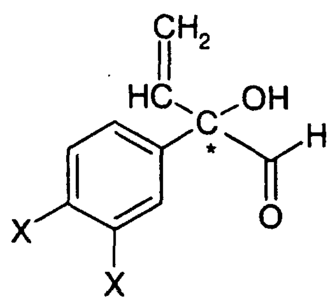

(XIX)

d) par réaction du composé de formule (XIX) ainsi obtenu avec du 2-(benzylamino)-1-éthanol en présence d'un acide, dans un solvant inerte, puis réduction du sel d'iminium formé intermédiairement au moyen d'un agent réducteur, et, éventuellement, transformation du composé de formule (IVa), énantiomériquement pur, en l'un de ses sels avec des acides minéraux ou organiques.

**14.** Procédé selon la revendication 3 **caractérisé en ce que** l'on prépare un composé, sous forme énantiomériquement pure, de formule :

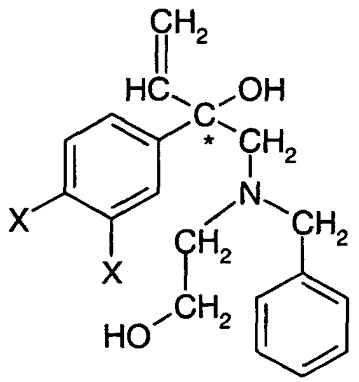

(IVa)

dans laquelle X représente un atome d'halogène, ou l'un de ses sels avec des acides minéraux ou organiques :

a) par réaction d'un composé de formule :

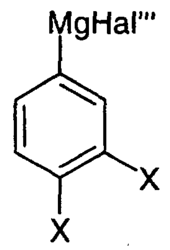

(XV)

dans laquelle X est tel que défini pour un composé de formule (IVa) et Hal''' représente un atome d'halogène, avec le (R)- ou le (S)-2-phénylhexahydro-pyrrolo[1,2-c]imidazole-3-carboxylate de méthyle, de formule :

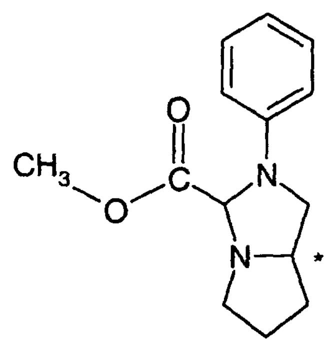

(XVI)

en présence de chlorure de magnésium, dans un solvant inerte, suivi d'une hydrolyse, pour obtenir un composé, sous forme énantiomériquement pure, de formule :

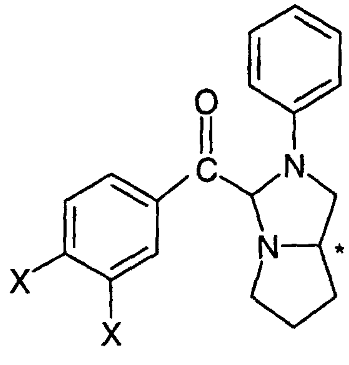

(XVII)

b) par réaction du composé de formule (XVII) ainsi obtenu avec un composé de formule :

$$CH_2 = CH\text{-}Mg\text{-}Hal'' \qquad (XI)$$

dans laquelle Hal" représente un atome d'halogène, dans un solvant inerte, suivi d'une hydrolyse, pour obtenir un composé, sous forme énantiomériquement pure, de formule :

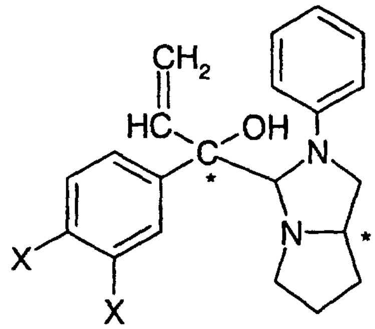

(XVIII)

c) par hydrolyse du composé de formule (XVIII) ainsi obtenu par action d'un acide, dans un solvant inerte en mélange avec de l'eau, pour obtenir un composé, sous forme énantiomériquement pure, de formule :

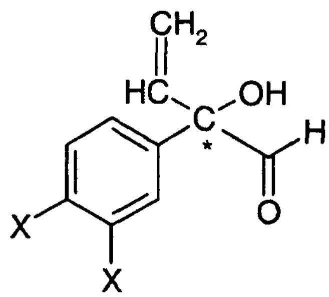

(XIX)

d) par réduction du composé de formule (XIX) ainsi obtenu, au moyen d'un agent réducteur, dans un solvant inerte, pour obtenir un composé de formule :

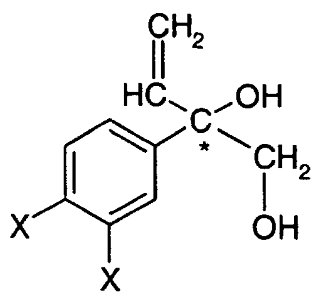

(XX)

e) par cyclisation du composé de formule (XX) ainsi obtenu, pour obtenir un composé, sous forme énantiomériquement pure, de formule :

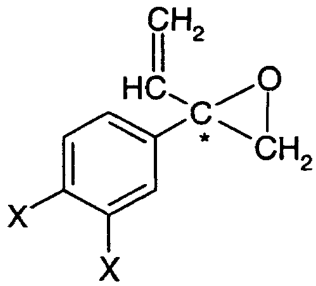

(VII)

f) par réaction du composé de formule (VII) ainsi obtenu avec du 2-(benzylamino)-1-éthanol, en présence d'une base et dans un solvant inerte, et, éventuellement, transformation du composé de formule (IVa), énantiomériquement pur, ainsi obtenu en l'un de ses sels avec des acides minéraux ou organiques.

**15.** Procédé selon l'une des revendications 13 ou 14 **caractérisé en ce qu'**à l'étape a) on utilise un composé de formule (XV) dans laquelle Hal''' représente un atome de chlore ou de brome et à l'étape b) on utilise un composé de formule (XI) dans laquelle Hal'' représente un atome de chlore ou de brome.

**16.** Procédé selon l'une des revendications 1, 2, 3, 4, 6, 8, 11, 13 ou 14 **caractérisé en ce que** l'on prépare des composés de formule (I), (IIa), (IIb), (IIc), (IId), (IIe), (IVa), (IVd), (IVe) ou (V) dans lesquelles X représentent un atome de chlore ou un atome de fluor.

**17.** Composé de formule :

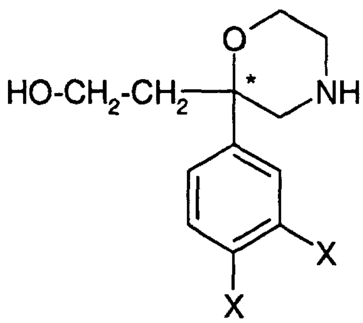

(I)

dans laquelle X représente un atome d'halogène, énantiomériquement pur, sous forme de sel optiquement actif avec l'acide L-(-) ou D-(+)-diparatoluoyltartrique.

**18.** Composé de formule :

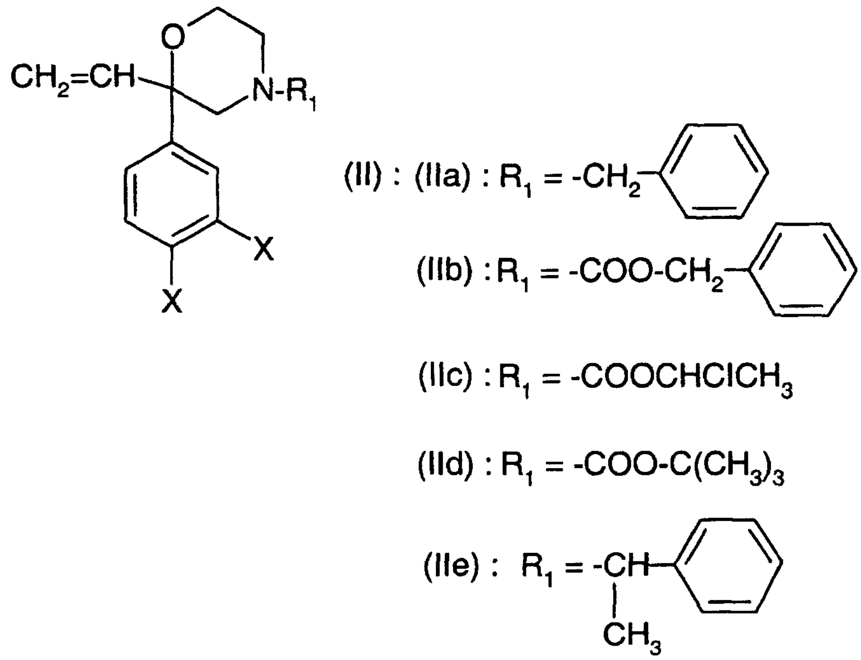

(II) : (IIa) :

(IIb) :

(IIc) :

(IId) :

(IIe) :

dans laquelle X représente un atome d'halogène et $R_1$ représente un groupe N-protecteur choisi parmi un groupe benzyle, un groupe benzyloxycarbonyle, un groupe 1-chloroéthyloxycarbonyle, un groupe *tert*-butyloxycarbonyle ou un groupe α-méthylbenzyle, sous forme racémique, sous forme énantiomériquement pure ou sous forme d'un mélange de diastéréoisomères et ses sels éventuels avec des acides minéraux ou organiques.

**19.** Composé de formule :

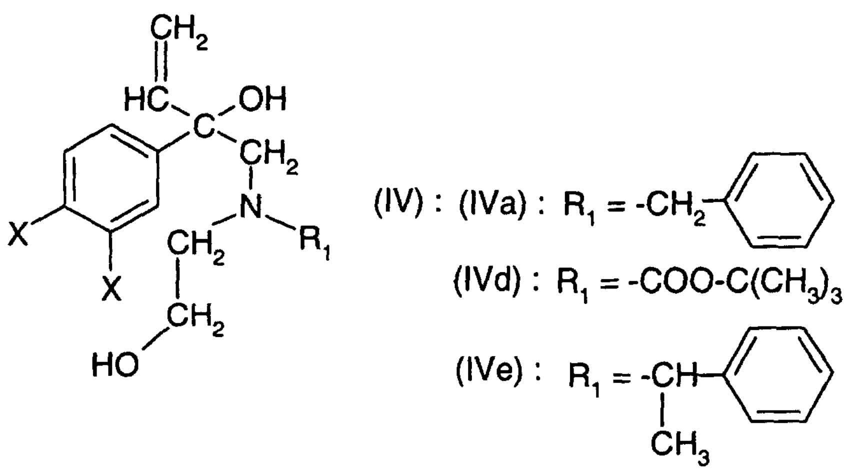

(IV) : (IVa) :

(IVd) :

(IVe) :

dans laquelle X représente un atome d'halogène et $R_1$ représente un groupe benzyle, un groupe *tert*-butyloxycarbonyle ou un groupe α-méthylbenzyle, sous forme racémique, sous forme énantiomériquement pure ou sous forme d'un mélange de diastéréoisomères et ses sels éventuels avec des acides minéraux ou organiques.

**20.** Composé de formule :

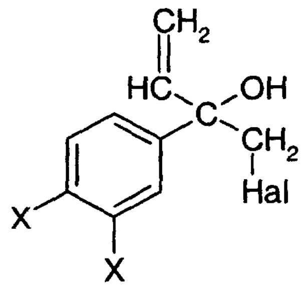

(V)

dans laquelle X représente un atome d'halogène et Hal représente un atome d'halogène.

**21.** Composé selon la revendication 20 de formule (V) dans laquelle Hal représente un atome de chlore ou de brome.

**22.** Composé de formule :

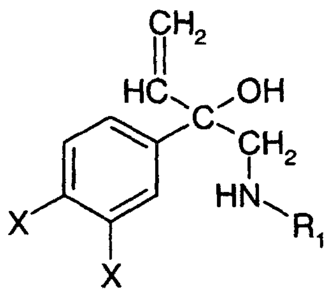

(VI) : (VIa) : $R_1$ =

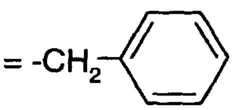

(VIe) : $R_1$ =

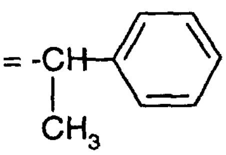

dans laquelle X représente un atome d'halogène et $R_1$ représente un groupe benzyle ou un groupe α-méthylbenzyle, sous forme racémique, sous forme énantiomériquement pure ou sous forme d'un mélange de diastéréoisomères, et ses sels avec des acides minéraux ou organiques ou ses sels avec des acides optiquement actifs.

**23.** Composé de formule (VIa) selon la revendication 22, énantiomériquement pur, sous forme de sel avec l'acide L-(+) ou D-(-)-mandélique.

**24.** Composé de formule :

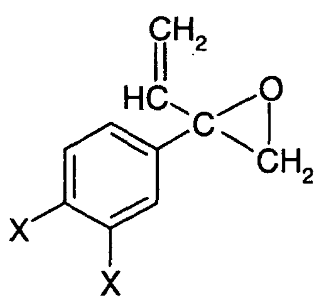

(VII)

dans laquelle X représente un atome d'halogène, sous forme racémique ou sous forme énantiomériquement pure.

**25.** Composé de formule :

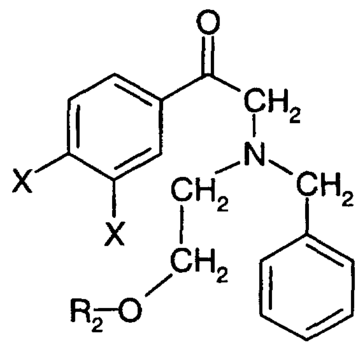

(XIII)

dans laquelle X représente un atome d'halogène et $R_2$ représente un groupe tétrahydropyran-2-yle, et ses sels avec des acides minéraux ou organiques.

**26.** Composé de formule :

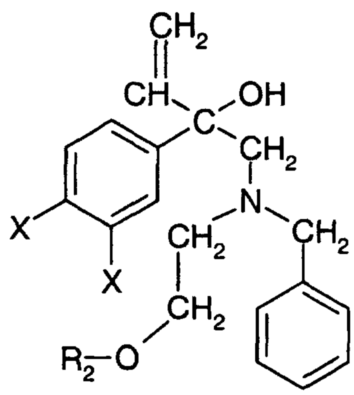

(XIV)

dans laquelle X représente un atome d'halogène et $R_2$ représente un groupe tétrahydropyran-2-yle, et ses sels avec des acides minéraux ou organiques.

**27.** Composé de formule :

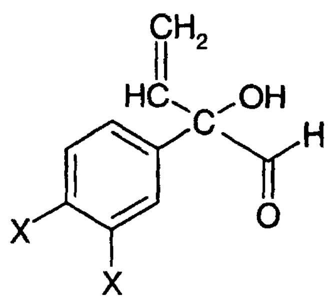

(XIX)

dans laquelle X représente un atome d'halogène, sous forme racémique ou sous forme énantiomériquement pure.

**28.** Composé de formule :

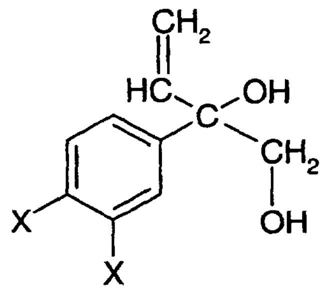

(XX)

dans laquelle X représente un atome d'halogène, sous forme racémique ou sous forme énantiomériquement pure.

**29.** Composé selon l'une des revendications 17 à 28 de formule (I), (II), (IV), (V), (VI), (VII), (XIII), (XIV), (XIX) ou (XX) dans lesquelles X représente un atome de chlore ou un atome de fluor.

**Patentansprüche**

**1.** Verfahren zur Herstellung von Verbindungen der folgenden Formel in Form der reinen Enantiomere:

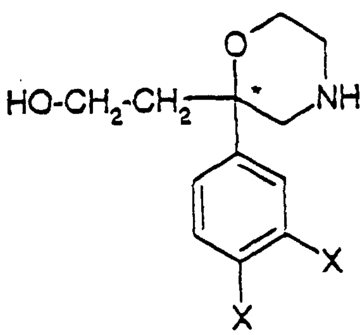

(I),

worin X ein Halogenatom bedeutet, ihrer Salze mit anorganischen oder organischen Säuren oder ihrer Salze mit optisch aktiven organischen Säuren, **dadurch gekennzeichnet, dass**

a) eine Verbindung, die als Racemat, als Gemisch von Diastereoisomeren oder in Form von reinen Enantiomeren vorliegt, der folgenden Formel:

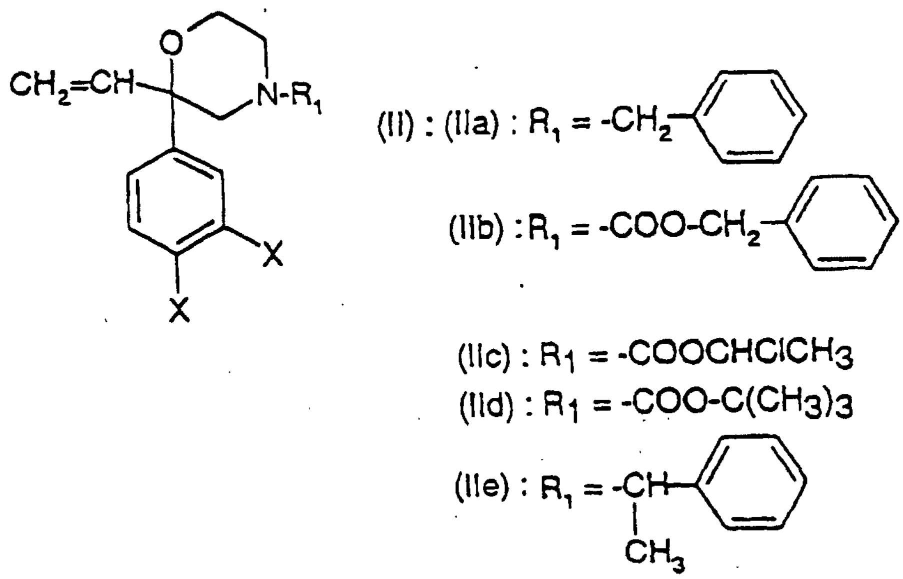

(II) ,

worin X die für die Verbindungen der Formel (I) angegebene Bedeutung aufweist und $R_1$ eine N-Schutzgruppe bedeutet, die unter Benzyl, Benzyloxycarbonyl, 1-Chlorethyloxycarbonyl, *t*-Butyloxycarbonyl oder α-Methylbenzyl ausgewählt ist, in Form des Racemats, in Form eines Gemisches von Diastereoisomeren oder in Form der reinen Enantiomere in eine Verbindung der folgenden Formel umgewandelt wird:

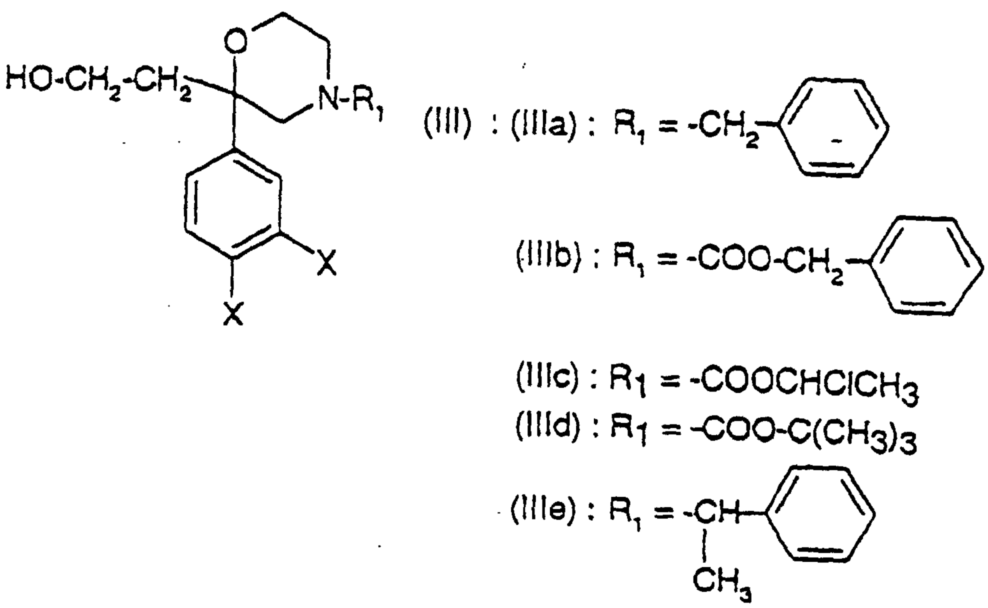

(III) ;

b) die Schutzgruppe von der auf diese Weise erhaltenen Verbindung (III) entfernt wird; und

c) gegebenenfalls die Enantiomeren getrennt werden, wenn die erhaltene Verbindung der Formel (I) als Racemat vorliegt, und gegebenenfalls die reinen Enantiomere der Formel (I) in eines ihrer Salze mit anorganischen oder organischen Säuren überführt werden.

2. Verfahren nach Anspruch 1, **dadurch gekennzeichnet, dass** in Form der reinen Enantiomere oder als Racemat eine Verbindung der folgenden Formel hergestellt wird:

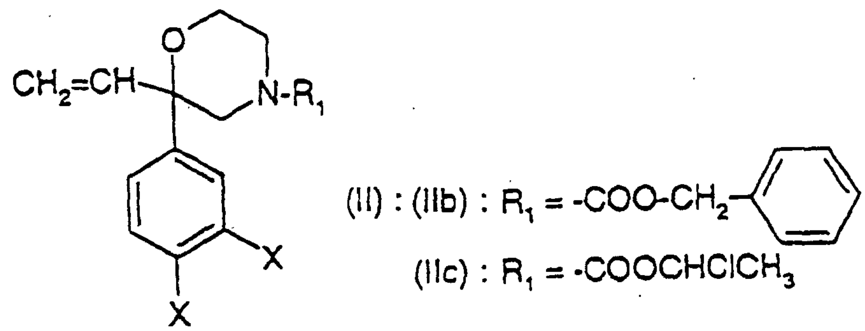

,

worin X ein Halogenatom bedeutet und $R_1$ eine Benzyloxycarbonylgruppe oder eine 1-Chlorethyloxycarbonylgruppe ist, indem eine enantiomer rein oder als Racemat vorliegende Verbindung der folgenden Formel:

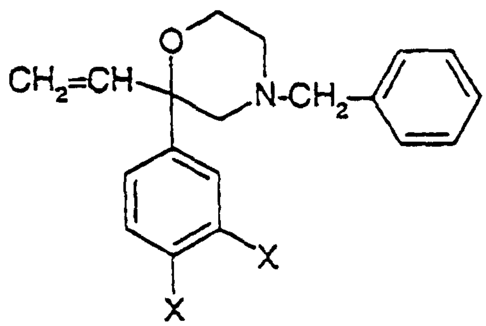

**(IIa),**

worin X die für die Verbindung der Formel (II) angegebene Bedeutung aufweist, mit Benzylchlorformiat oder 1-Chlorethylchlorformiat in Gegenwart einer Base und in Gegenwart oder Abwesenheit eines Lösungsmittels umgesetzt wird.

3. Verfahren nach einem der Ansprüche 1 oder 2, **dadurch gekennzeichnet, dass** in Form der reinen Enantiomere, in Form eines Gemisches von Diastereoisomeren oder in Form des Racemats eine Verbindung der folgenden Formel hergestellt wird:

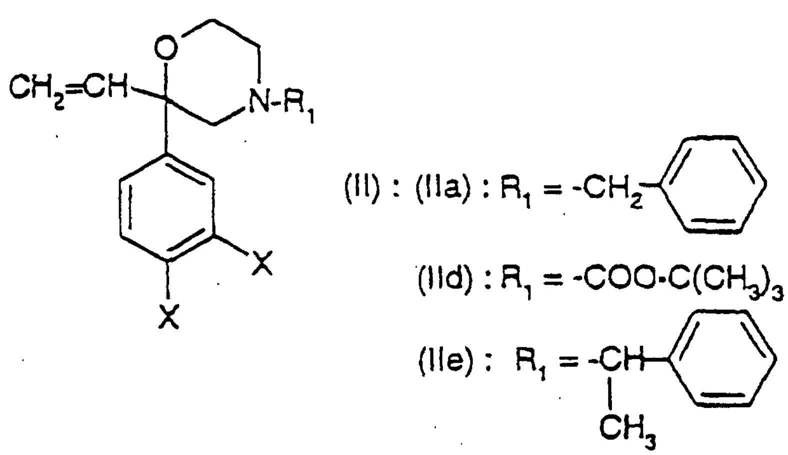

;

worin X ein Halogenatom bedeutet und $R_1$ Benzyl, *t*-Butyloxycarbonyl oder $\alpha$-Methylbenzyl bedeutet, und gegebenenfalls ihre Salze mit anorganischen oder organischen Säuren durch Cyclisierung einer in Form der reinen

Enantiomere, in Form eines Gemisches von Diastereoisomere oder in Form des Racemats vorliegenden Verbindung der folgenden Formel:

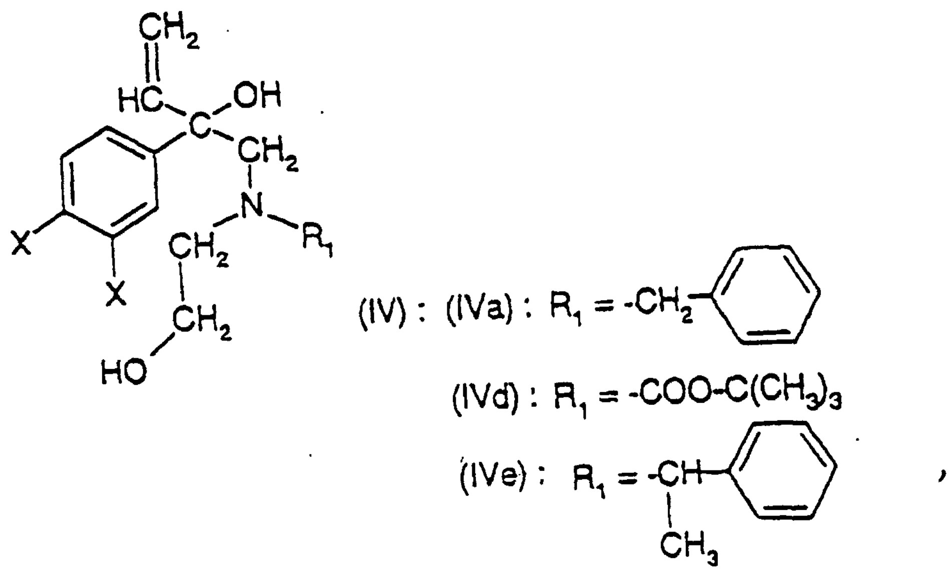

(IV) : (IVa) : (IVd) : (IVe) : ,

worin X und $R_1$ die oben für die Verbindung der Formel (I) angegebenen Bedeutungen aufweisen, und die auf diese Weise erhaltene Verbindung der Formel (II) gegebenenfalls in eines ihrer Salze umgewandelt wird.

**4.** Verfahren nach Anspruch 3, **dadurch gekennzeichnet, dass** die reinen Enantiomere der folgenden Formel hergestellt werden:

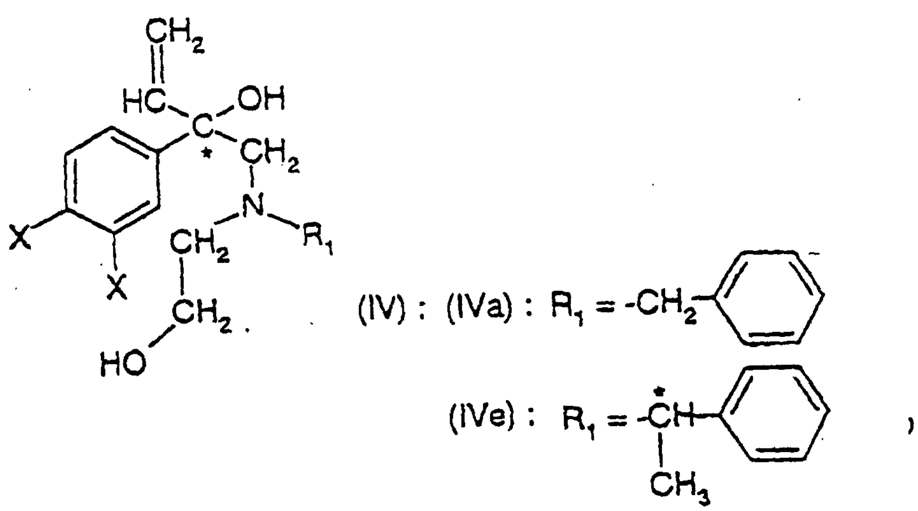

(IV) : (IVa) : (IVe) : ,

worin X ein Halogen bedeutet und $R_1$ eine Benzylgruppe oder eine $\alpha$-Methylbenzylgruppe ist, oder die Salze dieser Verbindungen mit anorganischen oder organischen Säuren:

a) durch Umsetzung einer als Racemat vorliegenden Verbindung der folgenden Formel:

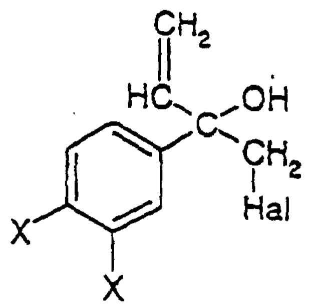

(V),

worin X die für die Verbindung der Formel (IV) angegebene Bedeutung aufweist und Hal ein Halogenatom ist, mit Benzylamin oder (R)(+)- oder (S)(-)-α-Methylbenzylamin in Gegenwart einer Base in einem inerten Lösungsmittel, um als Racemat eine Verbindung der folgenden Formel herzustellen:

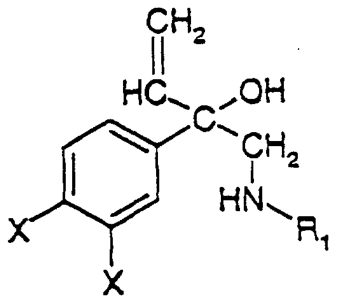

(VI) : (VIa) : 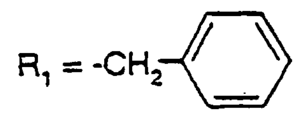

(VIe) : 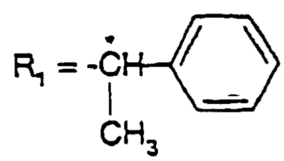

;

b) Trennung der Enantiomere oder Diastereoisomere der auf diese Weise erhaltenen Verbindungen der Formel (VI);

c) Umsetzung der so erhaltenen reinen Enantiomere der Formel (VI):

- entweder mit Ethylenoxid in Gegenwart einer Säure als Katalysator in einem inerten Lösungsmittel;
- oder mit einer Verbindung der Formel Hal""-$CH_2$-CH2-O-$R_2$ (XXI), worin $R_2$ eine O-Schutzgruppe bedeutet und Hal"" ein Halogenatom ist, in Gegenwart einer Base in einem inerten Lösungsmittel und anschließendes Entfernen der O-Schutzgruppe;
  und gegebenenfalls Umwandlung des so erhaltenen reinen Enantiomers der Formel (IV) in eines seiner Salze mit einer anorganischen oder organischen Säure.

5. Verfahren nach Anspruch 4, **dadurch gekennzeichnet, dass** in Schritt a) eine Verbindung der Formel (V) eingesetzt wird, worin Hal ein Chloratom oder ein Bromatom bedeutet.

6. Verfahren nach Anspruch 3, **dadurch gekennzeichnet, dass** eine Verbindung in Form des Racemats oder in Form eines Gemisches von Diastereoisomeren der folgenden Formel hergestellt wird:

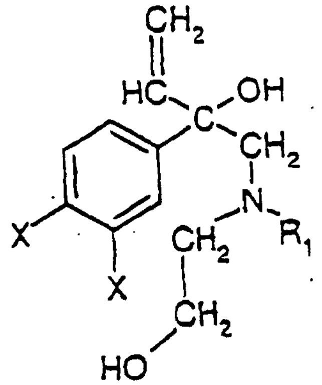

(IV) : (IVa) : 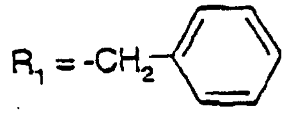

(IVd) : $R_1 = -COO-C(CH_3)_3$

(IVe) : 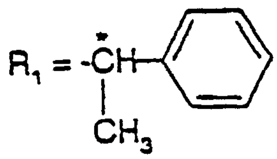 ,

worin X ein Halogenatom bedeutet und $R_1$ Benzyl, *t*-Butyloxycarbonyl oder α-Methylbenzyl bedeutet, oder gegebenenfalls eines ihrer Salze mit anorganischen oder organischen Säuren:

a) durch Umsetzung einer als Racemat vorliegenden Verbindung der folgenden Formel:

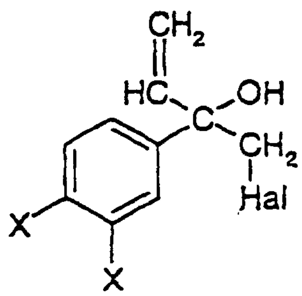

(V),

worin X die oben für die Verbindung der Formel (IV) angegebene Bedeutung aufweist und Hal ein Halogenatom ist, entweder mit 2-Benzylamino-1-ethanol oder mit 2-Amino-1-Ethanol oder mit (R)- oder (S)-2-(a-Methylbenzylamino)-1-ethanol in Gegenwart einer Base in einem inerten Lösungsmittel und gegebenenfalls Überführen der so erhaltenen Verbindung der Formel (IVa) oder (IVe) in eines ihrer Salze mit einer anorganischen oder organischen Säure;

b) gegebenenfalls, falls in Schritt a) die Verbindung der Formel (IV) mit 2-Amino-1-ethanol umgesetzt wird, durch Reaktion der erhaltenen Verbindung der Formel:

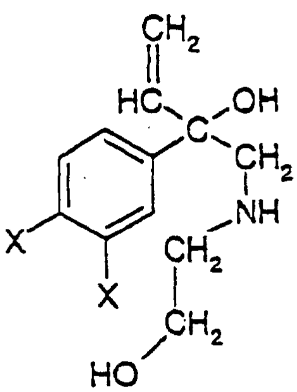

(IV'd)

mit Di-*t*-Butyldicarbonat in Gegenwart einer Base in einem inerten Lösungsmittel zur Herstellung der Verbindung der Formel (IVd).

7. Verfahren nach Anspruch 6, **dadurch gekennzeichnet, dass** eine Verbindung der Formel (V) verwendet wird, worin Hal ein Chloratom oder ein Bromatom bedeutet.

8. Verfahren nach einem der Ansprüche 4 oder 6, **dadurch gekennzeichnet, dass** eine Verbindung der folgenden Formel hergestellt wird:

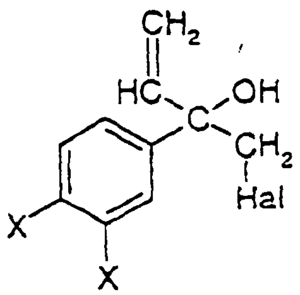

(V),

worin X ein Halogenatom bedeutet und Hal ein Halogenatom ist:

a) durch Umsetzung einer Verbindung der folgenden Formel:

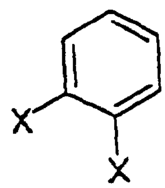

(VIII),

worin X die für die Verbindung der Formel (V) angegebene Bedeutung aufweist, mit einer Verbindung der folgenden Formel:

$$Hal'\text{-}CO\text{-}CH_2\text{-}Hal \qquad (IX),$$

worin Hal' und Hal ein Halogenatom bedeuten, in Gegenwart einer Lewis-Säure in einem inerten Lösungsmittel, zur Herstellung einer Verbindung der folgenden Formel:

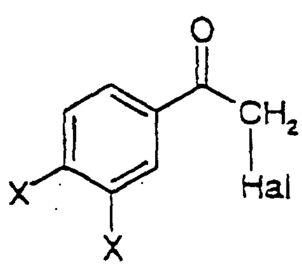

(X);

b) Umsetzung der auf diese Weise erhaltenen Verbindung der Formel (X) mit einer Verbindung der folgenden Formel:

$$CH_2=CH\text{-}Mg\text{-}Hal'' \quad (XI),$$

worin Hal" ein Halogenatom bedeutet, in einem inerten Lösungsmittel und anschließender Hydrolyse zur Herstellung der Verbindung der Formel (V).

**9.** Verfahren nach Anspruch 8, **dadurch gekennzeichnet, dass** eine Verbindung der Formel (V) hergestellt wird, worin Hal ein Chloratom oder ein Bromatom bedeutet.

**10.** Verfahren nach Anspruch 8, **dadurch gekennzeichnet, dass** in Schritt a) eine Verbindung der Formel (IX) verwendet wird, worin Hal' und Hal jeweils unabhängig voneinander ein Chloratom oder ein Bromatom bedeuten und in Schritt b) eine Verbindung der Formel (IX) verwendet wird, worin Hal" ein Chloratom oder ein Bromatom bedeutet.

**11.** Verfahren nach Anspruch 3, **dadurch gekennzeichnet, dass** eine Verbindung der folgenden Formel in Form des Racemats hergestellt wird:

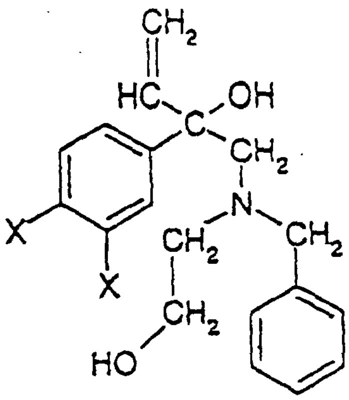

(IVa),

worin X ein Halogenatom bedeutet, oder ihre Salze mit einer anorganisches oder organischen Säure:

a) durch Umsetzung einer Verbindung der folgenden Formel:

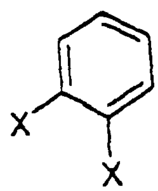

(VIII),

worin X die für die Verbindung der Formel (IVa) angegebene Bedeutung aufweist, mit einer Verbindung der folgenden Formel:

$$Hal'\text{-}CO\text{-}CH_2\text{-}Hal \quad (IX),$$

worin Hal' und Hal ein Halogenatom bedeuten, in Gegenwart einer Lewis-Säure in einem inerten Lösungsmittel zur Herstellung einer Verbindung der folgenden Formel:

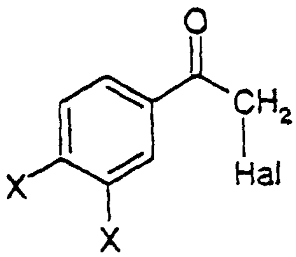

(X);

b) Umsetzung der Verbindung der Formel (X) mit einer Verbindung der folgenden Formel:

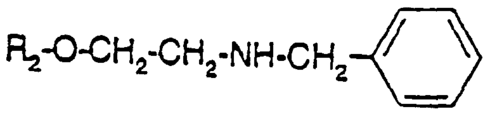

(XII),

worin $R_2$ eine O-Schutzgruppe bedeutet, in Gegenwart einer Base in einem inerten Lösungsmittel zur Herstellung einer Verbindung der folgenden Formel:

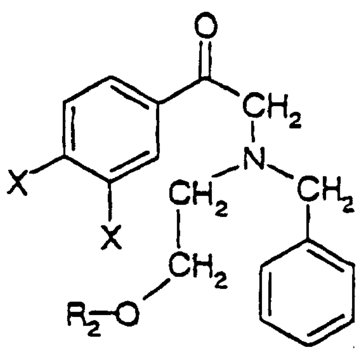

(XIII);

c) durch Umsetzung der so erhaltenen Verbindung der Formel (XIII) mit einer Verbindung der folgenden Formel:

$$CH_2=CH\text{-}Mg\text{-}Hal'' \qquad (XI),$$

worin Hal" ein Halogenatom bedeutet, in einem inerten Lösungsmittel und anschließender Hydrolyse zur Herstellung einer Verbindung der folgenden Formel:

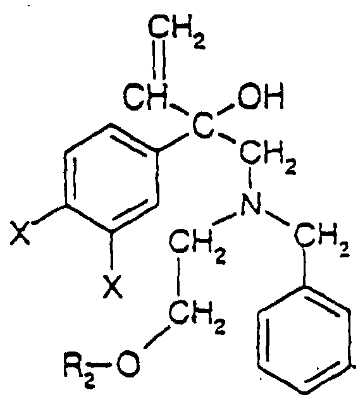

(XIV);

d) Entfernen der Schutzgruppe von der Verbindung der Formel (XIV) und gegebenenfalls Überführen der so erhaltenen Verbindung der Formel (IVa) in eines ihrer Salze mit einer anorganischen oder organischen Säure.

**12.** Verfahren nach Anspruch 11, **dadurch gekennzeichnet, dass** in Schritt a) eine Verbindung der Formel (IX) verwendet wird, worin Hal und Hal' jeweils unabhängig ein Chloratom oder ein Bromatom bedeuten, in Schritt b) eine Verbindung der Formel (XII) verwendet wird, worin $R_2$ eine Tetrahydropyran-2-yl-Gruppe ist, und in Schritt c) eine Verbindung der Formel (XI) verwendet wird, worin Hal" ein Chloratom oder ein Bromatom bedeutet.

**13.** Verfahren nach Anspruch 3, **dadurch gekennzeichnet, dass** eine Verbindung der folgenden Formel in Form der reinen Enantiomere hergestellt wird:

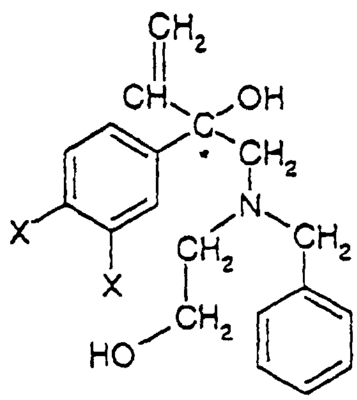

(IVa),

worin X ein Halogenatom bedeutet, oder ihre Salze mit einer anorganischen oder organischen Säure:

a) durch Umsetzung einer Verbindung der folgenden Formel:

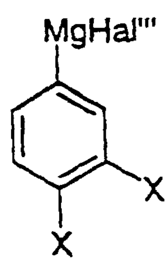

(XV),

worin X die für die Verbindung der Formel (IVa) angegebene Bedeutung aufweist und Hal''' ein Halogenatom bedeutet, mit (R)- oder (S)-2-Phenylhexahydro-pyrrolo[1,2-c]imidazol-3-carbonsäuremethylester der folgenden Formel:

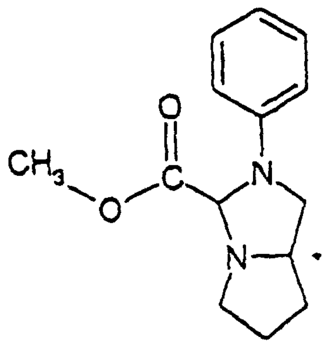

(XVI)

in Gegenwart von Magnesiumchlorid in einem inerten Lösungsmittel und anschließender Hydrolyse zur Herstellung einer Verbindung der folgenden Formel in Form der reinen Enantiomere:

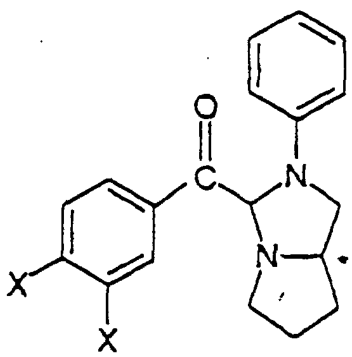

(XVII);

b) Umsetzung der so erhaltenen Verbindung der Formel (XVII) mit einer Verbindung der folgenden Formel:

$$CH_2=CH\text{-}Mg\text{-}Hal'' \quad (XI),$$

worin Hal'' ein Halogenatom bedeutet, in einem inerten Lösungsmittel und anschließender Hydrolyse zur Herstellung einer Verbindung in Form der reinen Enantiomere der folgenden Formel:

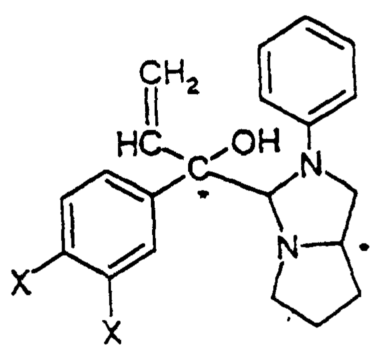

(XVIII);

c) Hydrolyse der erhaltenen Verbindung der Formel (XVIII) durch Einwirkung einer Säure in einem inerten Lösungsmittel im Gemisch mit Wasser zur Herstellung einer Verbindung der folgenden Formel in Form der reinen Enantiomere:

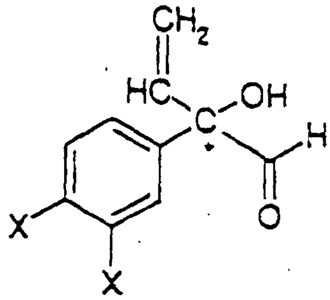

(XIX);

d) Umsetzung der Verbindung der Formel (XIX) mit 2-(Benzylamino)-1-ethanol in Gegenwart einer Säure in einem inerten Lösungsmittel und anschließender Reduktion des als Zwischenprodukt gebildeten Iminiumsalzes mit einem Reduktionsmittel und gegebenenfalls Überführen des reinen Enantiomers der Formel (IVa). in eines seiner Salze mit einer anorganischen oder organischen Säure.

**14.** Verfahren nach Anspruch 3, **dadurch gekennzeichnet, dass** eine Verbindung der folgenden Formel in Form des reinen Enantiomers hergestellt wird:

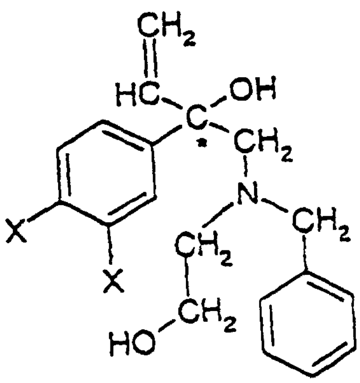

(IVa),

worin X ein Halogenatom bedeutet, oder ihre Salze mit einer anorganischen oder organischen Säure:

a) durch Umsetzung einer Verbindung der folgenden Formel:

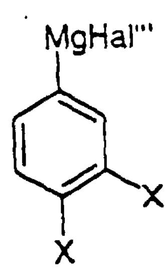

(XV),

worin X die für die Verbindung der Formal (IVa) angegebene Bedeutung aufweist und Hal''' ein Halogenatom ist, mit dem (R)- oder (S)-2-Phenylhexyhydro-pyrrolo[1,2-c]imidazol-3-carbonsäuremethylester der folgenden Formel:

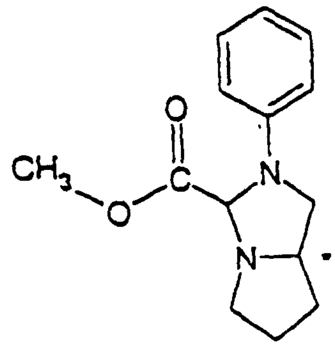

(XVI)

in Gegenwart von Magnesiumchlorid in einem inerten Lösungsmittel und anschließender Hydrolyse zur Herstellung einer Verbindung der folgenden Formel in Form der reinen Enantiomere:

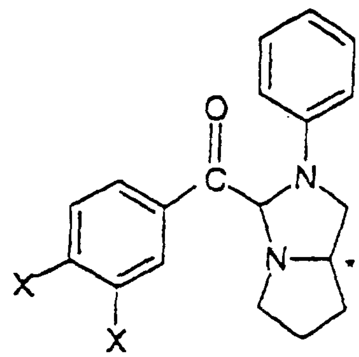

(XVII);

b) Umsetzung der so hergestellten Verbindung der Formel (XVII) mit einer Verbindung der folgenden Formel:

$$CH_2=CH\text{-}Mg\text{-}Hal'' , \qquad (XI),$$

worin Hal" ein Halogenatom bedeutet, in einem inerten Lösungsmittel und anschließender Hydrolyse zur Herstellung des reinen Enantiomers der folgenden Formel:

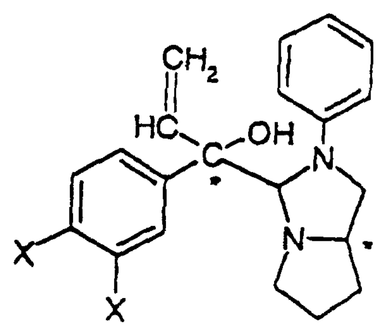

(XVIII);

c) Hydrolyse der so erhaltenen Verbindung der Formel (XVIII) durch Einwirkung einer Säure in einem inerten Lösungsmittel im Gemisch mit Wasser zur Herstellung einer Verbindung der folgenden Formel in Form des reinen Enantiomers:

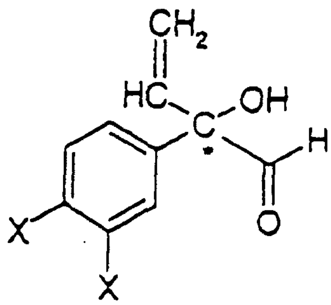

(XIX);

d) Reduktion der Verbindung der Formel (XIX) mit einem Reduktionsmittel in einem inerten Lösungsmittel zur Herstellung einer Verbindung der folgenden Formel:

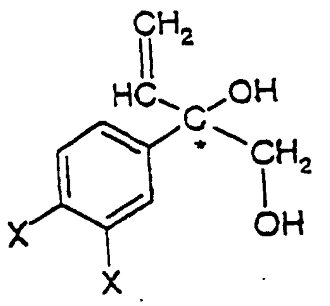

(XX);

e) Cyclisierung der so hergestellten Verbindung der Formel (XX) zur Herstellung eines reinen Enantiomers der folgenden Formel:

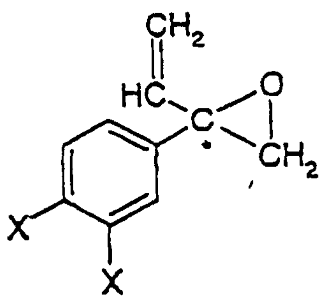

(VII);

f) Umsetzung der so erhaltenen Verbindung der Formel (VII) mit 2-(Benzylamino)-1-ethanol in Gegenwart einer Base in einem inerten Lösungsmittel und gegebenenfalls Überführen des reinen Enantiomers der Formel (IVa) in eines seiner Salze mit einer anorganische oder organischen Säure.

**15.** Verfahren nach einem der Ansprüche 13 oder 14, **dadurch gekennzeichnet, dass** in Schritt a) eine Verbindung der Formel (XV) verwendet wird, worin Hal''' ein Chloratom oder Bromatom bedeutet, und in Schritt b) eine Verbindung der Formel (XI) verwendet wird, worin Hal'' ein Chloratom oder Bromatom bedeutet.

**16.** Verfahren nach einem der Ansprüche 1, 2, 3, 4, 6, 8, 11, 13 oder 14, **dadurch gekennzeichnet, dass** Verbindungen der Formel (I), (IIa), (IIb), (IIc), (IId), (IIe), (IVa), (IVd), (IVe) oder (V) hergestellt werden, worin X ein Chloratom oder ein Fluoratom bedeutet.

**17.** Verbindungen der folgenden Formel:

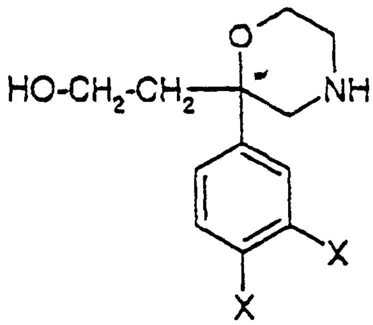

(I),

worin X ein Halogenatom bedeutet, enantiomer rein in Form des optisch aktiven Salzes mit L-(-)- oder D-(+)-Di-p-Toluolylweinsäure.

**18.** Verbindungen der folgenden Formel:

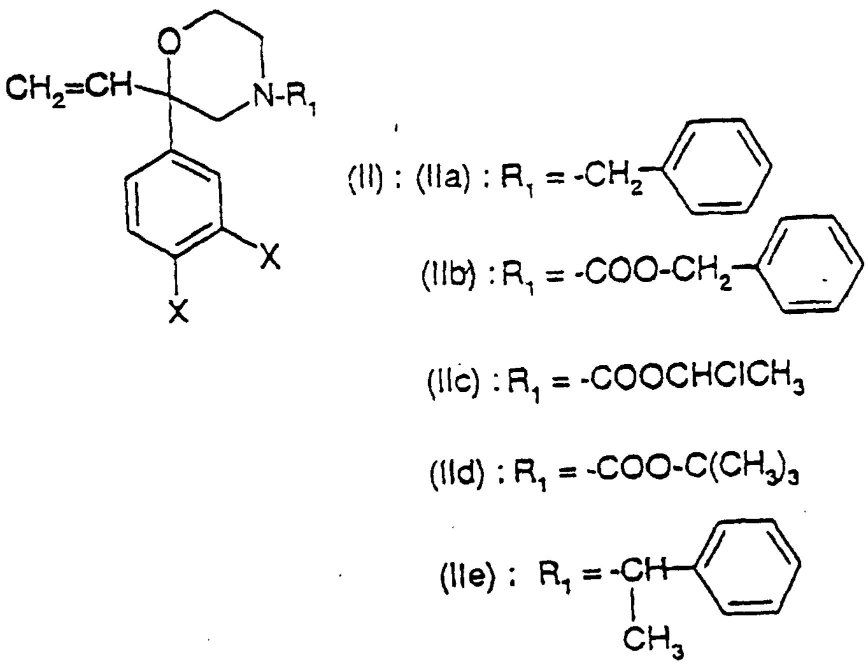

(II) : (IIa) : $R_1 = -CH_2-$

(IIb) : $R_1 = -COO-CH_2-$

(IIc) : $R_1 = -COOCHClCH_3$

(IId) : $R_1 = -COO-C(CH_3)_3$

(IIe) : $R_1 = -CH-$ $CH_3$ ,

worin X ein Halogenatom bedeutet und $R_1$ eine N-Schutzgruppe ist, die unter Benzyl, Benzyloxycarbonyl, 1-Chlorethyloxycarbonyl, *t*-Butyloxycarbonyl oder $\alpha$-Methylbenzyl ausgewählt ist, in Form des Racemats, in Form der reinen Enantiomere oder in Form eines Gemisches von Diastereoisomeren und ihre möglichen Salze mit einer anorganischen oder organischen Säure.

**19.** Verbindungen der Formel:

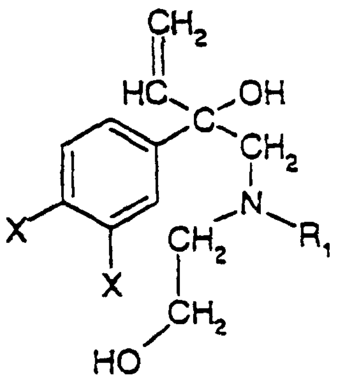

(IV) :

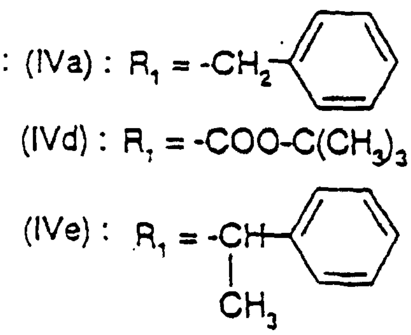

(IVa) : $R_1$ =

(IVd) : $R_1$ = $-COO-C(CH_3)_3$

(IVe) : $R_1$ =

worin X ein Halogenatom bedeutet und $R_1$ eine Benzylgruppe, eine *t*-Butyloxycarbonylgruppe oder eine α-Methylbenzylgruppe ist, in Form des Racemats, in Form der reinen Enantiomere oder in Form eines Gemisches von Diastereoisomeren und ihre möglichen Salze mit anorganischen oder organischen Säuren.

**20.** Verbindungen der Formel:

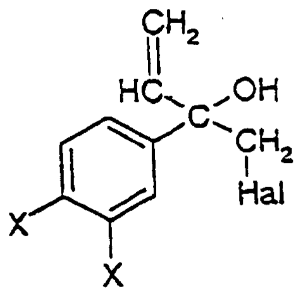

(V),

worin X ein Halogenatom bedeutet und Hal ein Halogenatom bedeutet.

**21.** Verbindungen nach Anspruch 20 der Formel (V), worin Hal ein Chloratom oder ein Bromatom bedeutet.

**22.** Verbindungen der Formel:

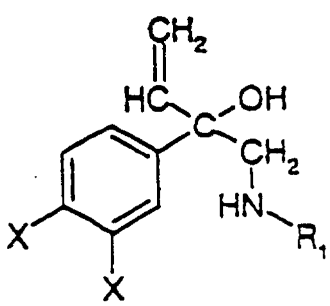

(VI) :

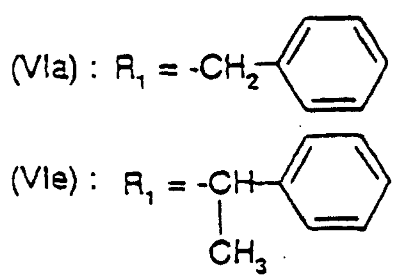

(VIa) : $R_1$ =

(VIe) : $R_1$ =

worin X ein Halogenatom bedeutet und $R_1$ ein Benzylgruppe oder eine α-Methylbenzylgruppe ist, in Form des

Racemats, in Form der reinen Enantiomere oder in Form eines Gemisches von Diastereoisomeren und ihre Salze mit organischen oder anorganischen Säuren oder ihre Salze mit optisch aktiven Säuren.

**23.** Verbindungen der Formel (IVa) nach Anspruch 22 enantiomer rein in Form des Salzes mit der L-(+)- oder D-(-)-Mandelsäure.

**24.** Verbindungen der Formel:

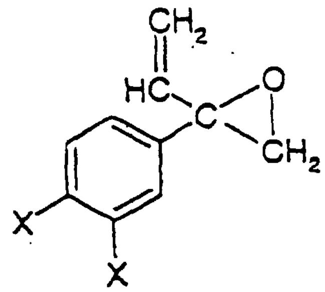

(VII),

worin X ein Halogenatom bedeutet, in Form des Racemats oder als reine Enantiomere.

**25.** Verbindungen der Formel:

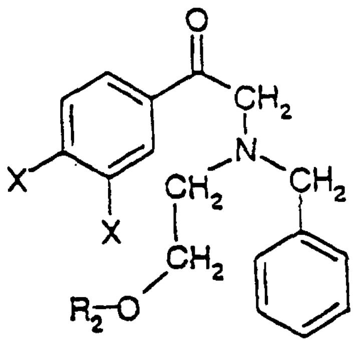

(XIII),

worin X ein Halogenatom bedeutet und $R_2$ die Tetrahydropyran-2-yl-Gruppe ist, und ihre Salze mit organischen oder anorganischen Säuren.

**26.** Verbindungen der Formel:

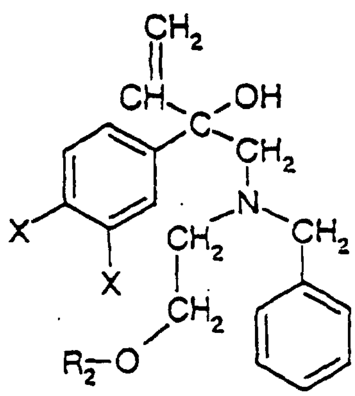

(XIV),

worin X ein Halogenatom bedeutet und $R_2$ die Tetrahydropyran-2-yl-Gruppe ist, und ihre Salze mit organischen oder anorganischen Säuren.

**27.** Verbindungen der Formel:

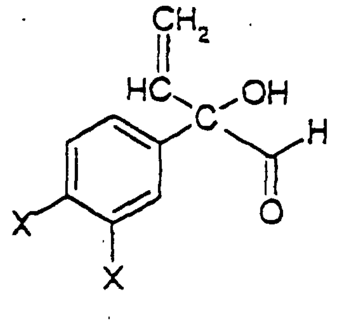

(XIX),

worin X ein Halogenatom bedeutet, als Racemat oder in Form der reinen Enantiomere.

**28.** Verbindungen der Formel:

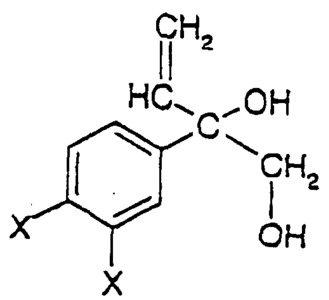

(XX),

worin X ein Halogenatom bedeutet, als Racemat oder in Form der reinen Enantiomere.

**29.** Verbindungen nach einem der Ansprüche 17 bis 28 der Formel (I), (II), (IV), (V), (VI), (VII), (XIII), (XIV), (XIX) oder (XX), worin X ein Chloratom oder ein Fluoratom bedeutet.

**Claims**

**1.** Process for the preparation of a compound, in the enantiomerically pure form, of formula:

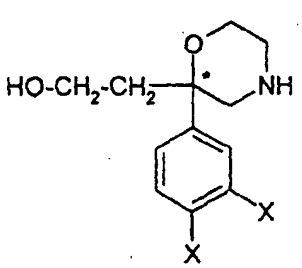

(I)

in which X represents a halogen atom, of its salts with inorganic or organic acids or of its salts with optically active organic acids, **characterized in that**:

a) a compound, in the racemic form, in the form of a mixture of diastereoisomers or in the enantiomerically pure form, of formula:

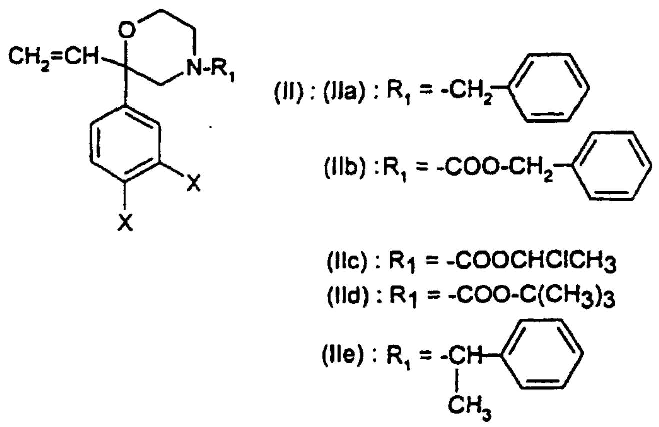

in which X is as defined for a compound of formula (I) and $R_1$ represents an N-protecting group chosen from a benzyl group, a benzyloxycarbonyl group, a 1-chloroethyloxycarbonyl group, a *tert*-butyloxycarbonyl group or an α-methylbenzyl group, is converted to a compound, in the racemic form, in the form of a mixture of diastereoisomers or in the enantiomerically pure form, of formula:

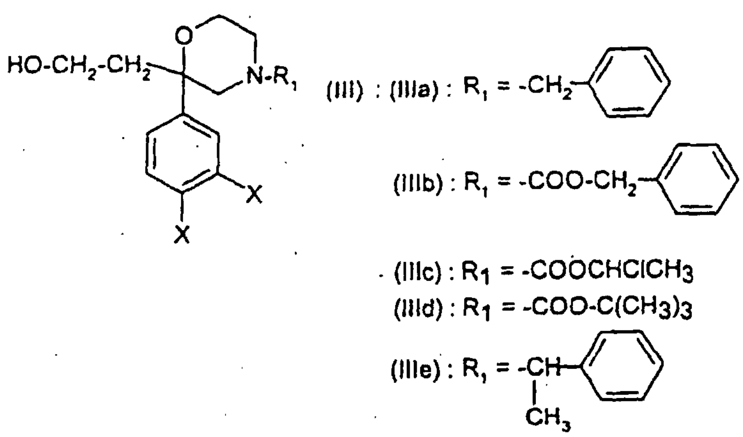

b) the compound of formula (III) thus obtained is deprotected;
c) if appropriate, when the compound of formula (I) thus obtained is in the racemic form, the enantiomers are separated, and, optionally, the enantiomerically pure compound of formula (I) is converted to one of its salts with inorganic or organic acids.

**2.** Process according to Claim 1, **characterized in that** a compound, in the enantiomerically pure form or in the racemic form, of formula:

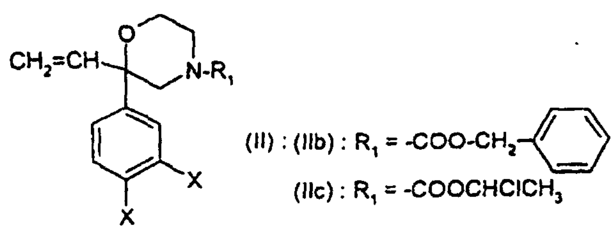

in which X represents a halogen atom and $R_1$ represents a benzyloxycarbonyl group or a 1-chloroethyloxycarbonyl group, is prepared by reaction of a compound, in the enantiomerically pure form or in the racemic form, of formula:

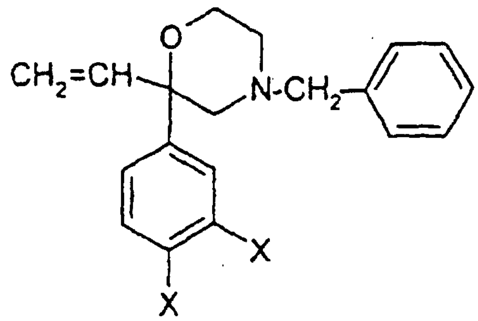

(IIa)

in which X is as defined for a compound of formula (II), with benzyl chloroformate or 1-chloroethyl chloroformate in the presence of a base, with or without solvent.

**3.** Process according to either of Claims 1 and 2, **characterized in that** a compound, in the enantiomerically pure form, in the form of a mixture of diastereoisomers or in the racemic form, of formula:

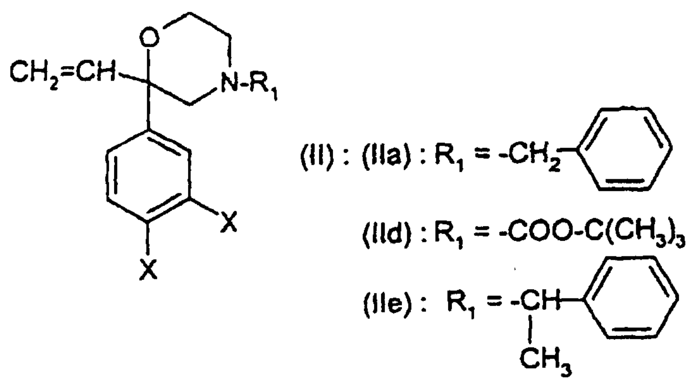

(II) : (IIa) : $R_1 = -CH_2-$

(IId) : $R_1 = -COO-C(CH_3)_3$

(IIe) : $R_1 = -CH-$

$CH_3$

in which X represents a halogen atom and $R_1$ represents a benzyl group, a *tert*-butyloxycarbonyl group or an α-methylbenzyl group, of its optional salts with inorganic or organic acids, is prepared by cyclization of a compound, in the enantiomerically pure form, in the form of a mixture of diastereoisomers or in the racemic form, of formula:

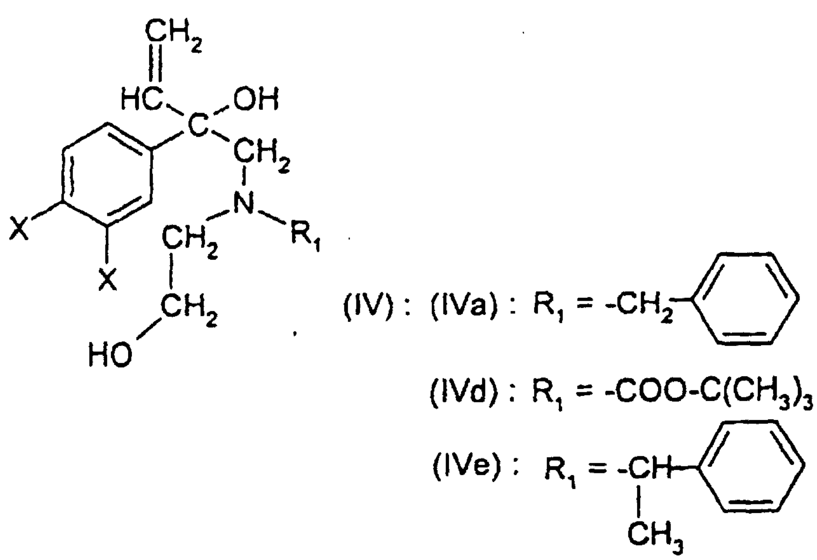

(IV) : (IVa) : $R_1 = -CH_2-$

(IVd) : $R_1 = -COO-C(CH_3)_3$

(IVe) : $R_1 = -CH-$

$CH_3$

in which X and $R_1$ are as defined for a compound of formula (II), and, optionally, the compound of formula (II) thus obtained is converted to one of its salts.

**4.** Process according to Claim 3, **characterized in that** a compound, in the enantiomerically pure form, of formula:

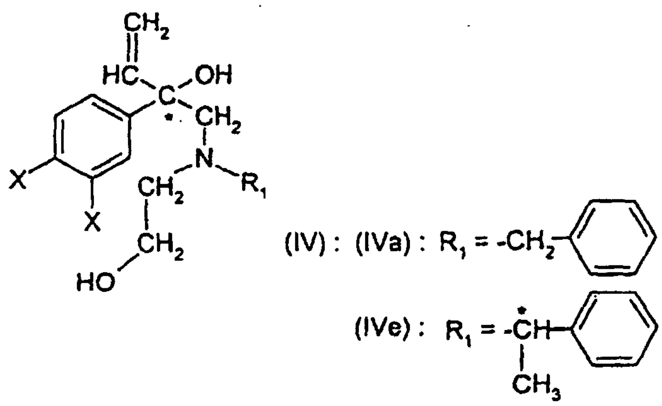

(IV) : (IVa) :

(IVe) :

in which X represents a halogen atom and $R_1$ represents a benzyl group or an $\alpha$-methylbenzyl group, of its salts with inorganic or organic acids, is prepared:

a) by reaction of a compound, in the racemic form, of formula:

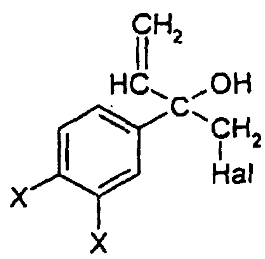

(V)

in which X is as defined for a compound of formula (IV) and Hal represents a halogen atom, with benzylamine or with R-(+)- or S-(-)-$\alpha$-methylbenzylamine in the presence of a base in an inert solvent, to produce a compound, in the racemic form, of formula:

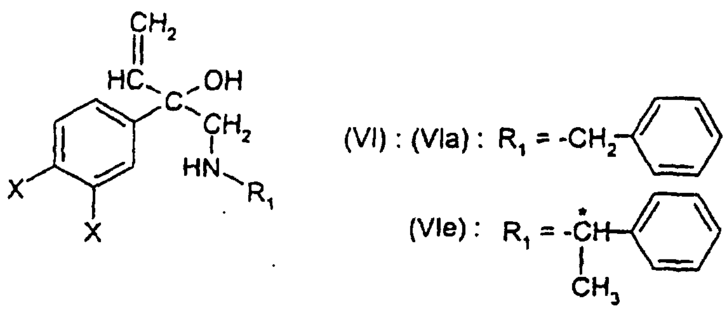

(VI) : (VIa) :

(VIe) :

b) by separation of the enantiomers or diastereoisomers of the compound of formula (VI) thus obtained;
c) by reaction of the enantiomerically pure compound of formula (VI) thus obtained:

- either with ethylene oxide in the catalytic presence of an acid in an inert solvent;
- or with a compound of formula Hal""-$CH_2$-$CH_2$-O-$R_2$ (XXI), in which $R_2$ represents an O-protecting group and Hal"" represents a halogen atom, in the presence of a base in an inert solvent, followed by the deprotection of the O-protecting group;

and, optionally, by conversion of the enantiomerically pure compound of formula (IV) thus obtained to one of its salts with inorganic or organic acids.

5. Process according to Claim 4, **characterized in that**, in stage a), use is made of a compound of formula (V) in which Hal represents a chlorine or bromine atom.

6. Process according to Claim 3, **characterized in that** a compound, in the racemic form or in the form of a mixture of diastereoisomers, of formula:

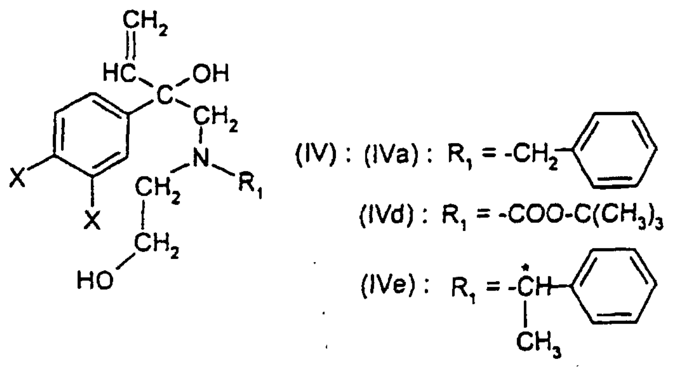

in which X represents a halogen atom and $R_1$ represents the benzyl group, the *tert*-butyloxycarbonyl group or the $\alpha$-methylbenzyl group, or one of its optional salts with inorganic or organic acids, is prepared:

a) by reaction of a compound, in the racemic form, of formula:

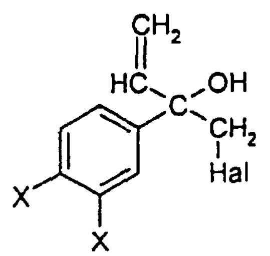

(V)

in which X is as defined for a compound of formula (IV) and Hal represents a halogen atom, either with 2-(benzylamino)-1-ethanol or with 2-amino-1-ethanol or with (R)- or (S)-2-($\alpha$-methylbenzylamino)-1-ethanol, in the presence of a base and in an inert solvent, and, optionally, by conversion of the compound of formula (IVa) or (IVe) thus obtained to one of its salts with inorganic or organic acids;
b) if appropriate, when the compound of formula (V) is employed with 2-amino-1-ethanol in stage a), by reaction of the compound thus obtained, of formula:

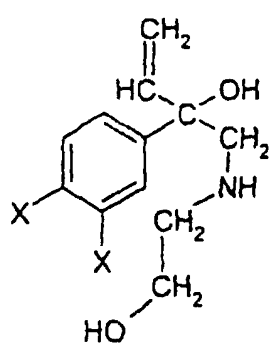

(IV'd)

with di-*tert*-butyl dicarbonate in the presence of a base and in an inert solvent, to produce the compound of formula (IVd).

7. Process according to Claim 6, **characterized in that** use is made of a compound of formula (V) in which Hal represents a chlorine or bromine atom.

8. Process according to either of Claims 4 and 6, **characterized in that** a compound of formula:

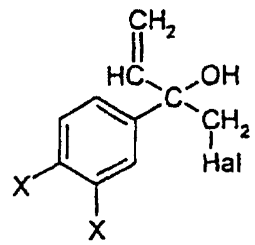

(V)

in which X represents a halogen atom and Hal represents a halogen atom, is prepared:

a) by reaction of a compound of formula:

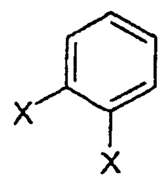

(VIII)

in which X is as defined for a compound of formula (V), with a compound of formula:

$$Hal'\text{-}CO\text{-}CH_2\text{-}Hal \quad (IX)$$

in which Hal' and Hal represent a halogen atom, in the presence of a Lewis acid and in an inert solvent, to produce a compound of formula:

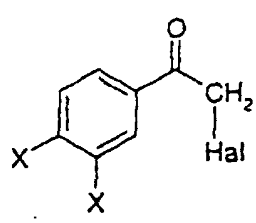

(X)

b) by reaction of the compound of formula (X) thus obtained with a compound of formula:

$$CH_2{=}CH\text{-}Mg\text{-}Hal'' \quad (XI)$$

in which Hal" represents a halogen atom, in an inert solvent, followed by hydrolysis, to produce the compound of formula (V).

9. Process according to Claim 8, **characterized in that** a compound of formula (V) in which Hal represents a chlorine or bromine atom is prepared.

**10.** Process according to Claim 8, **characterized in that**, in stage a), use is made of a compound of formula (IX) in which Hal' and Hal each independently represent a chlorine or bromine atom and, in stage b), use is made of a compound of formula (XI) in which Hal" represents a chlorine or bromine atom.

**11.** Process according to Claim 3, **characterized in that** a compound, in the racemic form, of formula:

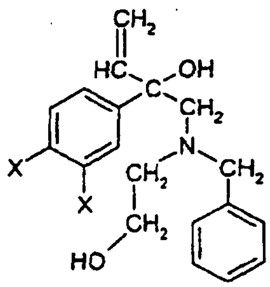
(IVa)

in which X represents a halogen atom, or one of its salts with inorganic or organic acids, is prepared:

a) by reaction of a compound of formula:

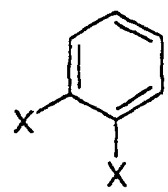
(VIII)

in which X is as defined for a compound of formula (IVa), with a compound of formula:

$$Hal'\text{-}CO\text{-}CH_2\text{-}Hal \qquad (IX)$$

in which Hal' and Hal represent a halogen atom, in the presence of a Lewis acid and in an inert solvent, to produce a compound of formula:

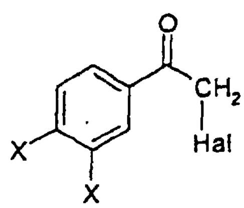
(X

b) by reaction of the compound of formula (X) thus obtained with a compound of formula:

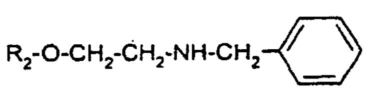
(XII)

in which $R_2$ represents an O-protecting group, in the presence of a base and in an inert solvent, to produce a compound of formula:

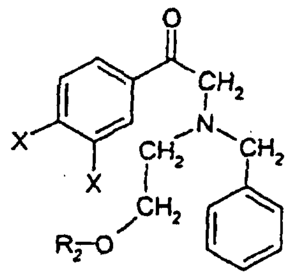

(XIII)

c) by reaction of the compound of formula (XIII) thus obtained with a compound of formula:

$$CH_2{=}CH\text{-}Mg\text{-}Hal'' \qquad (XI)$$

in which Hal" represents a halogen atom, in an inert solvent, followed by hydrolysis, to produce a compound of formula:

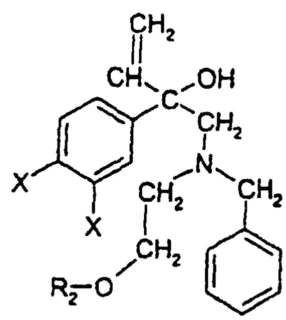

(XIV)

d) by deprotection of the compound of formula (XIV) and, optionally, by conversion of the compound of formula (IVa) thus obtained to one of its salts with inorganic or organic acids.

**12.** Process according to Claim 11, **characterized in that**, in stage a), use is made of a compound of formula (IX) in which Hal and Hal' each independently represent a chlorine or bromine atom, in stage b), use is made of a compound of formula (XII) in which $R_2$ represents a tetrahydropyran-2-yl group, and, in stage c), use is made of a compound of formula (XI) in which Hal" represents a chlorine or bromine atom.

**13.** Process according to Claim 3, **characterized in that** a compound, in the enantiomerically pure form, of formula:

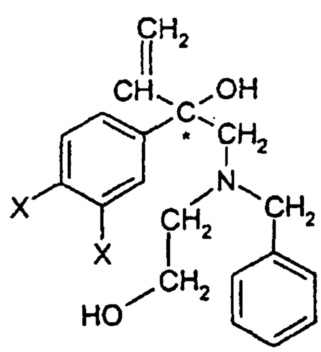

(IVa)

in which X represents a halogen atom, or one of its salts with inorganic or organic acids, is prepared:

a) by reaction of a compound of formula:

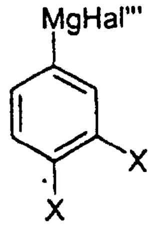

(XV)

in which X is as defined for a compound of formula (IVa) and Hal''' represents a halogen atom, with methyl (R)- or (S)-2-phenylhexahydropyrrolo[1,2-c]imidazole-3-carboxylate, of formula:

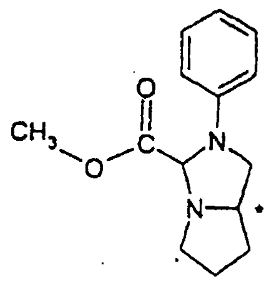

(XVI)

in the presence of magnesium chloride in an inert solvent, followed by hydrolysis, to produce a compound, in the enantiomerically pure form, of formula:

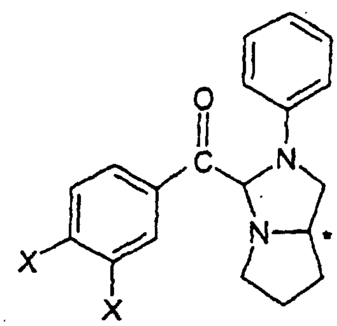

(XVII)

b) by reaction of the compound of formula (XVII) thus obtained with a compound of formula:

$$CH_2=CH\text{-}Mg\text{-}Hal'' \qquad (XI)$$

in which Hal'' represents a halogen atom, in an inert solvent, followed by hydrolysis, to produce a compound, in the enantiomerically pure form, of formula:

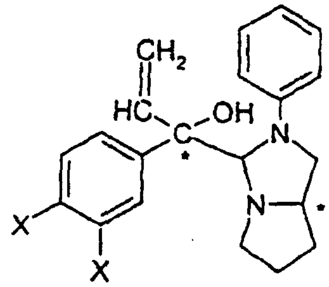

(XVIII)

c) by hydrolysis of the compound of formula (XVIII) thus obtained by the action of an acid in an inert solvent as a mixture with water, to produce a compound, in the enantiomerically pure form, of formula:

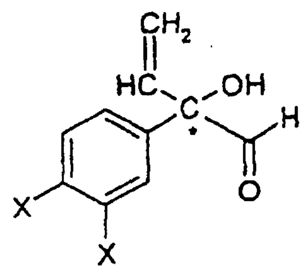

(XIX)

d) by reaction of the compound of formula (XIX) thus obtained with 2-(benzylamino)-1-ethanol in the presence of an acid in an inert solvent, then reduction of the iminium salt formed as an intermediate by means of a reducing agent and, optionally, conversion of the enantiomerically pure compound of formula (IVa) to one of its salts with inorganic or organic acids.

**14.** Process according to Claim 3, **characterized in that** a compound, in the enantiomerically pure form, of formula:

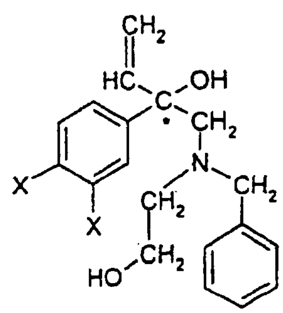

(IVa)

in which X represents a halogen atom, or one of its salts with inorganic or organic acids, is prepared:

a) by reaction of a compound of formula:

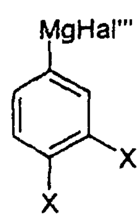

(XV)

in which X is as defined for a compound of formula (IVa) and Hal''' represents a halogen atom, with methyl

(R)- or (S)-2-phenylhexahydropyrrolo[1,2-c]imidazole-3-carboxylate, of formula:

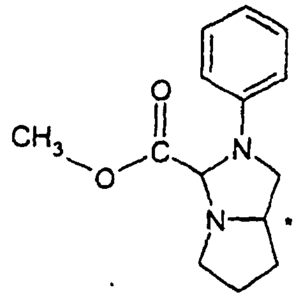

(XVI)

in the presence of magnesium chloride in an inert solvent, followed by hydrolysis, to produce a compound, in the enantiomerically pure form, of formula:

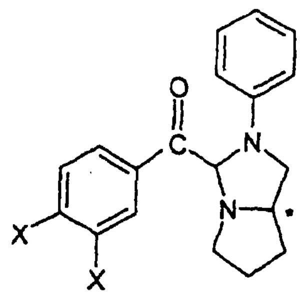

(XVII)

b) by reaction of the compound of formula (XVII) thus obtained with a compound of formula:

$$CH_2=CH\text{-}Mg\text{-}Hal'' \quad (XI)$$

in which Hal" represents a halogen atom, in an inert solvent, followed by hydrolysis, to produce a compound, in the enantiomerically pure form, of formula:

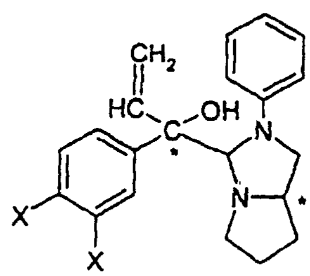

(XVIII)

c) by hydrolysis of the compound of formula (XVIII) thus obtained by the action of an acid in an inert solvent as a mixture with water, to produce a compound, in the enantiomerically pure form, of formula:

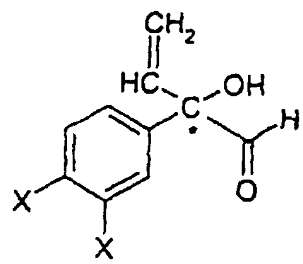

(XIX)

d) by reduction of the compound of formula (XIX) thus obtained by means of a reducing agent in an inert solvent, to produce a compound of formula:

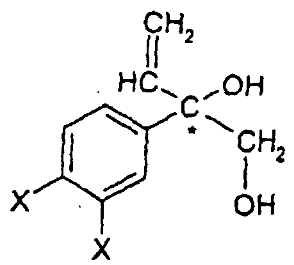

(XX)

e) by cyclization of the compound of formula (XX) thus obtained, to produce a compound, in the enantiomerically pure form, of formula:

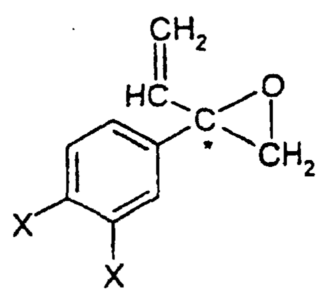

(VII)

f) by reaction of the compound of formula (VII) thus obtained with 2-(benzylamino)-1-ethanol in the presence of a base and in an inert solvent and, optionally, conversion of the enantiomerically pure compound of formula (IVa) thus obtained to one of its salts with inorganic or organic acids.

**15.** Process according to either of Claims 13 and 14, **characterized in that**, in stage a), use is made of a compound of formula (XV) in which Hal''' represents a chlorine or bromine atom and, in stage b), use is made of a compound of formula (XI) in which Hal'' represents a chlorine or bromine atom.

**16.** Process according to one of Claims 1, 2, 3, 4, 6, 8, 11, 13 and 14, **characterized in that** compounds of formula (I), (IIa), (IIb), (IIc), (IId), (IIe), (IVa), (IVd), (IVe) or (V) in which X represent a chlorine atom or a fluorine atom are prepared.

**17.** Enantiomerically pure compound of formula:

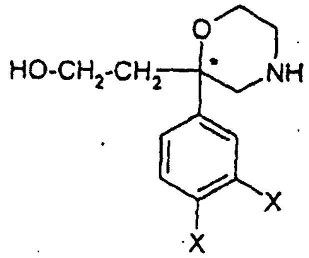

(I)

in which X represents a halogen atom, in the form of an optically active salt with L-(-)- or D-(+)-di-paratoluoyltartaric acid.

**18.** Compound of formula:

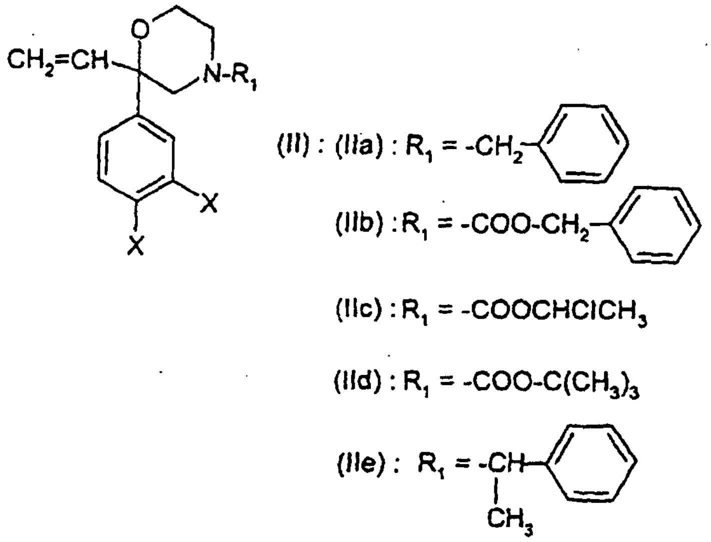

(II) : (IIa) (IIb) (IIc) (IId) (IIe)

in which X represents a halogen atom and $R_1$ represents an N-protecting group chosen from a benzyl group, a benzyloxycarbonyl group, a 1-chloroethyloxycarbonyl group, a *tert*-butyloxycarbonyl group or an α-methylbenzyl group, in the racemic form, in the enantiomerically pure form or in the form of a mixture of diastereoisomers, and its optional salts with inorganic or organic acids.

**19.** Compound of formula:

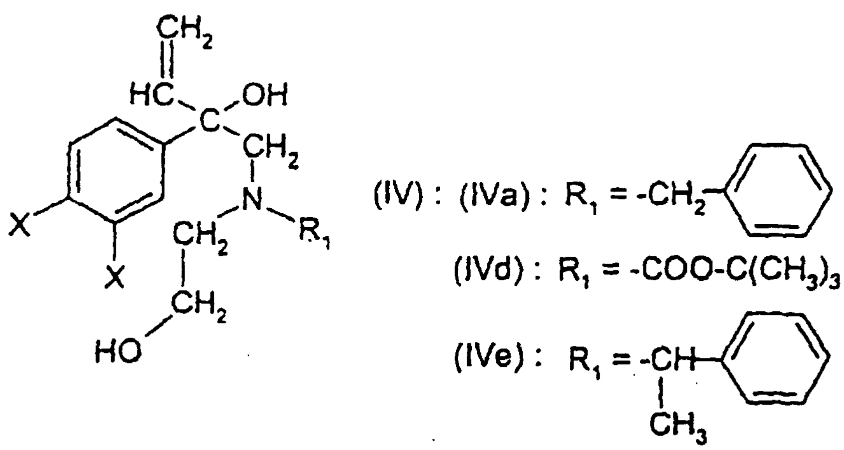

(IV) : (IVa) (IVd) (IVe)

in which X represents a halogen atom and $R_1$ represents a benzyl group, a *tert*-butyloxycarbonyl group or an α-methylbenzyl group, in the racemic form, in the enantiomerically pure form or in the form of a mixture of diastereoisomers, and its optional salts with inorganic or organic acids.

**20.** Compound of formula:

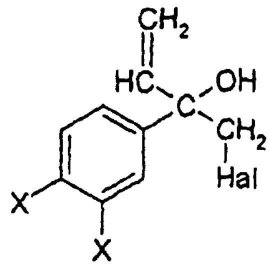

(V)

in which X represents a halogen atom and Hal represents a halogen atom.

**21.** Compound according to Claim 20 of formula (V), in which Hal represents a chlorine or bromine atom.

**22.** Compound of formula:

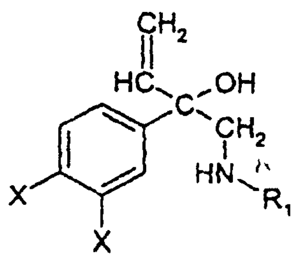

(VI) : (VIa) : 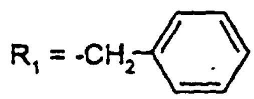

(VIe) : 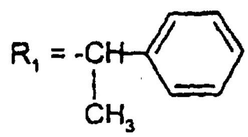

in which X represents a halogen atom and $R_1$ represents a benzyl group or an α-methylbenzyl group, in the racemic form, in the enantiomerically pure form or in the form of a mixture of diastereoisomers, and its salts with inorganic or organic acids or its salts with optically active acids.

**23.** Enantiomerically pure compound of formula (VIa) according to Claim 22, in the form of the salt with L-(+)- or D-(-)-mandelic acid.

**24.** Compound of formula:

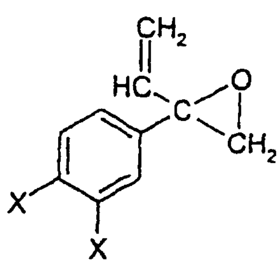

(VII)

in which X represents a halogen atom, in the racemic form or in the enantiomerically pure form.

**25.** Compound of formula:

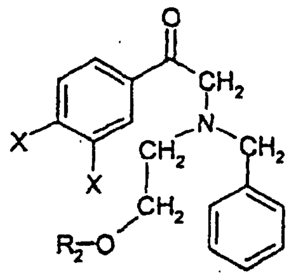

(XIII)

in which X represents a halogen atom and $R_2$ represents a tetrahydropyran-2-yl group, and its salts with inorganic or organic acids.

**26.** Compound of formula:

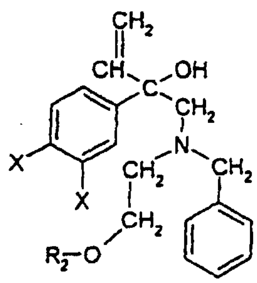

(XIV)

in which X represents a halogen atom and $R_2$ represents a tetrahydropyran-2-yl group, and its salts with inorganic or organic acids.

**27.** Compound of formula:

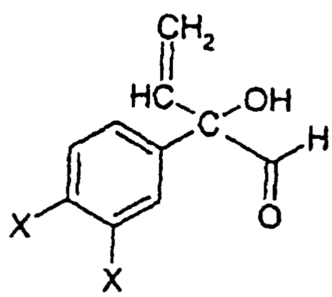

(XIX)

in which X represents a halogen atom, in the racemic form or in the enantiomerically pure form.

**28.** Compound of formula:

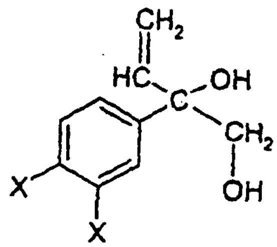

(XX)

in which X represents a halogen atom, in the racemic form or in the enantiomerically pure form.

**29.** Compound according to one of Claims 17 to 28 of formula (I), (II), (IV), (V), (VI), (VII), (XIII), (XIV), (XIX) or (XX) in which X represents a chlorine atom or a fluorine atom.